(19) **Europäisches Patentamt / European Patent Office / Office européen des brevets**

(11) **EP 4 238 648 A1**

(12) **EUROPEAN PATENT APPLICATION**
published in accordance with Art. 153(4) EPC

(43) Date of publication:
06.09.2023  Bulletin 2023/36

(21) Application number: 21885152.5

(22) Date of filing: 26.10.2021

(51) International Patent Classification (IPC):
*B01J 23/75* (2006.01)       *B01J 23/755* (2006.01)
*B01J 23/78* (2006.01)       *B01J 23/80* (2006.01)
*B01J 23/83* (2006.01)       *C07C 211/03* (2006.01)
*C07C 209/04* (2006.01)

(52) Cooperative Patent Classification (CPC):
B01J 23/75; B01J 23/755; B01J 23/78;
B01J 23/80; B01J 23/83; C07C 209/04;
C07C 211/03

(86) International application number:
PCT/CN2021/126422

(87) International publication number:
WO 2022/089425 (05.05.2022 Gazette 2022/18)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority:  30.10.2020  CN 202011187666
30.10.2020  CN 202011188198
30.10.2020  CN 202011188203
30.10.2020  CN 202011192975
30.10.2020  CN 202011188652

(71) Applicants:
• China Petroleum & Chemical Corporation
Beijing 100728 (CN)
• BEIJING RESEARCH INSTITUTE OF CHEMICAL
INDUSTRY,
CHINA PETROLEUM & CHEMICAL
CORPORATION
Chaoyang District
Beijing 100013 (CN)

(72) Inventors:
• TIAN, Baoliang
Beijing 100013 (CN)

• TANG, Guoqi
Beijing 100013 (CN)
• XIANG, Liangyu
Beijing 100013 (CN)
• ZHANG, Xiaorong
Beijing 100013 (CN)
• WANG, Guoqing
Beijing 100013 (CN)
• PENG, Hui
Beijing 100013 (CN)
• YANG, Yi
Beijing 100013 (CN)
• ZHANG, Lijun
Beijing 100013 (CN)
• SONG, Chao
Beijing 100013 (CN)
• JIANG, Jianzhun
Beijing 100013 (CN)
• MAN, Yi
Beijing 100013 (CN)

(74) Representative: Hoffmann Eitle
Patent- und Rechtsanwälte PartmbB
Arabellastraße 30
81925 München (DE)

(54) **AMINATION CATALYST AND PREPARATION AND USE THEREOF**

(57)     Disclosed is a catalyst useful for producing organic amines by catalytic amination, its preparation and application thereof, wherein the catalyst comprises an inorganic porous carrier containing aluminum and/or silicon and an active metal component supported on the carrier, the active metal component comprises at least one metal selected from the group consisting of Group VIII and Group IB metals, and the carrier has an ammonia adsorption capacity of 0.25 to 0.65 mmol/g, as measured by $NH_3$-TPD test. The catalyst has an improved performance, when used for producing organic amines by catalytic amination.

**Description**

Technical Field

The present application relates to the field of amination reaction, particularly to a catalyst for producing organic amine by catalytic amination, its preparation and application thereof.

Background Art

Amines are very important industrial organic compounds and are widely used in various fields, for example, as solvents, medical intermediates, resin raw materials, textile additives, insecticides, rubber stabilizers, resists, and also in cleaning and plastics processing. The three main processes for producing amines are the hydroamination of carbonyl compounds, the hydroamination of alcohols and the hydrogenation of nitriles. The hydroamination of carbonyl compounds is, for example, the reaction of acetone, hydrogen and ammonia to form isopropylamine. The hydroamination of alcohols includes, for example, the hydroamination of ethanol with ammonia in the presence of hydrogen to form ethylamine, the hydroamination of isopropanol with ammonia in the presence of hydrogen to form isopropylamine, the hydroamination of butanol with ammonia in the presence of hydrogen to form butylamine, and the hydroamination of hexanediol with ammonia in the presence of hydrogen to form hexanediamine, etc. Nitrile hydrogenation is, for example, the hydrogenation of acetonitrile to ethylamine and the hydrogenation of adiponitrile to hexanediamine.

US Patent No. US4409399 discloses a process for producing fatty amines. The catalyst used consists of (1) copper oxide or hydroxide, (2) nickel oxide or hydroxide and (3) an oxide or hydroxide of a Group IIA metal.

Chinese patent application No. CN102658162A discloses a catalyst for synthesizing ethyleneamine and a process for producing ethyleneamine. The catalyst consists of three parts, namely a main active component, an auxiliary agent and an aminated carrier, wherein the main active component is one or more selected from Ni and Co and accounts for 1-40% of the total weight of the catalyst, and the auxiliary agent is one or more selected from the group consisting of Fe, Cu, Ru, Re, K, Zn and B and oxides thereof, and accounts for 0.1-20% of the total weight of the catalyst; the aminated carrier is obtained by amination of one or more carriers selected from the group consisting of $SiO_2$ and $Al_2O_3$.

However, the catalytic activity, raw material conversion, product selectivity and catalyst stability of existing catalysts for amination reaction still need to be improved.

Summary of the Invention

It is an object of the present application to provide a catalyst useful for producing organic amines by catalytic amination, its preparation and application thereof, which catalyst shows improved performance when used for amination reaction, such as at least one of improved catalytic activity, improved reaction conversion, improved product selectivity and improved catalyst stability.

To achieve the above object, in one aspect, the present application provides a catalyst useful for producing organic amines by catalytic amination, comprising an inorganic porous carrier containing aluminum and/or silicon, and an active metal component supported on the carrier, the active metal component comprising at least one metal selected from Group VIII and Group IB metals, wherein the carrier has an ammonia adsorption capacity of 0.25 to 0.65 mmol/g as measured by $NH_3$-TPD test.

Preferably, the carrier comprises a matrix comprising a first carrier component and optionally a second carrier component, and a doping element, wherein the first carrier component is selected from alumina, silica, molecular sieves, aluminosilicates or combinations thereof, the second carrier component is selected from diatomite and titania, and the doping element is selected from a metallic element, a non-metallic element, or a combination thereof, excluding sodium and chlorine, the metallic element is at least one selected from the group consisting of Group IA metallic elements, Group IIA metallic elements, Group VA metallic elements, and lanthanide series metallic elements; the non-metallic element is at least one selected from the group consisting of Group IIIA non-metallic elements, Group VA non-metallic elements, Group VIA non-metallic elements and Group VIIA non-metallic elements.

Preferably, the catalyst further comprises a metal promoter supported on the carrier, and the metal promoter comprises at least one metal selected from the group consisting of Group VIB, Group VIIB, Group IB, Group IIB and lanthanide series metals.

In another aspect, there is provided a method for producing the catalyst of the present application, comprising the steps of:

1) providing an inorganic porous carrier containing aluminum and/or silicon, which has an ammonia adsorption capacity of 0.25 to 0.65 mmol/g as measured by $NH_3$-TPD test;
2) loading the active metal component and optionally the metal promoter on the carrier; and

3) carrying out a heat treatment and optionally a reduction treatment on the material obtained in step 2) to obtain the catalyst.

In yet another aspect, the present application provides a process for producing organic amines, comprising: contacting an amination raw material, an amination reagent and a catalyst according to the present application for amination reaction in the presence of hydrogen to obtain an organic amine, wherein the amination raw material is selected from the group consisting of alcohols, ketones, alcohol amines, aldehydes and combinations thereof; and the amination reagent is selected from the group consisting of ammonia, primary amines, secondary amines, and combinations thereof.

The catalyst of the present application shows an improved performance, particularly improved catalytic activity, reaction conversion, product selectivity and/or catalyst stability, when used for producing organic amines by catalytic amination.

Other characteristics and advantages of the present application will be described in detail in the detailed description hereinbelow.

Detailed Description of the Invention

The present application will be further described hereinafter in detail with reference to specific embodiments thereof. It should be noted that the specific embodiments of the present application are provided for illustration purpose only, and are not intended to be limiting in any manner.

Any specific numerical value, including the endpoints of a numerical range, described in the context of the present application is not restricted to the exact value thereof, but should be interpreted to further encompass all values close to said exact value, for example all values within $\pm$5% of said exact value. Moreover, regarding any numerical range described herein, arbitrary combinations can be made between the endpoints of the range, between each endpoint and any specific value within the range, or between any two specific values within the range, to provide one or more new numerical range(s), where said new numerical range(s) should also be deemed to have been specifically described in the present application.

Unless otherwise stated, the terms used herein have the same meaning as commonly understood by one skilled in the art; and if the terms are defined herein and their definitions are different from the ordinary understanding in the art, the definition provided herein shall prevail.

In the present application, the ammonia adsorption capacity of the carrier and the catalyst is measured by $NH_3$-TPD test, wherein the ammonia adsorption capacity is expressed as the measured ammonia desorption amount.

In the present application, the specific surface area, pore volume and proportion of pores having different pore diameters of the carrier are measured by a nitrogen adsorption-desorption method according to GB/T6609.35-2009.

In the present application, the expression "C2-20" means having 2 to 20 carbon atoms, for example having 2, 3, 4, 5, 6, 7, 8, 9, 10, 11, 12, 13, 14, 15, 16, 17, 18, 19 or 20 carbon atoms. Similarly, the expression "C1-12" means having 1-12 carbon atoms.

In the present application, the grain sizes of the active metal component and the metal promoter are measured by XRD analysis.

In the present application, unless otherwise indicated, the pressures given are gauge pressures.

In the context of the present application, in addition to those matters explicitly stated, any matter or matters not mentioned are considered to be the same as those known in the art without any change. Moreover, any of the embodiments described herein can be freely combined with another one or more embodiments described herein, and the technical solutions or ideas thus obtained are considered as part of the original disclosure or original description of the present application, and should not be considered to be a new matter that has not been disclosed or anticipated herein, unless it is clear to one skilled in the art that such a combination is obviously unreasonable.

All of the patent and non-patent documents cited herein, including but not limited to textbooks and journal articles, are hereby incorporated by reference in their entirety.

As described above, in a first aspect, the present application provides a catalyst useful for producing organic amines by catalytic amination, comprising an inorganic porous carrier containing aluminum and/or silicon, and an active metal component supported on the carrier, the active metal component comprising at least one metal selected from Group VIII and Group IB metals, wherein the carrier has an ammonia adsorption capacity of 0.25 to 0.65 mmol/g as measured by $NH_3$-TPD test.

According to the present application, the Group VIII metal may be, for example, cobalt, nickel or palladium, and the Group IB metal may be, for example, copper. In a preferred embodiment, the metal in the active metal component is selected from cobalt, nickel, palladium, copper or a combination thereof, more preferably selected from cobalt, nickel or a combination thereof.

In the catalyst of the present application, the Group IB metal, such as copper, can be used alone as the active metal component, in which case it is typically used in a relatively larger amount; it may also be used in combination with a Group VIII metal, in which case it is typically used in a relatively smaller amount. When used in combination with a Group VIII metal, such as cobalt, nickel and palladium, the Group IB metal is normally referred to herein as a metal promoter.

In a preferred embodiment, the carrier has an ammonia adsorption capacity of 0.3 to 0.6 mmol/g as measured by NH$_3$-TPD test.

In a preferred embodiment, the carrier comprises a matrix comprising a first carrier component and optionally a second carrier component, and a doping element, wherein the first carrier component is selected from alumina, silica, molecular sieves, aluminosilicates or combinations thereof, the second carrier component is selected from diatomite and titania, and the doping element is selected from a metallic element, a non-metallic element, or a combination thereof, excluding sodium and chlorine, the metallic element is at least one selected from the group consisting of Group IA metallic elements, Group IIA metallic elements, Group VA metallic elements, and lanthanide series metallic elements, preferably at least one selected from the group consisting of calcium, magnesium, potassium, bismuth, strontium, barium, and lanthanum; the non-metallic element is at least one selected from the group consisting of Group IIIA non-metallic elements, Group VA non-metallic elements, Group VIA non-metallic elements and Group VIIA non-metallic elements, and preferably at least one selected from the group consisting of boron, fluorine, phosphorus, sulfur and selenium.

In a further preferred embodiment, the doping element in the carrier is derived from metal cations and acid radical ions, but does not include sodium ion or chloride ion; the metal cation is at least one selected from the group consisting of Group IA metal cations, Group IIA metal ions, Group VA metal ions and lanthanide series metal ions, preferably at least one selected from the group consisting of calcium ion, magnesium ion, potassium ion, bismuth ion, strontium ion, barium ion and lanthanum ion; the acid radical ion is at least one selected from non-metallic acid radical ions, preferably at least one selected from the group consisting of borate ion, fluoride ion, phosphate ion, sulfate ion and selenate ion.

In a preferred embodiment, the carrier has at least one of the following characteristics:

the carrier has a carbon dioxide adsorption capacity of 0.05-0.4 mmol/g, preferably 0.05-0.3mmol/g, more preferably 0.06-0.2 mmol/g, and where the carrier has a carbon dioxide adsorption capacity of within the above-described range, it is favorable to improve the product selectivity of the catalyst and reduce the generation of byproducts;

the doping element is present in the carrier in an amount of 0.03 to 6 wt%, preferably 0.05 to 6 wt%, more preferably 0.08 to 4 wt%, relative to the total weight of the matrix;

the carrier has a specific surface area of 120-240 m$^2$/g, preferably 120-210 m$^2$/g, and more preferably 125-200 m$^2$/g;

the carrier has a pore volume of 0.45-1.2 ml/g, preferably 0.45-1.1 ml/g, and more preferably 0.5-1 ml/g;

the proportion of the pore volume of pores having a pore diameter in a range of 7-27 nm to the pore volume of the carrier is greater than 65%, preferably more than or equal to 70%, more preferably 70-90%, and the proportion of the pore volume of pores having a pore diameter less than 7 nm to the pore volume of the carrier is preferably 0-10%, for example 0-8%; where the carrier has the above-described pore diameter distribution, it is favorable to increase the surface diffusivity of the catalyst and improve the activity and the product selectivity of the catalyst;

the matrix of the carrier comprises a combination of alumina and titania at a weight ratio of 1.5-5 : 1, preferably 2-4.5 : 1; and

the alumina content in the carrier is 70 wt% or more, preferably 75 wt% or more, more preferably 80-100 wt%, based on the total weight of the matrix.

In a preferred embodiment of the catalyst of the present application, the active metal component is present in an amount of 5 to 46 g, preferably 10 to 42 g, for example 13 to 40 g, per 100g of the matrix.

In a preferred embodiment, the catalyst further comprises a metal promoter supported on the carrier, and the metal promoter comprises at least one metal selected from the group consisting of Group VIB, Group VIIB, Group IB, Group IIB and lanthanide series metals, preferably comprises at least one metal selected from the group consisting of Cr, Mo, W, Mn, Re, Cu, Ag, Au, Zn, La and Ce. Further preferably, the metal promoter is present in an amount of 0 to 10g, preferably 0.1 to 10g, more preferably 0.5 to 8 g, per 100 g of the matrix.

In some further preferred embodiments, the metal promoter comprises a combination of at least one Group VIIB metal and at least one Group IB metal, wherein the weight ratio of the Group VIIB metal to the Group IB metal, calculated as metal element, is 0.05-15 : 1, preferably 0.1-12 : 1; or the metal promoter comprises a combination of at least one Group VIIB metal and at least one Group IIB metal, wherein the weight ratio of the Group VIIB metal to the Group IIB metal is 0.2-20 : 1, preferably 0.3 to 6 : 1; or the metal promoter comprises a combination of at least one Group VIB metal, at least one Group IB metal and at least one Group IIB metal at a weight ratio of the Group VIB metal to the Group IB metal and to the Group IIB metal of 0.1-10 : 0.1-10 : 1, preferably 0.2-8 : 0.2-8 : 1. Particularly preferably, the Group VIIB metal is selected from manganese and/or rhenium, the Group IB metal is at least one selected from the group consisting of copper, silver and gold, the Group IIB metal is selected from zinc, and the Group VIB metal is selected from molybdenum and/or tungsten.

According to the present application, the carrier for the catalyst can be obtained by methods known in the art useful for producing carriers having the above-mentioned properties, to which there is no particular limitation in the present application. Preferably, the carrier can be prepared by a method comprising the following steps: subjecting a mixture comprising a doping element and a matrix or a precursor thereof to shaping, drying and calcining sequentially to obtain the carrier, wherein the matrix comprises a first carrier component and optionally a second carrier component, the first carrier component is selected from alumina, silica, molecular sieves, aluminosilicates or combinations thereof, and the second carrier component is selected from diatomite, titanium white powder or a combination thereof. The molecular sieve may be, for example, a ZSM-5 or ZSM-11 molecular sieve. Where a precursor of the matrix is used, the precursor of alumina may be pseudo-boehmite, and the precursor of silica may be silicic acid, orthosilicic acid, or silica gel.

In the above method for producing the carrier, the precursor of the matrix is preferably pseudo-boehmite. The pseudo-boehmite may be prepared by at least one of carbonization method, organoaluminum hydrolysis method, aluminum sulfate method, and nitric acid method. The specific surface area of the pseudo-boehmite is preferably 250-410 $m^2$/g, more preferably 260-400 $m^2$/g, and further preferably 260-380 $m^2$/g, such as 250-330 $m^2$/g or 265-410 $m^2$/g; the pseudo-boehmite preferably has a pore volume of 0.7 to 1.3 ml/g, more preferably 0.7 to 1.2 ml/g, further preferably 0.8 to 1.2 ml/g, for example 0.8 to 1.3 ml/g or 0.78 to 1.2 ml/g. A catalyst with better performance can be obtained by using the pseudo-boehmite with a specific pore structure.

In the above method for producing the carrier, where the raw material providing the precursor of the matrix already contains a desired amount of the doping element, the shaping may be simply performed using such raw material, and where the raw material providing the precursor of the matrix does not contain the doping element or the content of the doping element is low (insufficient), an introduction of additional doping element may be performed.

In the above method for producing the carrier, the doping element may be provided using a carrier modifier comprising at least one compound capable of providing a cation (excluding sodium ion) and/or an anion (excluding chloride ion), wherein the cation is at least one selected from the group consisting of Group IA cations, Group IIA metal ions, Group VA metal ions, and lanthanide series metal ions, preferably at least one selected from the group consisting of calcium ion, magnesium ion, potassium ion, bismuth ion, strontium ion, barium ion, and lanthanum ion; the anion is at least one selected from the group consisting of non-metallic acid radical ions, preferably at least one selected from the group consisting of borate ion, fluoride ion, phosphate ion, sulfate ion and selenate ion.

Preferably, the carrier modifier is at least one selected from the group consisting of boric acid, nickel borate, cobalt borate, potassium borate, hydrofluoric acid, potassium fluoride, cobalt fluoride, nickel fluoride, phosphoric acid, aluminum phosphate, tripotassium phosphate, potassium dihydrogen phosphate, potassium hydrogen phosphate, magnesium phosphate, calcium phosphate, sulfuric acid, cobalt sulfate, nickel sulfate, aluminum sulfate, calcium sulfate, bismuth nitrate, potassium nitrate, potassium sulfate, potassium carbonate, magnesium nitrate, magnesium sulfate, basic magnesium carbonate, calcium nitrate, basic calcium carbonate, strontium nitrate, strontium phosphate, strontium sulfate, barium nitrate, lanthanum nitrate, and selenic acid.

In the above method for producing the carrier, the method for shaping may be selected from kneading, rolling, flaking, or the like.

In the above method for producing the carrier, the carrier modifier is used in such an amount that the doping element is present in an amount of 0.03 to 6 wt%, preferably 0.05 to 6 wt%, more preferably 0.08 to 4 wt%, for example 0.08 to 1 wt% (e.g., may be 0.08 wt%, 0.1 wt%, 0.2 wt%, 0.3 wt%, 0.4 wt%, 0.45 wt%, 0.5 wt%, 0.55 wt%, 0.6 wt%, 0.7 wt%, 0.8 wt%, 0.85 wt%, 0.9 wt%, 0.95 wt%, 1 wt%, or a value between any two of them), relative to the total weight of the matrix. One skilled in the art can determine the amount of the raw material (e.g., the carrier modifier) for a component based on the amount of said component in the final carrier, and therefore, the amounts of some raw materials are not given herein.

In the above method for producing the carrier, the drying conditions may include: a temperature of 80-150 °C (e.g., 80 °C, 85 °C, 90 °C, 95 °C, 100 °C, 110 °C, 115 °C, 120 °C, 125 °C, 130 °C, 140 °C, 150 °C, or a value between any two of them), such as 100-150°C, 80-120°C or 100-120°C, a drying time of 6-20 h (e.g., 6h, 7 h, 7.5 h, 8 h, 8.5 h, 9 h, 9.5 h, 10 h, 10.5 h, 10 h, 11 h, 11.5 h, 12 h, 12.5 h, 13 h, 14 h, 14.5 h, 15 h, 15.5 h, 16 h, 17 h, 18 h, 19 h, 20 h, or a value between any two of them), such as 10-20 h, 5-15 h or 8-12 h.

In the above method for producing the carrier, the calcining conditions may include: a temperature of 500-1100 °C (e.g., 600 °C, 650 °C, 680 °C, 700 °C, 750 °C, 800 °C, 850 °C, 900 °C, 950 °C, 990 °C, 1000 °C, 1050 °C, 1100 °C, or a value between any two of them), such as 600-1100 °C, 550-1050 °C, 530-1000 °C or 550-1000 °C, and a calcining time of 2-20h (e.g., 2 h, 2.5 h, 3 h, 3.5 h, 4 h, 4.5 h, 5 h, 5.5 h, 6 h, 7 h, 8 h, 9 h, 9.5 h, 10 h, 10.5 h, 11 h, 12 h, 15 h, 18 h, 20h, or a value between any two of them), such as 4-20 h, 4-10 h or 5-8 h.

The catalyst of the present application can be used after reduction, for example, it can be reduced by using a hydrogen-containing gas at 350-500 °C, preferably at 350-450 °C, such as 400-450 °C. The hydrogen-containing gas may be pure hydrogen gas or hydrogen gas diluted with an inert gas, such as a mixture of nitrogen and hydrogen. The reduction temperature is gradually increased during the reduction, and the temperature rise is preferably not too fast,

EP 4 238 648 A1

for example, not more than 20 °C per hour. The reduction time can be determined by monitoring the generation of $H_2O$ in the reduction system, that is when no new $H_2O$ is generated in the reduction system, the reduction is terminated, and one skilled in the art can select the reduction time accordingly, of which the detailed description is omitted herein for brevity, for example, the reduction time can be 2-5h at the highest temperature. The reduction may be carried out directly in the reactor, followed by the catalytic reaction. It is also possible to carry out the reduction in a separate reactor, also referred to as out-of-reactor reduction, and a passivation may be carried out after the reduction with a gas mixture comprising oxygen before the catalyst being discharged from the reactor, the passivation temperature being, for example, from 10 to 60 °C and particularly from 20 to 40 °C. The catalyst undergone the out-of-reactor reduction and passivation can be activated before use using hydrogen or a mixture of hydrogen and nitrogen at a temperature of, for example, 150-250 °C, preferably 170-240 °C, such as 170-200 °C. The activation time can be determined by monitoring the generation of $H_2O$ in the activation system, that is when no new $H_2O$ is generated in the activation system, the activation is terminated and one skilled in the art can select the activation time accordingly, of which the detailed description is omitted herein for brevity. For example, the activation time at the highest temperature may be, for example, 1 to 5 hours, preferably 2 to 3 hours, or the catalyst can be used without activation, depending on the extent to which the active metal component and metal promoter of the catalyst have been oxidized.

In a second aspect, there is provided a method for producing the catalyst of the present application, comprising the steps of:

1) providing an inorganic porous carrier containing aluminum and/or silicon, which has an ammonia adsorption capacity of 0.25 to 0.65 mmol/g as measured by $NH_3$-TPD test;
2) loading the active metal component and optionally the metal promoter on the carrier; and
3) carrying out a heat treatment and optionally a reduction treatment on the material obtained in step 2) to obtain the catalyst,

In a preferred embodiment, the carrier has an ammonia adsorption capacity of 0.3 to 0.6 mmol/g as measured by $NH_3$-TPD test.

In a preferred embodiment, said "providing an inorganic porous carrier containing aluminum and/or silicon" of step 1) comprises subjecting a mixture comprising a doping element and a matrix or a precursor thereof to shaping, drying and calcining sequentially to obtain the carrier, wherein the matrix comprises a first carrier component and optionally a second carrier component, the first carrier component is selected from alumina, silica, molecular sieves, aluminosilicates or combinations thereof, the second carrier component is selected from diatomite, titanium white powder or a combination thereof, preferably the first carrier component is alumina, the precursor of the first carrier component is a pseudo-boehmite having a specific surface area of 250-410 $m^2$/g, preferably 260-400 $m^2$/g, more preferably 260-380 $m^2$/g and a pore volume of 0.7-1.3 ml/g, preferably 0.7-1.2 ml/g, more preferably 0.8 to 1.2 ml/g; the doping element is selected from a metallic element, a non-metallic element or a combination thereof, excluding sodium and chlorine, and the metallic element is at least one selected from the group consisting of Group IA metal elements, Group IIA metal elements, Group VA metal elements and lanthanide series metal elements, preferably at least one selected from the group consisting of calcium, magnesium, potassium, bismuth, strontium, barium and lanthanum; the non-metallic element is at least one selected from the group consisting of Group IIIA non-metallic elements, Group VA non-metallic elements, Group VIA non-metallic elements and Group VIIA non-metallic elements, and preferably at least one selected from the group consisting of boron, fluorine, phosphorus, sulfur and selenium.

In a further preferred embodiment, the doping element is provided using a carrier modifier, and the doping element and the carrier modifier may be selected as described above in the first aspect, of which the detailed description is omitted herein for brevity. Still more preferably, the carrier modifier is used in such an amount that the resulting carrier has a doping element content of 0.03 to 6 wt%, preferably 0.05 to 6 wt%, more preferably 0.08 to 4 wt%, for example 0.08 to 1 wt% (e.g., 0.08 wt%, 0.1 wt%, 0.2 wt%, 0.3 wt%, 0.4 wt%, 0.45 wt%, 0.5 wt%, 0.55 wt%, 0.6 wt%, 0.7 wt%, 0.8 wt%, 0.85 wt%, 0.9 wt%, 0.95 wt%, 1 wt%, or a value between any two of them), relative to the total weight of the matrix.

In a further preferred embodiment, the method for shaping may be selected from kneading, rolling, flaking, or the like.

In a further preferred embodiment, the drying conditions of step 1) include: a temperature of 80-150 °C, and a drying time of 6-20 h; and/or the calcining conditions include: a temperature of 500-1100 °C, and a calcining time of 2-20 h.

In a further preferred embodiment, the step 1) has the characteristics as described above for the method for producing the carrier in the first aspect, of which the detailed description is omitted herein for brevity.

In a preferred embodiment, said loading of step 2) comprises impregnating said carrier with a solution comprising a precursor of said active metal component and optionally a precursor of said metal promoter, preferably the impregnation solution has a pH in a range of 3.5-5.5. Controlling the pH of the impregnation solution within the above range can further improve the dispersibility of the active metal component.

According to the present application, the impregnation is carried out by immersing the carrier in an appropriate solution comprising precursors of said active metal component and metal promoter, so that the precursor is loaded onto the carrier by adsorption. The method for impregnation may be classified as dry impregnation, wet impregnation, multiple impregnation, mixed impregnation, spray impregnation and the like. The dry impregnation and wet impregnation respectively mean impregnating a carrier that is in a dry state or wetted with water in advance with the precursor of the active metal component. The multiple impregnation means impregnating with a mixed solution of the precursor(s) of one or more components in multiple times or impregnating in multiple times with different precursors, and in the multiple impregnation, drying and calcining need to be performed after each time of impregnation to "anchor" the impregnated components. The mixed impregnation means impregnating with the precursors of the active metal component and the metal promoter together, where no precipitation reaction would occur between those precursors. The spray impregnation means spraying an impregnation solution onto a continuously rotating carrier by a spray gun so that the impregnation solution just fills the pore volume of the carrier to saturation. These impregnation methods can be appropriately selected according to the actual conditions for the preparation of the catalyst of the present application.

In a preferred embodiment, the precursors of the active metal component and the metal promoter are soluble salts of the corresponding metals, such as nitrates, formates, oxalates, lactates, and the like. The solvent used to form the metal salt solution for impregnating the carrier is preferably water, although some organic solvents may be used, such as ethanol. The impregnation of the carrier with the metal salt solution can be carried out in any desired sequence, or continuously with a plurality of solutions containing one or more metal salts. All impregnation steps or a single impregnation step may be carried out in several stages, and the order of impregnation may also be varied. The concentration of the solution is selected so that a desired amount of metal is loaded on the carrier.

According to the present application, the carrier loaded with the active metal component and optionally the metal promoter is subjected to a heat treatment in step 3), said heat treatment preferably comprising calcining or a combination of drying and calcining. For example, the heat treatment may comprise drying the impregnated carrier at 80 to 150 °C, more preferably 80 to 120 °C. The drying time can be appropriately selected according to the drying temperature, the amount of the material to be dried, the drying equipment and the like, and can be, for example, 6 to 20 hours, such as 8 hours, as long as the moisture content after drying does not affect the subsequent calcining. Further, the drying may be followed by calcining at 150-500 °C to remove the water of crystallization in the salt or to decompose the salt into oxides, preferably at 300-500 °C for 1-6 h. In the case of multiple impregnations, it is preferable to carry out drying and calcining after each impregnation.

In the present application, the loading of the active metal component and the metal promoter has no substantial impact on the microstructure of the catalyst, and therefore, the catalyst obtained has a similar pore structure to that of the carrier.

In a third aspect, the present application provides a carrier which is an inorganic porous material containing aluminum and/or silicon, wherein the carrier has an ammonia adsorption capacity of 0.25 to 0.65 mmol/g as measured by $NH_3$-TPD test.

In a preferred embodiment, the carrier has an ammonia adsorption capacity of 0.3 to 0.6 mmol/g as measured by $NH_3$-TPD test.

In a preferred embodiment, the carrier comprises a matrix comprising a first carrier component and optionally a second carrier component, and a doping element, wherein the first carrier component is selected from alumina, silica, molecular sieves, aluminosilicates or combinations thereof, the second carrier component is selected from diatomite and titania, and the doping element is selected from a metallic element, a non-metallic element, or a combination thereof, excluding sodium and chlorine, the metallic element is at least one selected from the group consisting of Group IA metallic elements, Group IIA metallic elements, Group VA metallic elements, and lanthanide series metallic elements, preferably at least one selected from the group consisting of calcium, magnesium, potassium, bismuth, strontium, barium, and lanthanum; the non-metallic element is at least one selected from the group consisting of Group IIIA non-metallic elements, Group VA non-metallic elements, Group VIA non-metallic elements and Group VIIA non-metallic elements, and preferably at least one selected from the group consisting of boron, fluorine, phosphorus, sulfur and selenium.

In a further preferred embodiment, the doping element in the carrier is derived from metal cations and acid radical ions, but does not include sodium ion or chloride ion; the metal cation is at least one selected from the group consisting of Group IA metal cations, Group IIA metal ions, Group VA metal ions and lanthanide series metal ions, preferably at least one selected from the group consisting of calcium ion, magnesium ion, potassium ion, bismuth ion, strontium ion, barium ion and lanthanum ion; the acid radical ion is at least one selected from non-metallic acid radical ions, preferably at least one selected from the group consisting of borate ion, fluoride ion, phosphate ion, sulfate ion and selenate ion.

In a preferred embodiment, the carrier has at least one of the following characteristics:

the carrier has a carbon dioxide adsorption capacity of 0.05 to 0.4 mmol/g, preferably 0.05 to 0.3 mmol/g, more preferably 0.06 to 0.2 mmol/g,
the doping element is present in the carrier in an amount of 0.03 to 6 wt%, preferably 0.05 to 6 wt%, more preferably 0.08 to 4 wt%, relative to the total weight of the matrix,
the carrier has a specific surface area of 120-240 $m^2/g$, preferably 120-210 $m^2/g$, and more preferably 125-200 $m^2/g$;
the carrier has a pore volume of 0.45-1.2 ml/g, preferably 0.45-1.1 ml/g, and more preferably 0.5-1 ml/g;
the proportion of the pore volume of pores having a pore diameter in a range of 7-27 nm to the pore volume of the carrier is greater than 65%, preferably 70% or more, more preferably 70 to 90%, and preferably the proportion of the pore volume of pores having a pore diameter of less than 7 nm to the pore volume of the carrier is 0 to 10%, for example 0 to 8%;
the matrix of the carrier comprises a combination of alumina and titania at a weight ratio of 1.5-5 : 1, preferably 2-4.5 : 1; and
the alumina content in the carrier is 70 wt% or more, preferably 75 wt% or more, more preferably 80-100 wt%, based on the total weight of the matrix.

In a fourth aspect, the present application provides the use of the catalyst according to the present application or the carrier according to the present application for producing organic amines by catalytic amination.

In a fifth aspect, the present application provides a process for producing organic amines, comprising: in the presence of hydrogen, contacting an amination raw material and an amination reagent with the catalyst according to the present application for amination reaction to obtain an organic amine.

In a preferred embodiment, the amination raw material is selected from the group consisting of alcohols, ketones, alcohol amines, aldehydes, and combinations thereof, more preferably selected from the group consisting of C2-20 alcohols, C3-20 ketones, C2-20 alcohol amines, C2-20 aldehydes, and mixtures thereof. Further preferably, the amination raw material is selected from the group consisting of ethanol, acetaldehyde, n-propanol, propionaldehyde, isopropanol, n-butanol, butyraldehyde, isobutanol, isobutyraldehyde, 2-ethylhexanol, 2-ethylhexaldehyde, octanol, octanal, dodecanol, dodecanal, hexadecanol, hexadecanal, cyclopentanol, cyclohexanol, cyclooctanol, cyclododecanol, benzyl alcohol, benzaldehyde, phenethyl alcohol, phenylacetaldehyde, 1,4-butanediol, 1,4-butanedial, 1,5-pentanediol, 1,5-glutaraldehyde, 1,6-hexanediol, 1,6-hexanedial, 1,8-octanediol, 1,8-octanedial, 1,12-dodecanediol, 1,12-dodecanedialdehyde, ethanolamine, propanolamine, isopropanolamine, 6-aminohexanol, diethanolamine, diisopropanolamine, dimethylethanolamine, acetone, ethylene glycol, 1,3-propanediol, and mixtures thereof.

In the present application, the amination reagent refers to a reactant capable of providing an amino group and/or an amine group. Preferably, the amination reagent is selected from the group consisting of ammonia, primary amines, secondary amines, and combinations thereof, more preferably selected from the group consisting of ammonia, C1-12 primary amines, C2-12 secondary amines, and mixtures thereof, such as at least one of alkyl amine, cycloalkyl amine, and aralkyl amine, and even more preferably a C1-4 alkyl amine. Particularly preferably, the amination reagent is selected from the group consisting of ammonia, monomethylamine, dimethylamine, methylethylamine, monoethylamine, diethylamine and mixtures thereof.

In a preferred embodiment, the amination conditions include: a molar ratio of hydrogen to the amination reagent and to the amination raw material of 1-5 : 2-35 : 1, preferably 1-5 : 2-33 : 1, more preferably 1-5 : 2-30 : 1, a temperature of 105-220 °C, preferably 110-220 °C, more preferably 130-200 °C, a pressure of 0.7-25MPa, preferably 0.8-25 MPa, more preferably 1-15 MPa, and a liquid phase volume space velocity of the amination raw material of 0.06-1 $m^3/(m^3 \cdot h)$;

In some preferred embodiments, the amination raw material is a monohydric alcohol and the amination conditions include: a molar ratio of hydrogen to the amination reagent and to the amination raw material of 1-4 : 2-10 : 1, preferably 1-4 : 2-9 : 1, more preferably 1-4 : 2-8 : 1, a temperature of 130-210 °C, preferably 130-200 °C, a pressure of 1-4 MPa, preferably 1-3.5 MPa, more preferably 1-2.5 MPa, and a liquid phase volume space velocity of the amination raw material of 0.1-0.8 $m^3/(m^3 \cdot h)$.

In some preferred embodiments, the amination raw material is a ketone or an aldehyde and the amination conditions include: a molar ratio of hydrogen to the amination reagent and to the amination raw material of 1-4 : 2-6 : 1, preferably 1-4 : 2-5 : 1, a temperature of 105-180 °C, preferably 110-180 °C, more preferably 110-170 °C, a pressure of 0.7-3.5 MPa, preferably 0.7-2.5 MPa, more preferably 0.8-2.5 MPa, and a liquid phase volume space velocity of the amination raw material of 0.1-1 $m^3/(m^3 \cdot h)$, preferably 0.1-0.8 $m^3/(m^3 \cdot h)$.

In some preferred embodiments, the amination raw material is an alcohol amine and the amination conditions include: a molar ratio of hydrogen to the amination reagent and to the amination raw material of 1-4 : 3-25 : 1, preferably 1-4 : 3-20 : 1, a temperature of 130-200 °C, preferably 135-200 °C, a pressure of 1-18 MPa, preferably 1-15 MPa, more preferably 1-11 MPa, and a liquid phase volume space velocity of the amination raw material of 0.06-0.8 $m^3/(m^3 \cdot h)$.

In some preferred embodiments, the amination raw material is a dihydric alcohol and the amination conditions include: a molar ratio of hydrogen to the amination reagent and to the amination raw material of 0.3-4 : 3-45 : 1, preferably 1-4 : 3-35 : 1, more preferably 1-4 : 3-33 : 1, a temperature of 130-220°C, preferably 130-210°C, a pressure of 1-15 MPa, preferably 4-25 MPa, and a liquid phase volume space velocity of the amination raw material of 0.06-0.8 $m^3/(m^3 \cdot h)$, preferably 0.1-0.8 $m^3/(m^3 \cdot h)$.

In some preferred embodiments, the amination raw material is a mixture of 1,6-hexanediol, cycloheximide, and 6-amino-1-hexanol, and the amination conditions include: a molar ratio of hydrogen to the amination reagent and to the amination raw material of 0.3-4 : 3-45 : 1, preferably 1-4 : 3-35 : 1, more preferably 1-4 : 3-33 : 1, further preferably 1-4 : 3-30 : 1, a temperature of 130-220°C, preferably 130-210°C, a pressure of 1-25 MPa, preferably 2-25 MPa, more preferably 4-25 MPa, and a liquid phase volume space velocity of the amination raw material of 0.06-0.8 $m^3/(m^3 \cdot h)$, preferably 0.1-0.8 $m^3/(m^3 \cdot h)$.

First type of embodiments

In the first type of embodiments of the present application, there is provided a catalyst having a function of catalyzing the hydroamination of alcohols to produce organic amines, the catalyst comprising a carrier and an active metal component and optionally a metal promoter supported on the carrier, characterized in that the carrier is at least one selected from the group consisting of doped alumina, doped silica, doped molecular sieves and doped aluminosilicates; the carrier has an ammonia adsorption capacity of 0.25-0.6 mmol/g, and the carrier has a carbon dioxide adsorption capacity of 0.05-0.3 mmol/g; the active metal component is cobalt and/or nickel.

The catalyst of the first type of embodiments of the present application has a high catalytic activity and a high selectivity when used for the hydroamination reaction. For example, when used for the hydroamination of ethanol, the catalyst has a high reaction activity and a high selectivity for producing ethylamine, with a less production of methyl ethylamine, methyl diethylamine, ethyl-n-propylamine and ethyl-sec-butylamine. When the catalyst is used for the hydroamination of 1,6-hexanediol, the production of heavy components and other impurities is less, and the selectivity of the hexanediamine generated is high. After a long-period of life evaluation, it is found that the catalyst of the first type of embodiments of the present application has a stable catalytic performance, by controlling the acidity and the alkalinity of the catalyst within an appropriate range, the adsorption-desorption performance of the catalyst is improved, so that the diffusion of the reaction system is promoted, the reaction rate is accelerated, the carbon deposition is reduced, and the blockage of pore channel is retarded.

Preferably, the carrier has an ammonia adsorption capacity of 0.3 to 0.5 mmol/g, more preferably 0.3 to 0.42 mmol/g.

Preferably, the carrier has a carbon dioxide adsorption capacity of 0.06 to 0.2 mmol/g, more preferably 0.06 to 0.17 mmol/g.

Preferably, the carrier comprises a matrix that is at least one selected from the group consisting of alumina, silica, molecular sieves and aluminosilicates, and a doping element comprising a metallic element and a non-metallic element. Preferably, the weight ratio of the metallic element to the non-metallic element may be 1 : 0.05-50, more preferably 1 : 0.2-8.

More preferably, the metallic element is at least one selected from the group consisting of Group IA metal elements, Group IIA metal elements, Group VA metal elements, and lanthanide series metal elements, and further preferably at least one selected from the group consisting of calcium, magnesium, potassium, bismuth, strontium, barium, and lanthanum.

More preferably, the non-metallic element is at least one selected from the group consisting of Group IIIA non-metallic elements, Group VA non-metallic elements, Group VIA non-metallic elements, and Group VIIA non-metallic elements, and further preferably at least one selected from the group consisting of boron, fluorine, phosphorus, sulfur, and selenium.

More preferably, the doping element in the carrier is derived from metal cations and acid radical ions, but does not include sodium ion or chloride ion. Since the doping element is introduced during the production of the carrier, the doping element is mainly present in the bulk phase of the carrier. Further preferably, the metal cation may be at least one selected from the group consisting of Group IA metal cations, Group IIA metal ions, Group VA metal ions, and lanthanide series metal ions, further preferably at least one selected from the group consisting of calcium ion, magnesium ion, potassium ion, bismuth ion, strontium ion, barium ion, and lanthanum ion; the acid radical ion may be at least one selected from non-metallic acid radical ions, and more preferably at least one selected from the group consisting of borate ion, fluoride ion, phosphate ion, sulfate ion, and selenate ion.

Preferably, the doping element is present in the carrier in an amount of 0.03 to 2 wt%, more preferably 0.08 to 1 wt%, relative to the total weight of the matrix.

Preferably, the carrier has a specific surface area of 120-240 $m^2/g$.

Preferably, the carrier has a pore volume of 0.5 to 1 ml/g.

According to the first type of embodiments of the present application, the active metal component may be present in an amount of 5 to 42g, preferably 10 to 35 g, more preferably 10 to 30 g, per 100g of the matrix.

According to the first type of embodiments of the present application, the catalyst may further comprise a metal promoter to better exert the performance of the catalyst, to optimize the proportion of reaction products, and to reduce unwanted side reactions. The metal promoter can be at least one selected from the group consisting of Group VIB, Group VIIB, Group IB, Group IIB and lanthanide series elements, preferably at least one of Cr, Mo, W, Mn, Re, Cu, Ag, Au, Zn, La and Ce. Preferably, the metal promoter may be present in an amount of 0 to 10g, preferably 0.5 to 6g, per 100 g of the matrix.

In the first type of embodiments of the present application, there is also provided a process for producing organic amines, comprising: contacting an amination raw material and an amination reagent with the catalyst as described above in the presence of hydrogen for amination reaction.

According to the first type of embodiments of the present application, the amination raw material or amination reagent can be selected as described above, of which the detailed description is omitted herein for brevity.

According to the first type of embodiments of the present application, the amination conditions may include: a mole ratio of hydrogen to the amination reagent and to the amination raw material of 1-5 : 2-35 : 1, a temperature of 130-200°C, a pressure of 1-15 MPa, and a liquid phase volume space velocity of the amination raw material of 0.06-1 $m^3/(m^3 \cdot h)$.

Preferably, the amination raw material is a monohydric alcohol, and the amination conditions include: a molar ratio of hydrogen to the amination reagent and to the amination raw material of 1-4 : 2-10 : 1, preferably 2-3 : 4-6 : 1, a temperature of 130-200 °C, preferably 160-180 °C, a pressure of 1-4 MPa, preferably 1-2 MPa, and a liquid phase volume space velocity of the amination raw material of 0.1-0.8 $m^3/(m^3 \cdot h)$, preferably 0.4-0.6 $m^3/(m^3 \cdot h)$.

Preferably, the amination raw material is a ketone or an aldehyde, and the amination conditions include: a mole ratio of hydrogen to the amination reagent and to the amination raw material of 1-4 : 2-6 : 1, a temperature of 110-180°C, a pressure of 1-3.5 MPa, and a liquid phase volume space velocity of the amination raw material of 0.1-1 $m^3/(m^3 \cdot h)$.

Preferably, the amination raw material is an alcohol amine, and the amination conditions include: a mole ratio of hydrogen to the amination reagent and to the amination raw material of 1-4 : 3-20 : 1, preferably 2-3 : 10-15 : 1, a temperature of 135-200 °C, preferably 170-190 °C, a pressure of 1-11 MPa, preferably 8-10 MPa, and a liquid phase volume space velocity of the amination raw material of 0.06-0.8 $m^3/(m^3 \cdot h)$, preferably 0.4-0.6 $m^3/(m^3 \cdot h)$.

Preferably, the amination raw material is a dihydric alcohol, and the amination conditions include: a molar ratio of hydrogen to the amination reagent and to the amination raw material of 0.3-4 : 3-45 : 1, preferably 1-4 : 3-35 : 1, more preferably 2-3 : 10-15 : 1, a temperature of 130-210°C, preferably 180-190°C, a pressure of 1-15 MPa, preferably 8-10 MPa, and a liquid phase volume space velocity of the amination raw material of 0.1-0.8 $m^3/(m^3 \cdot h)$, preferably 0.4-0.6 $m^3/(m^3 \cdot h)$.

Preferably, the amination raw material is a mixture of 1,6-hexanediol, hexamethyleneimine and 6-amino-1-hexanol (referred to as aminohexanol for short), and the amination conditions include: a molar ratio of hydrogen to the amination reagent and to the amination raw material of 0.3-4 : 3-45 : 1, preferably 1-4 : 3-35 : 1, more preferably 3-4 : 10-20 : 1, a temperature of 130-210°C, preferably 180-200°C, a pressure of 1-15 MPa, preferably 5-10 MPa, and a liquid phase volume space velocity of the amination raw material of 0.1-0.8 $m^3/(m^3 \cdot h)$, preferably 0.4-0.6 $m^3/(m^3 \cdot h)$.

Second type of embodiments

In the second type of embodiments of the present application, there is provided a catalyst having a function of producing amines by catalytic amination, comprising a carrier, and an active metal component and a metal promoter supported on the carrier, wherein the active metal component is cobalt and/or nickel, the metal promoter is a combination of at least one Group VIIB metal and at least one Group IB metal, and the carrier has an ammonia adsorption capacity of 0.3-0.6 mmol/g.

Preferably, the carrier has an ammonia adsorption capacity of 0.3 to 0.56 mmol/g, more preferably 0.35 to 0.45 mmol/g.

The catalyst of the second type of embodiments of the present application comprises a specific metal promoter, has a high catalytic activity, and at the same time, has a high selectivity.

Preferably, the carrier comprises a matrix comprising alumina and optionally an additional carrier selected from silica and/or molecular sieves, and a doping element. Further preferably, the doping element is present in the carrier in an amount of 0.05-6 wt%, more preferably 0.08-4 wt%, relative to the weight of the matrix.

Further preferably, the carrier is mainly composed of (doped) alumina, and may further comprise (doped) silica or the like, thereby further improving the pore structure and structural stability of the catalyst. Particularly preferably, the alumina content of the carrier is 75 wt% or more, preferably 80-100 wt%, based on the total weight of the matrix.

Preferably, the doping element in the carrier is a non-metallic element, preferably at least one selected from the group consisting of boron, fluorine, phosphorus, sulfur and selenium. Further preferably, the doping element is incorporated during the production of the carrier in the form of at least one selected from the group consisting of borate ion, fluoride ion, phosphate ion, sulfate ion and selenate ion.

Preferably, the proportion of the pore volume of pores having a pore diameter in a range of 7-27 nm to the pore volume of the carrier is 70 to 90%.

Preferably, the proportion of the pore volume of pores having a pore diameter less than 7 nm to the pore volume of the carrier is 0-8%.

Preferably, the proportion of the pore volume of pores having a pore diameter of more than 27 nm to the pore volume of the carrier is 15-30%.

Preferably, the carrier has a specific surface area of 125-200 $m^2/g$.

Preferably, the carrier has a pore volume of 0.45 to 1.1 ml/g.

Preferably, the active metal component may be present in an amount of 8 to 45 g (e.g., may be any of 8, 9, 15, 18, 20, 23, 25, 26, 30, 35, 36, 38, 40, 42, 45, or a value between any two of them), per 100g of the matrix.

Preferably, the metal promoter may be present in an amount of 0.1 to 10g (e.g., may be any of 0.1, 0.5, 1, 2, 3, 4, 5, 6, 7, 8, 8.5, 8.7, 9, 10, or a value between any two of them), per 100 g of the matrix.

According to the second type of embodiments of the present application, the catalyst further comprises a metal promoter to better exert the performance of the catalyst, to optimize the proportion of reaction products, and to reduce unwanted side reactions. The metal promoter is a mixture of at least one Group VIIB metal and at least one Group IB metal, wherein the weight ratio of the Group VIIB metal to the Group IB metal in the metal promoter is preferably 0.05-15 : 1, more preferably 0.1-12 : 1, further more preferably 0.5-2 : 1. Preferably, the Group VIIB metal is selected from manganese and/or rhenium. Preferably, the Group IB metal is at least one selected from the group consisting of copper, silver, and gold. The inventors of the present application have found that a catalyst having a better catalytic effect can be obtained by using the preferred combination of metal promoter.

According to the second type of embodiments of the present application, the use of a carrier with a specific pore structure and ammonia adsorption capacity can provide a catalyst showing a high catalytic activity and a high selectivity when used for the hydroamination of alcohols; and producing few by-products including methylethylamine, methyldiethylamine, ethyl-n-propylamine, ethyl-sec-butylamine, and the like, when used for the hydroamination of ethanol. When the catalyst is used for the hydroamination of 1,6-hexanediol, the production of heavy components and other impurities is less. After a long-period of life evaluation, it is found that the catalyst has a stable catalytic performance, and by controlling the acidity (especially the ammonia adsorption capacity) of the catalyst within an appropriate range, the adsorption-desorption performance of the catalyst is improved, and by combining with a pore channel having a specific structure, the diffusion of the reaction system is promoted, the reaction rate is accelerated, the carbon deposition is reduced, and the blockage of pore channel is retarded.

In the second type of embodiments of the present application, there is also provided a process for producing organic amines, comprising: contacting an amination raw material and an amination reagent with the catalyst as described above in the presence of hydrogen for amination reaction.

According to the second type of embodiments of the present application, the amination raw material or amination reagent can be selected as described above, of which the detailed description is omitted herein for brevity.

According to the second type of embodiments of the present application, the amination conditions may include: a mole ratio of hydrogen to the amination reagent and to the amination raw material of 1-5 : 2-33 : 1, a temperature of 110-220°C, a pressure of 0.8-25 MPa, and a liquid phase volume space velocity of the amination raw material of 0.06-1 $m^3/(m^3 \cdot h)$.

Preferably, where the amination raw material is a monohydric alcohol, the amination conditions include: a mole ratio of hydrogen to the amination reagent and to the amination raw material of 1-4 : 2-10 : 1, a temperature of 130-210°C, a pressure of 1-3.5 MPa, and a liquid phase volume space velocity of the amination raw material of 0.1-0.8 $m^3/(m^3 \cdot h)$.

Preferably, where the amination raw material is a ketone or an aldehyde, the amination conditions include: a mole ratio of hydrogen to the amination reagent and to the amination raw material of 1-4 : 2-5 : 1, a temperature of 110-170°C, a pressure of 0.8-2.5 MPa, and a liquid phase volume space velocity of the amination raw material of 0.1-1 $m^3/(m^3 \cdot h)$.

Preferably, where the amination raw material is an alcohol amine, the amination conditions include: a mole ratio of hydrogen to the amination reagent and to the amination raw material of 1-4 : 3-25 : 1, a temperature of 130-200°C, a pressure of 1-18 MPa, and a liquid phase volume space velocity of the amination raw material of 0.06-0.8 $m^3/(m^3 \cdot h)$.

Preferably, where the amination raw material is a mixture of 1,6-hexanediol, hexamethyleneimine and 6-amino-1-hexanol or a dihydric alcohol, the amination conditions include: a mole ratio of hydrogen to the amination reagent and to the amination raw material of 1-4 : 3-33 : 1, a temperature of 130-220°C, a pressure of 4-25 MPa, and a liquid phase volume space velocity of the amination raw material of 0.06-0.8 $m^3/(m^3 \cdot h)$.

Third type of embodiments

In the third type of embodiments of the present application, there is provided a catalyst having a function of catalyzing hydroamination of alcohols, comprising a carrier, and a metal active component and a metal promoter supported on the carrier, wherein the metal active component is cobalt and/or nickel, the metal promoter is a combination of at least one Group VIIB metal and at least one Group IIB metal, and the carrier has an ammonia adsorption capacity of 0.3-0.7 mmol/g.

The catalyst of the third type of embodiments of the present application comprises a specific metal promoter, has a high catalytic activity, and at the same time, has a high selectivity and produces few byproducts.

Preferably, the carrier has an ammonia adsorption capacity of 0.3-0.6 mmol/g and a carbon dioxide adsorption capacity of 0.05-0.3 mmol/g.

Preferably, the carrier comprises a matrix and a doping element, the matrix comprises an alumina carrier and optionally an additional carrier that is at least one selected from the group consisting of silica, molecular sieves and diatomite. Further preferably, the doping element is present in the carrier in an amount of 0.05 to 6 wt%, more preferably 0.08 to 4 wt%, relative to the total weight of the matrix.

More preferably, the carrier is mainly composed of (doped) alumina carrier, and may further comprise (doped) silica and the like, thereby further improving the channel diffusivity and the pore structure stability of the catalyst. Particularly preferably, the content of the alumina carrier in the carrier is 70 wt% or more, preferably 80 to 100 wt%, based on the total weight of the matrix.

Preferably, the doping element in the carrier is selected from a metallic element and a non-metallic element, and does not include sodium or chlorine. The weight ratio of the metallic element to the non-metallic element is preferably 1: 0.1-40. The doping element is present in the carrier precursor or added during the production of the carrier, so that the doping element is mainly present in the bulk phase of the carrier.

Further preferably, the metallic element may be at least one selected from the group consisting of Group IA metal elements, Group IIA metal elements, Group VA metal elements, and lanthanide series metal elements, and further preferably at least one selected from the group consisting of calcium, magnesium, potassium, bismuth, strontium, barium, and lanthanum.

Further preferably, the non-metallic element may be derived from at least one non-metallic acid radical ion, further preferably derived from at least one selected from the group consisting of borate ion, fluoride ion, phosphate ion, sulfate ion, and selenate ion. The non-metallic element is at least one selected from the group consisting of boron, fluorine, phosphorus, sulfur and selenium.

Preferably, the proportion of the pore volume of pores having a pore diameter in a range of 7-27 nm to the pore volume of the carrier is greater than 65%, preferably 70 to 90%, more preferably 70 to 75%. More preferably, the proportion of the pore volume of pores having a pore diameter of less than 7 nm to the pore volume of the carrier is 0-10%, preferably 5-8%. More preferably, the proportion of the pore volume of pores having a pore diameter of more than 27 nm to the pore volume of the carrier is 10% to 30%, preferably 20% to 30%.

Preferably, the carrier has a specific surface area of 120-205 $m^2$/g.

Preferably, the carrier has a pore volume of 0.45 to 1.2 ml/g.

Preferably, the carrier has a carbon dioxide adsorption capacity of 0.05 to 0.4 mmol/g.

Preferably, the metal active component may be present in an amount of 14 to 46g (e.g. may be 14, 15, 20, 25, 30, 32, 35, 38, 40, 42, 45, 46, or a value between any two of them), per 100g of the matrix.

Preferably, the metal promoter may be present in an amount of 0.1 to 10g (e.g., may be 0.1, 0.5, 1, 1.2, 1.5, 1.8, 2, 3, 4, 5, 6, 7, 7.2, 7.5, 7.8, 8, 9, 10, or a value between any two of them), per 100 g of the matrix.

According to the second type of embodiments of the present application, the catalyst further comprises a metal promoter as described above to better exert the performance of the catalyst, to optimize the proportion of reaction products, and to reduce unwanted side reactions. The weight ratio of Group VIIB metal to Group IIB metal in the metal promoter is preferably 0.2-20 : 1, more preferably 0.3-6 : 1, more preferably 1-5 : 1. Preferably, the Group VIIB metal is selected from manganese and/or rhenium. Preferably, the Group IIB metal is selected from zinc.

According to the third type of embodiments of the present application, the use of a carrier having a specific pore structure, ammonia adsorption capacity and carbon dioxide adsorption capacity may allow the catalyst to show a high catalytic activity, and at the same time, show a high selectivity, when used for the hydroamination of alcohols; and when the catalyst is used for the hydroamination of n-propanol, the production of other impurities is little. When the catalyst is used for the hydroamination of 1,6-hexanediol, the production of heavy components and other impurities is less. After a long-period of life evaluation, it is found that the catalyst has a stable catalytic performance, and by controlling the acidity and the alkalinity of the catalyst within an appropriate range, the adsorption-desorption performance of a reaction intermediate product on the surface of the catalyst is improved, so that the diffusion of the reaction system is promoted, the reaction rate is accelerated, the carbon deposition and the blockage of pore channel are reduced, and the service life of the catalyst is effectively prolonged.

In the third type of embodiments of the present application, there is also provided a process for producing organic amines, comprising: contacting an amination raw material and an amination reagent with the catalyst as described above in the presence of hydrogen for amination reaction.

According to the third type of embodiments of the present application, the amination raw material or amination reagent can be selected as described above, of which the detailed description is omitted herein for brevity.

According to the third type of embodiments of the present application, the amination conditions may include: a mole ratio of hydrogen to the amination reagent and to the amination raw material of 1-5 : 2-30 : 1, a temperature of 110-220°C, a pressure of 0.8-25 MPa, and a liquid phase volume space velocity of the amination raw material of 0.06-1

m$^3$/(m$^3$·h).

Preferably, the amination raw material is a monohydric alcohol, and the amination conditions include: a mole ratio of hydrogen to the amination reagent and to the amination raw material of 1-4 : 2-9 : 1, a temperature of 130-200°C, a pressure of 1-2.5 MPa, and a liquid phase volume space velocity of the amination raw material of 0.1-0.8 m$^3$/(m$^3$ ·h).

Preferably, the amination raw material is a ketone or an aldehyde, and the amination conditions include: a mole ratio of hydrogen to the amination reagent and to the amination raw material of 1-4 : 2-6 : 1, a temperature of 110-180°C, a pressure of 0.8-2.5 MPa, and a liquid phase volume space velocity of the amination raw material of 0.1-0.8 m$^3$/(m$^3$·h).

Preferably, the amination raw material is an alcohol amine, and the amination conditions include: a mole ratio of hydrogen to the amination reagent and to the amination raw material of 1-4 : 3-20 : 1, a temperature of 130-200°C, a pressure of 1-15 MPa, and a liquid phase volume space velocity of the amination raw material of 0.06-0.8 m$^3$/(m$^3$·h).

Preferably, the amination raw material is a mixture of 1,6-hexanediol, cyclohexide and 6-amino-1-hexanol or a dihydric alcohol, and the amination conditions include: a mole ratio of hydrogen to the amination reagent and to the amination raw material of 1-4 : 3-30 : 1, a temperature of 130-220°C, a pressure of 1-25 MPa, and a liquid phase volume space velocity of the amination raw material of 0.1-0.8 m$^3$/(m$^3$·h).

Fourth type of embodiments

In the fourth type of embodiments of the present application, there is provided a catalyst having a function of catalyzing the amination of alcohols, comprising a carrier, and an active metal component and a metal promoter supported on the carrier, wherein the active metal component is cobalt and/or nickel, the metal promoter is a combination of at least one Group VIB metal, at least one Group IB metal and at least one Group IIB metal, and the carrier has an ammonia adsorption capacity of 0.25-0.6 mmol/g.

The catalyst of the fourth type of embodiments of the present application comprises a specific metal promoter, has a high catalytic activity, and at the same time, has a high selectivity and produces few by-products.

Preferably, the carrier has an ammonia adsorption capacity of 0.3 to 0.6 mmol/g.

Preferably, the carrier comprises a matrix and a doping element, wherein the matrix comprises an alumina carrier and an additional carrier, and said additional carrier is selected from silica and/or molecular sieves.

Preferably, a silica precursor and/or a molecular sieve precursor and the like are/is added during the preparation of the alumina precursor used in the carrier, and the diffusivity and the pore structure stability of the catalyst can be further greatly improved after the carrier is prepared. Preferably, the alumina carrier content in the carrier is 70 wt% or more, preferably 80-97 wt%, based on the total amount of the matrix.

Preferably, the doping element is present in the carrier in an amount of 0.05 to 5 wt%, preferably 0.08 to 3 wt%, relative to the total weight of the matrix.

Preferably, the doping element is incorporated in the form of at least one selected from the group consisting of borate ion, fluoride ion, phosphate ion, sulfate ion, and selenate ion. The doping element is preferably at least one selected from the group consisting of boron, fluorine, phosphorus, sulfur and selenium. According to the fourth type of embodiments of the present application, the non-metallic element is incorporated during the process for producing the precursor of the carrier, so that the doping element is mainly present in the bulk phase of the carrier, rather than attached to its surface.

Preferably, the proportion of the pore volume of pores having a pore diameter in a range of 7-27 nm to the pore volume of the carrier is greater than 65%, preferably 70-90%. More preferably, the proportion of the pore volume of pores having a pore diameter of less than 7 nm to the pore volume of the carrier is 0-8%, preferably 0-5%.

Preferably, the carrier has a specific surface area of 120-210 m$^2$/g.

Preferably, the carrier has a pore volume of 0.45 to 1.1 ml/g.

Preferably, the active metal component may be present in an amount of 10 to 46g, preferably 18 to 38g, per 100g of the matrix.

Preferably, the metal promoter may be present in an amount of 0.1 to 10g, preferably 0.5 to 6g, per 100 g of the matrix.

According to the fourth type of embodiments of the present application, the catalyst further comprises a metal promoter as described above to better exert the performance of the catalyst, to optimize the proportion of reaction products, and to reduce unwanted side reactions. The weight ratio of Group VIB metal, Group IB metal and Group IIB metal in the metal promoter is preferably 0.1-10 : 0.1-10 : 1, more preferably 0.2-8 : 0.2-8 : 1, more preferably 0.5-4 : 0.5-6 : 1. Preferably, the Group VIB metal is selected from molybdenum and/or tungsten. Preferably, the Group IB metal is at least one selected from the group consisting of copper, silver and gold. Preferably, the Group IIB metal is selected from zinc.

According to the fourth type of embodiments of the present application, the use of a carrier having a specific pore structure and ammonia adsorption capacity may allow the catalyst to have a high catalytic activity, and at the same time, have a high selectivity, when used for the hydroamination of alcohols; and when the catalyst is used for the hydroamination of n-propanol, the production of other impurities is little. When the catalyst is used for the hydroamination of 1,6-hexanediol, the production of heavy components and other impurities is less. After a long-period of life evaluation, it is found that the catalyst has a stable catalytic performance, and through the modification of the carrier, the adsorption-desorption performance of the catalyst is improved, the diffusion of the reaction system is promoted, the reaction rate is accelerated, the carbon deposition is reduced, and the blockage of pore channel is retarded.

In the fourth type of embodiments of the present application, there is also provided a process for producing organic amines, comprising: contacting an amination raw material and an amination reagent with the catalyst as described above in the presence of hydrogen for amination reaction.

According to the fourth type of embodiments of the present application, the amination raw material or amination reagent can be selected as described above, of which the detailed description is omitted herein for brevity.

According to the fourth type of embodiments of the present application, the amination conditions may include: a mole ratio of hydrogen to the amination reagent and to the amination raw material of 1-5 : 2-35 : 1, a temperature of 105-220 °C, a pressure of 0.7-25 MPa, and a liquid phase volume space velocity of the amination raw material of 0.06-1 $m^3/(m^3 \cdot h)$.

Preferably, the amination raw material is a monohydric alcohol, and the amination conditions include: a mole ratio of hydrogen to the amination reagent and to the amination raw material of 1-4 : 2-8 : 1, a temperature of 130-200 °C, a pressure of 1-2.5 MPa, and a liquid phase volume space velocity of the amination raw material of 0.1-0.8 $m^3/(m^3 \cdot h)$.

Preferably, the amination raw material is a ketone or an aldehyde, and the amination conditions include: a mole ratio of hydrogen to the amination reagent and to the amination raw material of 1-4 : 2-5 : 1, a temperature of 105-180 °C, a pressure of 0.7-2.5 MPa, and a liquid phase volume space velocity of the amination raw material of 0.1-0.8 $m^3/(m^3 \cdot h)$.

Preferably, the amination raw material is an alcohol amine, and the amination conditions include: a mole ratio of hydrogen to the amination reagent and to the amination raw material of 1-4 : 3-25 : 1, a temperature of 130-200 °C, a pressure of 5-18 MPa, and a liquid phase volume space velocity of the amination raw material of 0.06-0.8 $m^3/(m^3 \cdot h)$.

Preferably, the amination raw material is a mixture of 1,6-hexanediol, cycloheximide and 6-amino-1-hexanol or a dihydric alcohol, and the amination conditions include: a molar ratio of hydrogen to the amination reagent and to the amination raw material of 0.3-4 : 3-45 : 1, preferably 1-4 : 3-35 : 1, a temperature of 130-220 °C, a pressure of 2-25 MPa, and a liquid phase volume space velocity of the amination raw material of 0.1-0.8 $m^3/(m^3 \cdot h)$.

Fifth type of embodiments

In the fifth type of embodiments of the present application, there is provided a titanium-containing catalyst having an amination function, comprising a carrier and an active metal component and optionally a metal promoter supported on the carrier, wherein the carrier comprises a matrix and optionally a doping element, wherein the matrix comprises alumina and titania at a weight ratio of 1.5-5 : 1, the active metal component is cobalt and/or nickel, and the catalyst has an ammonia adsorption capacity of 0.2-0.7 mmol/g, preferably 0.3-0.4 mmol/g.

The catalyst of the fifth type of embodiments of the present application has a high catalytic activity, and at the same time, has a high selectivity, when used for the hydroamination of alcohols. Particularly, when the catalyst is used for the hydroamination of 1,6-hexanediol, the production of heavy components and other impurities is less, and the selectivity to hexanediamine is high. After a long-period of life evaluation, it is found that the catalyst has a stable catalytic performance, the reaction rate is accelerated, the carbon deposition is reduced and the blockage of pore channel is retarded.

Titania has strong acidity, comprises both B acid and L acid, and has a strong interaction with components dispersed on it, which is different from alumina. The composite carrier compounded by alumina and titania has different catalytic effects on different reactions. The inventors of the present application, through careful research and fine differentiation, have found that the incorporation of an appropriate amount of titania into pseudo-boehmite during the forming of the carrier can particularly effectively improve the catalytic performance of the catalyst obtained. Preferably, the weight ratio of the alumina to the titania is 2-4.5 : 1. By using such a specific ratio, the catalytic performance of the catalyst obtained can be further improved.

Preferably, the active metal component may be present in an amount of 13 to 40 g, preferably 20 to 36g, per 100 g of the matrix.

Preferably, the metal promoter may be present in an amount of 0 to 10 g, preferably 2.5 to 8g, per 100 g of the matrix.

According to the fifth type of embodiments of the present application, the titanium-containing catalyst may further comprise a metal promoter to better exert the performance of the catalyst, to optimize the proportion of reaction products, and to reduce unwanted side reactions. The metal promoter can be at least one selected from the group consisting of Group VIB, Group VIIB, Group IB, Group IIB and lanthanide series elements, preferably at least one of Cr, Mo, W, Mn, Re, Cu, Ag, Au, Zn, La and Ce, and more preferably at least one of Mo, Mn, Re, Cu, Ag, Zn and La.

Preferably, the titanium-containing catalyst has a carbon dioxide adsorption capacity of 0.07-0.2 mmol/g.

Preferably, the titanium-containing catalyst has a specific surface area of 130-180 m2/g.

Preferably, the titanium-containing catalyst has a pore volume of 0.55 to 0.75 ml/g.

Preferably, in the titanium-containing catalyst, the proportion of the pore volume of pores having a pore radius of less than 4 nm to the total pore volume is preferably less than 20% (e.g., 3%, 5%, 7%, 8.5%, 10.5%, 11.5%, 12.5%, 15.5%, 18.5%, 19%, 19.5%, or a value between any two of them), the proportion of the pore volume of pores having a pore radius of greater than 10nm to the total pore volume is preferably less than 15% (e.g., 3%, 5%, 7%, 8.5%, 9%, 10.5%, 11.5%, 12.5%, 13.5%, 14%, 14.5%, or a value between any two of them), and the proportion of the pore volume of pores having a pore radius of 4 to 10nm to the total pore volume is greater than or equal to 65% (e.g., 66%, 70% 72%, 73%, 75%, 76.5%, 77%, 77.5%, 78.5%, 79%, 80%, or a value between any two of them).

Preferably, the carrier of the titanium-containing catalyst is doped with sulfur in an amount of 0.1 to 0.5 g, more preferably 0.15 to 0.4g, per 100g of the matrix.

In the fifth type of embodiments of the present application, the loading of the active metal component and the metal promoter has no substantial compact on the ammonia adsorption capacity, the carbon dioxide adsorption capacity, the specific surface area, the pore structure characteristics, and the like of the catalyst, and thus the relevant properties of the carrier of the catalyst are similar to those described above (e.g., the difference is within $\pm$ 5%), of which the detailed description is omitted herein for brevity.

Preferably, the titanium-containing catalyst may be in the form of a strip, a sheet, a clover or a dentate sphere.

Catalysts according to the fifth type of embodiments of the present application may be prepared by way of loading, by first providing a carrier comprising a matrix and optionally a doping element, the matrix comprising alumina and titania at a weight ratio of 2-5 : 1, and then loading an active metal component and optionally a metal promoter on the carrier.

In the fifth type of embodiments of the present application, there is provided a method for producing a titanium-containing catalyst having amination function, comprising:

1) subjecting pseudo-boehmite and titania to kneading, shaping and calcining to obtain a carrier, wherein the pseudo-boehmite and titania are used in such an amount that the weight ratio of alumina to titania in the carrier obtained is 1.5-5 : 1;

2) loading an active metal component and optionally a metal promoter on the carrier obtained, wherein the active metal component comprises cobalt and/or nickel.

Preferably, the pseudo-boehmite and titania are used in such an amount that the weight ratio of the alumina to the titania in the carrier obtained is 2-4.5 : 1.

Preferably, the pseudo-boehmite is prepared by at least one of carbonization method, organoaluminum hydrolysis method, aluminum sulfate method, and nitric acid method. The organic aluminum is preferably C1-C10 organic aluminum, preferably aluminum isopropoxide.

Preferably, the titania is provided by titanium white powder, which is prepared by a precipitation method and/or a sol-gel method, and the raw material capable of providing titanium used in the method can be an organic titanium source (such as organic titanate) and/or an inorganic titanium source (such as inorganic titanium salt). The inorganic titanium source can be one or more of $TiCl_4$, $Ti(SO_4)_2$, $TiOSO_4$, $TiOCl_2$, titanium hydroxide, titanium nitrate salt, titanium phosphate salt and the like, and the organic titanium source can be one or more of titanium alkoxides of fatty alcohols and organotitanates. The organotitanate is preferably an organotitanate having a formula of $M_4TiO_4$, wherein M is preferably an alkyl group having 1 to 4 carbon atoms and the four M may be the same or different, preferably the organotitanate is one or more selected from isopropyl titanate, n-propyl titanate, tetrabutyl titanate and tetraethyl titanate. Specific examples of the raw material capable of providing titanium may be, but are not limited to: $TiOCl_2$, titanium tetrachloride, titanium sulfate, titanyl sulfate, tetrapropyl titanate (including various isomers of tetrapropyl titanate, such as tetraisopropyl titanate and tetra-n-propyl titanate), tetrabutyl titanate (including various isomers of tetrabutyl titanate, such as tetra-n-butyl titanate), and tetraethyl titanate.

Preferably, the content of sulfate radical in the titanium white powder is 0.2-3 wt%. The titanium white powder is more preferably titanium white powder prepared by titanium sulfate- or titanium oxysulfate-ammonia hydrolysis method, and by using such titanium white powder, a catalyst comprising a carrier with more desirable pore channel structure and having a better catalytic performance can be obtained.

Preferably, the active metal component is used in such an amount that the active metal component is present in an amount of 13-40g (e.g., 13g, 15g, 18g, 20g, 24g, 25g, 26g, 27g, 28g, 30g, 33g, 35g, 36g, 37g, 38g, 40g, or a value between any two of them), per 100 g of the carrier.

Preferably, to better exert the performance of the catalyst, optimize the proportion of reaction products and reduce unwanted side reactions, a metal promoter can also be loaded on the carrier. The metal promoter is used in such an amount that the metal promoter is present in an amount of 0-10g (e.g., 1g, 2g, 2.5g, 3g, 4g, 5g, 6g, 7g, 8g, 8.5g, 9g, 10g, or a value between any two of them), per 100 g of the carrier.

Preferably, the metal promoter may be at least one selected from the group consisting of Group VIB, Group VIIB, Group IB, Group IIB and lanthanide series metals, preferably at least one of Cr, Mo, W, Mn, Re, Cu, Ag, Au, Zn, La and Ce, more preferably at least one of Mo, Mn, Re, Cu, Ag, Zn and La.

Preferably, in step 1), there is no particular requirement on the conditions of the kneading, shaping and calcining. To obtain a carrier having an appropriate strength, the kneading is carried out in the presence of an acid (particularly at least one of an inorganic acid such as nitric acid, sulfuric acid and hydrofluoric acid and an organic acid such as formic acid, acetic acid, citric acid) in an amount of 2 to 10 g, per 100 g of the powder. The temperature of the calcining in step 1) may be 550-1100 °C, preferably 800-1050 °C. The calcining time in step 1) can be 2-6 h.

Preferably, in step 2), the loading may be carried out by impregnation, that is, the carrier may be impregnated with a solution containing an active metal component precursor and optionally a metal promoter precursor, and then dried and calcined.

Preferably, the method may further comprise a step of reducing the product obtained in step 2) to obtain a catalyst with the active metal component and a reducible metal promoter present in a reduced state. The reduction can be carried out with a hydrogen-containing gas at 350-500 °C, preferably at 350-450 °C. The hydrogen-containing gas may be pure hydrogen gas or hydrogen gas diluted with an inert gas, such as a mixture of nitrogen and hydrogen. The reduction temperature is gradually increased during the reduction, and the temperature rise is preferably not too fast, for example, not more than 20 °C per hour. The reduction time can be determined by monitoring the generation of $H_2O$ in the reduction system, that is when no new $H_2O$ is generated in the reduction system, the reduction is terminated, and one skilled in the art can select the reduction time accordingly, of which the detailed description is omitted herein for brevity, for example, the reduction time can be 2-5h at the highest temperature.

The reduction may be carried out directly in the reactor, followed by the catalytic reaction. It is also possible to carry out the reduction in a separate reactor, also referred to as out-of-reactor reduction, and a passivation may be carried out after the reduction with a gas mixture comprising oxygen before the catalyst being discharged from the reactor, the passivation temperature being, for example, from 10 to 60 °C and particularly from 20 to 40 °C. The catalyst undergone the out-of-reactor reduction and passivation can be activated before use using hydrogen or a mixture of hydrogen and nitrogen at, for example, 150 °C to 250 °C, preferably 170 °C to 200 °C. The activation time can be determined by monitoring the generation of $H_2O$ in the activation system, that is when no new $H_2O$ is generated in the activation system, the activation is terminated and one skilled in the art can select the activation time accordingly, of which the detailed description is omitted herein for brevity. For example, the activation time at the highest temperature may be, for example, 1 to 5 hours, preferably 2 to 3 hours, or the catalyst can be used without activation, depending on the extent to which the active metal component and metal promoter of the catalyst have been oxidized.

In the fifth type of embodiments of the present application, there is also provided a process for producing organic amines, comprising: contacting ammonia and/or an organic amine with an alcohol in the presence of the titanium-containing catalyst as described above for amination reaction.

Preferably, the alcohol may be at least one selected from the group consisting of substituted or unsubstituted monohydric alcohols having 2 to 20 carbon atoms and substituted or unsubstituted dihydric alcohols having 2 to 20 carbon atoms (the substituent group may be an amino group), for example, at least one of ethanol, n-propanol, isopropanol, n-butanol, isobutanol, 2-ethylhexanol, octanol, dodecanol, hexadecanol, cyclopentanol, cyclohexanol, benzyl alcohol, phenethyl alcohol, ethylene glycol, 1,3-propanediol, 1,4-butanediol, 1,4-butanedial, 1,5-pentanediol, 1,6-hexanediol, 1,8-octanediol, 1,8-octanedial, 1,12-dodecanediol,ethanolamine (2-aminoethanol), 6-amino-1-hexanol (referred to as aminohexanol for short), and the like.

Preferably, the organic amine may be a primary amine and/or a secondary amine. For example, an alkylamine having 1 to 4 carbon atoms, such as at least one of monomethylamine, dimethylamine, methylethylamine, monoethylamine, diethylamine, and the like, can be used. The amination reaction is preferably carried out using an excess of ammonia or amine, for example the ammonia or organic amine may be used in an amount of 1 to 50 moles, preferably 3 to 20 moles, more preferably 3 to 15 moles, per mole of hydroxyl groups in the alcohol. The amination reaction of the alcohol is carried out in the presence of hydrogen, i.e. under hydro conditions. The hydrogen may be used in an amount of 1 to 10 mol, per mol of hydroxyl groups in the alcohol. The hydrogen partial pressure is, for example, from 0.05 to 2 MPa. The temperature of the amination reaction may be 100-300 °C. The pressure of the amination reaction may be from 0.1 to 25 MPa, preferably from 1 to 15 MPa. The liquid hourly volume space velocity of the alcohol may be from 0.1-0.8 $m^3/(m^3\ h)$.

For example, the amination reaction is, for example, the hydroamination of ethanol for producing ethylamine, the hydroamination of n-propanol for producing n-propylamine, the hydroamination of isopropanol for producing isopropylamine, the hydroamination of n-butanol for producing n-butylamine, the hydroamination of ethylene glycol for producing ethylene diamine, the hydroamination of ethanolamine for producing ethylene glycol, the hydroamination of 1,3-propanediol for producing 1,3-propanediamine, the hydroamination of 1,4-butanediol for producing 1,4-butanediamine, the hydroamination of 1,6-hexanediol for producing 1,6-hexanediamine, or the hydroamination of 1,12-dodecanediol for producing 1,12-dodecanediamine.

According to the fifth type of embodiments of the present application, the amination reaction can be carried out batchwise or continuously. The amination reaction can be a gas phase reaction or a liquid phase trickle bed reaction.

There is also provided herein some specific embodiments for carrying out amination reactions using the catalyst according to the fifth type of embodiments of the present application.

For example, the present application provides a process for producing 1,6-hexanediamine from 1,6-hexanediol using the catalyst according to the fifth type of embodiments of the present application, comprising conducting a hydroamination reaction with 1,6-hexanediol, ammonia and hydrogen at a temperature of 130-200 °C and a reaction pressure of 1-11 MPa, a liquid hourly volume space velocity of 1,6-hexanediol feeding of 0.1-0.8 $m^3/(m^3 \cdot h)$, and a molar ratio of hydrogen to ammonia and to hexanediol of 1-4 : 3-20 : 1, to produce hexanediamine, 6-amino-1-hexanol and hexamethyleneimine.

The present application provides a process for producing 1,6-hexanediamine from 1,6-hexanediol, hexamethyleneimine and 6-amino-1-hexanol using the catalyst according to the fifth type of embodiments, comprising conducting a hydroamination reaction with a mixture of 1,6-hexanediol, hexamethyleneimine and 6-amino-1-hexanol, ammonia and hydrogen at a reaction temperature of 130-200 °C, a reaction pressure of 1-11 MPa, a liquid hourly volume space velocity of the mixture of 0.1-0.8 $m^3/(m^3 \cdot h)$, and a molar ratio of hydrogen to ammonia and to the mixture of 1-4 : 3-20 : 1, to produce a reaction solution containing 1,6-hexanediamine, 6-amino-1-hexanol and cyclohexamide.

In particularly preferred embodiments, the present application discloses the following technical solutions:

A1, a catalyst having a function of catalyzing alcohol hydroamination to produce organic amine, which comprises a carrier, an active metal component and an optional metal promoter, wherein the active metal component and the optional metal promoter are supported on the carrier, and the carrier is at least one selected from the group consisting of doped alumina, doped silica, doped molecular sieves and doped aluminosilicates; the carrier has an ammonia adsorption capacity of 0.25-0.6 mmol/g, and a carbon dioxide adsorption capacity of 0.05-0.3 mmol/g; and the active metal component is cobalt and/or nickel.

A2, the catalyst according to Item A1, wherein the carrier has an ammonia adsorption capacity of 0.3 to 0.5 mmol/g;

and/or the carrier has a carbon dioxide adsorption capacity of 0.06-0.2 mmol/g;
and/or, the content of the doped element in the carrier accounts for 0.03 to 2 wt%, preferably 0.08 to 1 wt%, of the total weight of the elements other than the doping element in the carrier;
and/or the doping element in the carrier comprises a metallic element and a non-metallic element, wherein the metallic element is at least one selected from the group consisting of Group IA metal elements, Group IIA metal elements, Group VA metal elements and lanthanide series metal elements, preferably at least one selected from the group consisting of calcium, magnesium, potassium, bismuth, strontium, barium and lanthanum; the non-metallic element is at least one selected from the group consisting of Group IIIA non-metallic elements, Group VA non-metallic elements, Group VIA non-metallic elements and Group VIIA non-metallic elements, preferably at least one selected from the group consisting of boron, fluorine, phosphorus, sulfur and selenium; preferably, the doping element in the carrier is derived from metal cations and acid radical ions, but does not include sodium ion or chloride ion; the metal cation is at least one selected from the group consisting of Group IA metal cations, Group IIA metal ions, Group VA metal ions and lanthanide series metal ions, preferably at least one selected from the group consisting of calcium ion, magnesium ion, potassium ion, bismuth ion, strontium ion, barium ion and lanthanum ion; the acid radical ion is at least one selected from the group consisting of non-metallic acid radical ions, preferably at least one selected from the group consisting of borate ion, fluoride ion, phosphate ion, sulfate ion and selenate ion;
and/or the carrier has a specific surface area of 120-240 $m^2/g$;
and/or the carrier has a pore volume of 0.5-1 ml/g;
and/or the content of the active metal component is 5 to 42g, preferably 10 to 35 g, per 100g of the matrix.

A3, the catalyst according to Item A1 or A2, wherein the carrier is prepared by a method comprising the steps of: sequentially shaping, drying and calcining a mixture comprising the doping element and a carrier source, wherein the carrier source is at least one selected from the group consisting of pseudo-boehmite, silica, molecular sieves and aluminosilicates.

A4. the catalyst according to Item A3, wherein the doping element is provided by a carrier modifier, preferably the carrier modifier is at least one of compounds capable of providing a cation and an anion, wherein the cation is at least one selected from the group consisting of Group IA cations, Group IIA metal ions, Group VA metal ions, and lanthanide metal ions, preferably at least one selected from the group consisting of calcium ion, magnesium ion, potassium ion, bismuth ion, strontium ion, barium ion, and lanthanum ion;
and/or the anion is at least one selected from the group consisting of non-metallic acid radical ions, preferably at least one selected from the group consisting of borate ion, fluoride ion, phosphate ion, sulfate ion and selenate ion.

A5, the catalyst according to Item A4, wherein the carrier modifier is at least one selected from the group consisting of boric acid, nickel borate, cobalt borate, potassium borate, hydrofluoric acid, potassium fluoride, cobalt fluoride, nickel fluoride, phosphoric acid, aluminum phosphate, tripotassium phosphate, potassium dihydrogen phosphate, potassium hydrogen phosphate, magnesium phosphate, calcium phosphate, sulfuric acid, cobalt sulfate, nickel sulfate, aluminum sulfate, calcium sulfate, bismuth nitrate, potassium nitrate, potassium sulfate, potassium carbonate, magnesium nitrate, magnesium sulfate, basic magnesium carbonate, calcium nitrate, basic calcium carbonate, strontium nitrate, strontium phosphate, strontium sulfate, barium nitrate, lanthanum nitrate, and selenic acid;

preferably, the pseudo-boehmite has a specific surface area of 250-330 $m^2$/g, and a pore volume of 0.8-1.3 ml/g.

A6, the catalyst according to any one of Items A3 to A5, wherein the drying conditions include: a temperature of 80-150 °C, and a time of 6-20 h;

and/or the calcining conditions include: a temperature of 600-1100 °C, and a time of 2-20 h.

A7, a method for producing the catalyst according to any one of Items A1 to A6, comprising: loading the active metal component and optionally the metal promoter on the carrier.

A8, a carrier as defined in any one of Items A1 to A6.

A9, Use of the catalyst according to any one of Items A1 to A6, or the method according to Item A7, or the carrier according to Item A8, in the amination for producing organic amines.

A10, a process for producing an organic amine, comprising: contacting an amination raw material and an amination reagent with the catalyst according to any one of Items A1 to A6 in the presence of hydrogen for amination reaction; or alternatively, the method comprises: screening a catalyst comprising the carrier as defined in any one of Items A1 to A6, and contacting an amination raw material and an amination reagent with the screened catalyst in the presence of hydrogen for amination reaction.

A11, the process according to Item A10, wherein the amination conditions include: a mole ratio of hydrogen to the amination reagent and to the amination raw material of 1-5 : 2-35 : 1, a temperature of 130-200 °C, a pressure of 1-15 MPa, and a liquid phase volume space velocity of the amination raw material of 0.06-1 $m^3$/($m^3$ h);

and/or the amination raw material is at least one selected from the group consisting of C2-20 alcohols, C3-20 ketones, C2-20 alcohol amines and C2-20 aldehydes, preferably at least one of ethanol, acetaldehyde, n-propanol, propionaldehyde, isopropanol, n-butanol, butyraldehyde, isobutanol, isobutyraldehyde, 2-ethylhexanol, 2-ethylhexaldehyde, octanol, octanal, dodecanol, dodecanal, hexadecanol, hexadecanal, cyclopentanol, cyclohexanol, cyclooctanol, cyclododecanol, benzyl alcohol, benzaldehyde, phenethyl alcohol, phenylacetaldehyde, 1,4-butanediol, 1,4-butanedial, 1,5-pentanediol, 1,5-glutaraldehyde, 1,6-hexanediol, 1,6-hexanedial, 1,8-octanediol, 1,8-octanedial, 1,12-dodecanediol, 1,12-dodecanedialdehyde, ethanolamine, propanolamine, isopropanolamine, 6-aminohexanol, diethanolamine, acetone, ethylene glycol, and 1,3-propanediol;

and/or the amination reagent is at least one selected from the group consisting of ammonia, C1-12 primary amine and C2-12 secondary amine, preferably at least one of ammonia, monomethylamine, dimethylamine, methyl ethyl amine, monoethylamine and diethylamine.

A12, the process according to Item A11, wherein:

where the amination raw material is a monohydric alcohol, the amination conditions include: a mole ratio of hydrogen to the amination reagent and to the amination raw material of 1-4 : 2-10 : 1, a temperature of 130-200 °C, a pressure of 1-4 MPa, and a liquid phase volume space velocity of the amination raw material of 0.1-0.8 $m^3$/($m^3$·h);

or, where the amination raw material is a ketone or an aldehyde, the amination conditions include: a mole ratio of hydrogen to the amination reagent and to the amination raw material of 1-4 : 2-6 : 1, a temperature of 110-180 °C, a pressure of 1-3.5 MPa, and a liquid phase volume space velocity of the amination raw material of 0.1-1 $m^3$/($m^3$·h);

or, where the amination raw material is an alcohol amine, the amination conditions include: a mole ratio of hydrogen to the amination reagent and to the amination raw material of 1-4 : 3-20 : 1, a temperature of 135-200 °C, a pressure of 1-11 MPa, and a liquid phase volume space velocity of the amination raw material of 0.06-0.8 $m^3$/($m^3$·h);

or, where the amination raw material is a dihydric alcohol, the amination conditions include: a mole ratio of hydrogen to the amination reagent and to the amination raw material of 1-4 : 3-35 : 1, a temperature of 130-210 °C, a pressure of 1-15 MPa, and a liquid phase volume space velocity of the amination raw material of 0.1-0.8 $m^3$/($m^3$·h);

or, where the amination raw material is a mixture of 1,6-hexanediol, hexamethyleneimine and 6-amino-1-hexanol, the amination conditions include: a mole ratio of hydrogen to the amination reagent and to the amination raw material of 1-4 : 3-35 : 1, a temperature of 130-210 °C, a pressure of 1-15 MPa, and a liquid phase volume space velocity of the amination raw material of 0.1-0.8 $m^3/(m^3 \cdot h)$.

B1, a catalyst having a function of catalyzing amination to produce amine, comprising a carrier, and an active metal component and a metal promoter supported on the carrier, wherein the active metal component is cobalt and/or nickel; and the metal promoter is a combination of at least one Group VIIB metal and at least one Group IB metal.

B2, the catalyst according to Item B1, wherein the carrier comprises an alumina carrier, a doping element and optionally an additional carrier selected from silica and/or molecular sieves; the carrier has an ammonia adsorption capacity of 0.3-0.6 mmol/g; the proportion of the pore volume of pores having a pore diameter in a range of 7-27 nm to the pore volume of the carrier is more than or equal to 70%.

B3, the catalyst according to Item B1 or B2, wherein the content of the alumina carrier in the carrier is 75 wt% or more, preferably 80-100 wt%, based on the total amount of the alumina carrier and said additional carrier;

and/or the content of the doping element is 0.05-6 wt%, preferably 0.08-4 wt%, relative to the weight of the matrix;

and/or the doping element in the carrier is a non-metallic element, and is preferably incorporated during the preparation of the carrier in the form of at least one selected from the group consisting of borate ion, fluoride ion, phosphate ion, sulfate ion and selenate ion;

and/or the carrier has an ammonia adsorption capacity of 0.3-0.56 mmol/g;

and/or the proportion of the pore volume of pores having a pore diameter in a range of 7-27 nm to the pore volume of the carrier is 70-90%, and the proportion of the pore volume of pores having a pore diameter of less than 7 nm to the pore volume of the carrier is 0-8%;

and/or the carrier has a specific surface area of 125-200 $m^2/g$;

and/or the carrier has a pore volume of 0.45-1.1 ml/g;

and/or the active metal component is present in an amount of 8 to 45g, per 100g of the matrix;

and/or the content of the metal promoter is 0.1-10 g, per 100 g of the matrix;

and/or the weight ratio of the Group VIIB metal to the Group IB metal in the metal promoter is 0.05-15 : 1, preferably 0.1-12 : 1;

and/or, the Group VIIB metal is selected from manganese and/or rhenium;

and/or, the Group IB metal is at least one selected from the group consisting of copper, silver and gold.

B4 the catalyst according to any one of Items B1 to B3, wherein the carrier is prepared by a method comprising: sequentially shaping, drying and calcining a mixture comprising an alumina precursor, a doping element and optionally an additional carrier precursor, wherein said additional carrier precursor is selected from a silica precursor and/or a molecular sieve precursor, the doping element is provided by a carrier modifier, and the carrier modifier is at least one inorganic acid.

B5, The catalyst according to Item B4, wherein the inorganic acid is at least one selected from inorganic acids containing non-metallic acid radical ions, preferably at least one selected from boric acid, hydrofluoric acid, phosphoric acid, sulfuric acid, and selenic acid.

B6, the catalyst according to Item B4 or B5, wherein the alumina precursor is pseudo-boehmite, the pseudo-boehmite has a specific surface area of 260-380 $m^2/g$, and a pore volume of 0.78-1.2 ml/g.

B7, the catalyst according to any one of Items B4 to B6, wherein the drying conditions include: a temperature of 80-150 °C, and a time of 6-20 h;

and/or the calcining conditions include: a temperature of 550-1050 °C, and a time of 2-20 h.

B8, a method for producing the catalyst according to any one of Items B1 to B7, comprising: loading the active metal component and the metal promoter on the carrier.

B9, a carrier as defined in any one of Items B2 to B7.

B10, Use of the catalyst according to any one of Items B1 to B7, or the method according to Item B8, or the carrier according to Item B9, in the amination for producing organic amines.

B11, a process for producing organic amine, comprising the following steps: contacting an amination raw material and an amination reagent with the catalyst according to any one of Items B1-B7 in the presence of hydrogen for amination reaction;

or alternatively, the method comprises: screening a catalyst comprising the carrier as defined in any one of Items B1 to B7, and contacting an amination raw material and an amination reagent with the screened catalyst in the presence of hydrogen for amination reaction.

B12, the method according to Item B11, wherein the amination conditions include: a mole ratio of hydrogen to the amination reagent and to the amination raw material of 1-5 : 2-33 : 1, a temperature of 110-220 °C, a pressure of 0.8-25 MPa, and a liquid phase volume space velocity of the amination raw material of 0.06-1 $m^3/(m^3 \cdot h)$;

and/or the amination raw material is at least one selected from the group consisting of C2-20 alcohols, C3-20 ketones, C2-20 alcohol amines and C2-20 aldehydes, preferably at least one of ethanol, acetaldehyde, n-propanol, propionaldehyde, isopropanol, n-butanol, butyraldehyde, isobutanol, isobutyraldehyde, 2-ethylhexanol, 2-ethylhexaldehyde, octanol, octanal, dodecanol, dodecanal, hexadecanol, hexadecanal, cyclopentanol, cyclohexanol, cyclooctanol, cyclododecanol, benzyl alcohol, benzaldehyde, phenethyl alcohol, phenylacetaldehyde, 1,4-butanediol, 1,4-butanedial, 1,5-pentanediol, 1,5-glutaraldehyde, 1,6-hexanediol, 1,6-hexanedial, 1,8-octanediol, 1,8-octanedial, ethanolamine, propanolamine, isopropanolamine, 6-aminohexanol, diethanolamine, diisopropanolamine, dimethylethanolamine, acetone, ethylene glycol, 1,3-propanediol, and 1,12-dodecanediol;

and/or the amination reagent is at least one selected from the group consisting of ammonia, C1-12 primary amines and C1-12 secondary amines, preferably at least one of ammonia, monomethylamine, dimethylamine, methyl ethyl amine, monoethylamine and diethylamine.

B13, the method according to Item B12, wherein:

where the amination raw material is a monohydric alcohol, the amination conditions include: a mole ratio of hydrogen to the amination reagent and to the amination raw material of 1-4 : 2-10 : 1, a temperature of 130-210 °C, a pressure of 1-3.5 MPa, and a liquid phase volume space velocity of the amination raw material of 0.1-0.8 $m^3/(m^3 \cdot h)$;

or, where the amination raw material is a ketone or an aldehyde, the amination conditions include: a mole ratio of hydrogen to the amination reagent and to the amination raw material of 1-4 : 2-5 : 1, a temperature of 110-170 °C, a pressure of 0.8-2.5 MPa, and a liquid phase volume space velocity of the amination raw material of 0.1-1 $m^3/(m^3 \cdot h)$;

or, where the amination raw material is an alcohol amine, the amination conditions include: a mole ratio of hydrogen to the amination reagent and to the amination raw material of 1-4 : 3-25 : 1, a temperature of 130-200 °C, a pressure of 1-18 MPa, and a liquid phase volume space velocity of the amination raw material of 0.06-0.8 $m^3/(m^3 \cdot h)$;

or, where the amination raw material is a mixture of 1,6-hexanediol, hexamethyleneimine and 6-amino-1-hexanol or a dihydric alcohol, the amination conditions include: a mole ratio of hydrogen to the amination reagent and to the amination raw material of 1-4 : 3-33 : 1, a temperature of 130-220 °C, a pressure of 4-25 MPa, and a liquid phase volume space velocity of the amination raw material of 0.06-0.8 $m^3/(m^3 \cdot h)$.

C1, a catalyst having a function of catalyzing alcohol hydroamination, comprising a carrier, and a metal active component and a metal promoter supported on the carrier, wherein the metal active component is cobalt and/or nickel; the metal promoter is a combination of at least one Group VIIB metal and at least one Group IIB metal.

C2, the catalyst according to Item C1, wherein the carrier comprises an alumina carrier, a doping element and optionally an additional carrier that is at least one selected from the group consisting of silica, molecular sieves and diatomite; the carrier has an ammonia adsorption capacity of 0.3-0.7 mmol/g and a carbon dioxide adsorption capacity of 0.05-0.4 mmol/g; a proportion of the pore volume of pores having a pore diameter in a range of 7-27 nm to the pore volume of the carrier of greater than 65%.

C3, the catalyst according to Item C1 or C2, wherein the content of the alumina carrier in the carrier is 70 wt% or more, preferably 80 to 100 wt%, based on the total amount of the alumina carrier and said additional carrier;

and/or the doping element is present in the carrier in an amount of 0.05-6 wt%, preferably 0.08-4 wt%, relative to the total weight of the matrix;

and/or the doping element in the carrier is selected from metallic elements and non-metallic elements and does not include sodium or chlorine; the metallic element is at least one selected from the group consisting of Group IA metal elements, Group IIA metal elements, Group VA metal elements and lanthanide series metal elements, preferably at least one of calcium, magnesium, potassium, bismuth, strontium, barium ions and lanthanum; the non-metallic elements are derived from at least one of non-metallic acid radical ions, preferably from at least one selected from the group consisting of borate ion, fluoride ion, phosphate ion, sulfate ion and selenate ion;

and/or the carrier has an ammonia adsorption capacity of 0.3-0.6 mmol/g, and a carbon dioxide adsorption capacity of 0.05-0.3 mmol/g;

and/or the proportion of the pore volume of pores having a pore diameter in a range of 7-27 nm to the pore volume of the carrier is 70-90%, and the proportion of the pore volume of pores having a pore diameter of less than 7 nm to the pore volume of the carrier is 0-10%;

and/or the carrier has a specific surface area of 120-205 $m^2/g$;

and/or the carrier has a pore volume of 0.45-1.2 ml/g;

and/or the content of the metal active component is 14-46g, per 100g of the matrix;

and/or the content of the metal promoter is 0.1-10 g, per 100 g of the matrix;
and/or the weight ratio of Group VIIB metal to Group IIB metal in the metal promoter is 0.2-20 : 1, preferably 0.3-6 : 1;
and/or, the Group VIIB metal is selected from manganese and/or rhenium;
and/or the Group IIB metal is selected from zinc.

C4, the catalyst according to any one of Items C1 to C3, wherein the carrier is prepared by a method comprising the steps of: sequentially shaping, drying and calcining a mixture comprising the doping element, an alumina precursor and optionally an additional carrier precursor, wherein said additional carrier precursor is at least one selected from the group consisting of silica precursor, molecular sieve precursor and diatomite precursor.

C5, The catalyst according to Item C4, wherein the doping element is provided by a carrier modifier that is at least one selected from the group consisting of boric acid, nickel borate, cobalt borate, potassium borate, hydrofluoric acid, potassium fluoride, cobalt fluoride, nickel fluoride, phosphoric acid, aluminum phosphate, tripotassium phosphate, potassium dihydrogen phosphate, potassium hydrogen phosphate, magnesium phosphate, calcium phosphate, sulfuric acid, cobalt sulfate, nickel sulfate, aluminum sulfate, calcium sulfate, bismuth nitrate, potassium nitrate, potassium sulfate, potassium carbonate, magnesium nitrate, magnesium sulfate, basic magnesium carbonate, calcium nitrate, basic calcium carbonate, strontium nitrate, strontium phosphate, strontium sulfate, barium nitrate, lanthanum nitrate, and selenic acid.

C6, the catalyst according to Item C4 or C5, wherein the alumina precursor is pseudo-boehmite, the pseudo-boehmite has a specific surface area of 265-410 $m^2$/g, and a pore volume of 0.7-1.2 ml/g.

C7, the catalyst according to any one of Items C4 to C6, wherein the drying conditions include: a temperature of 80-150 °C, and a time of 6-20 h;
and/or the calcining conditions include: a temperature of 500-1100 °C, and a time of 2-20 h.

C8, a method for producing the catalyst according to any one of Items C1 to C7, comprising: loading the metal active component and the metal promoter on the carrier.

C9, A carrier as defined in any one of Items C2 to C7.

C10, Use of the catalyst according to any one of Items C1 to C7, or the method according to Item C8, or the carrier according to Item C9, in the amination for producing organic amines.

C11, a method for producing organic amine, comprising the following steps: contacting an amination raw material and an amination reagent with the catalyst according to any one of Items C1 to C7 in the presence of hydrogen for amination reaction;
or alternatively, the method comprises: screening a catalyst comprising the carrier as defined in any one of Items C1 to C7, and contacting the amination raw material, and the amination reagent with the screened catalyst in the presence of hydrogen for amination reaction.

C11, the method according to Item C11, wherein the amination conditions include: a mole ratio of hydrogen to the amination reagent and to the amination raw material of 1-5 : 2-30 : 1, a temperature of 110-220 °C, a pressure of 0.8-25 MPa, and a liquid phase volume space velocity of the amination raw material of 0.06-1 $m^3$/($m^3$·h);

and/or the amination raw material is at least one selected from the group consisting of C2-20 alcohols, C3-20 ketones, C2-20 alcohol amines and C2-20 aldehydes, preferably at least one of ethanol, acetaldehyde, n-propanol, propionaldehyde, isopropanol, n-butanol, butyraldehyde, isobutanol, isobutyraldehyde, 2-ethylhexanol, 2-ethylhexaldehyde, octanol, octanal, dodecanol, dodecanal, hexadecanol, hexadecanal, cyclopentanol, cyclohexanol, cyclooctanol, cyclododecanol, benzyl alcohol, benzaldehyde, phenethyl alcohol, phenylacetaldehyde, 1,4-butanediol, 1,4-butanedial, 1,5-pentanediol, 1,5-glutaraldehyde, 1,6-hexanediol, 1,6-hexanedial, 1,8-octanediol, 1,8-octanedial, ethanolamine, propanolamine, isopropanolamine, 6-aminohexanol, diethanolamine, dimethylethanolamine, acetone, ethylene glycol, 1,3-propanediol, and 1,12-dodecanediol;
and/or the amination reagent is at least one selected from the group consisting of ammonia, C1-12 primary amines and C1-12 secondary amines, preferably at least one of ammonia, monomethylamine, dimethylamine, methyl ethyl amine, monoethylamine and diethylamine.

C13, the method according to Item C12, wherein, where the amination raw material is a monohydric alcohol, the amination conditions include: a mole ratio of hydrogen to the amination reagent and to the amination raw material of 1-4 : 2-9 : 1, a temperature of 130-200 °C, a pressure of 1-2.5 MPa, and a liquid phase volume space velocity of the amination raw material of 0.1-0.8 $m^3$/($m^3$·h);

or, where the amination raw material is a ketone or an aldehyde, the amination conditions include: a mole ratio of hydrogen to the amination reagent and to the amination raw material of 1-4 : 2-6 : 1, a temperature of 110-180 °C, a pressure of 0.8-2.5 MPa, and a liquid phase volume space velocity of the amination raw material of 0.1-0.8

$m^3/(m^3 \cdot h)$;

or, where the amination raw material is an alcohol amine, the amination conditions include: a mole ratio of hydrogen to the amination reagent and to the amination raw material of 1-4 : 3-20 : 1, a temperature of 130-200 °C, a pressure of 1-15 MPa, and a liquid phase volume space velocity of the amination raw material of 0.06-0.8 $m^3/(m^3 \cdot h)$;

or, where the amination raw material is a mixture of 1,6-hexanediol, hexamethyleneimine and 6-amino-1-hexanol or a dihydric alcohol, the amination conditions include: a mole ratio of hydrogen to the amination reagent and to the amination raw material of 1-4 : 3-30 : 1, a temperature of 130-220 °C, a pressure of 1-25 MPa, and a liquid phase volume space velocity of the amination raw material of 0.1-0.8 $m^3/(m^3 \cdot h)$.

D1, a catalyst having a function of catalyzing the amination of alcohols, comprising a carrier, and an active metal component and a metal promoter supported on the carrier, wherein the active metal component is cobalt and/or nickel; the metal promoter is a combination of at least one Group VIB metal, at least one Group IB metal and at least one Group IIB metal.

D2, The catalyst according to Item D1, wherein the carrier comprises an alumina carrier, a doping element and an additional carrier selected from silica and/or molecular sieves; the carrier has an ammonia adsorption capacity of 0.25-0.6 mmol/g; the proportion of the pore volume of pores having a pore diameter in a range of 7-27 nm to the pore volume of the carrier is greater than 65%.

D3, the catalyst according to Item D1 or D2, wherein the content of the alumina carrier in the carrier is 70 wt% or more, preferably 80 to 97 wt%, based on the total amount of the alumina carrier and said additional carrier;

and/or the content of the doping element is 0.05-5 wt%, preferably 0.08-3 wt%, based on the weight of the carrier;
and/or the doping element in the carrier is a non-metallic element, preferably incorporated in the form of at least one selected from the group consisting of borate ion, fluoride ion, phosphate ion, sulfate ion and selenate ion;
and/or the carrier has an ammonia adsorption capacity of 0.3-0.6 mmol/g;
and/or the proportion of the pore volume of pores having a pore diameter in a range of 7-27 nm to the pore volume of the carrier is 70-90%, and the proportion of the pore volume of pores having a pore diameter of less than 7 nm to the pore volume of the carrier is 0-8%;
and/or the carrier has a specific surface area of 120-210 $m^2/g$;
and/or the carrier has a pore volume of 0.45-1.1 ml/g;
and/or the active metal component is present in an amount of 10-46g, preferably 18-38g, per 100g of the carrier;
and/or the content of the metal promoter is 0.1-10 g, preferably 0.5-6g, relative to 100 g of the carrier;
and/or the metal promoter comprises a Group VIB metal, a Group IB metal and a Group IIB metal at a weight ratio of 0.1-10 : 0.1-10 : 1, preferably 0.2-8 : 0.2-8 : 1;
and/or the Group VIB metal is selected from molybdenum and/or tungsten;
and/or, the Group IB metal is at least one selected from the group consisting of copper, silver and gold;
and/or the Group IIB metal is selected from zinc.

D4, the catalyst according to any one of Items D1 to D3, wherein the carrier is prepared by a method comprising the steps of: sequentially shaping, drying and calcining a mixture comprising the alumina precursor, the doping element and an additional carrier precursor, wherein said additional carrier precursor is selected from a silica precursor and/or a molecular sieve precursor.

D5, the catalyst according to Item D4, wherein the doping element is provided by at least one of boric acid, hydrofluoric acid, phosphoric acid, sulfuric acid, and selenic acid.

D6, the catalyst according to Item D4 or D5, wherein the alumina precursor is pseudo-boehmite, the pseudo-boehmite has a specific surface area of 260-400 $m^2/g$, and a pore volume of 0.8-1.2 ml/g.

D7, the catalyst according to any one of Items D4 to D6, wherein the drying conditions include: a temperature of 80-150 °C, and a time of 6-20 h;
and/or the calcining conditions include: a temperature of 500-1100 °C, and a time of 2-20 h.

D8, a method for producing the catalyst according to any one of Items D1 to D7, comprising: loading the active metal component and the metal promoter on the carrier.

D9, a carrier as defined in any one of Items D2 to D7.

D10, Use of the catalyst according to any one of Items D1 to D7, or the method according to Item D8, or the carrier according to Item D9, in the amination for producing organic amines.

D11, a method for producing organic amine, comprising the following steps: contacting an amination raw material and an amination reagent with the catalyst as defined in any one of Items D1 to D7 in the presence of hydrogen for amination reaction.

D12, the method according to Item D11, wherein the amination conditions include: a mole ratio of hydrogen to the amination reagent and to the amination raw material of 1-5 : 2-35 : 1, a temperature of 105-220 °C, a pressure of 0.7-25 MPa, and a liquid phase volume space velocity of the amination raw material of 0.06-1 m$^3$/(m$^3$·h);

and/or the amination raw material is at least one selected from the group consisting of C2-20 alcohols, C3-20 ketones, C2-20 alcohol amines and C2-20 aldehydes, preferably at least one of ethanol, acetaldehyde, n-propanol, propionaldehyde, isopropanol, n-butanol, butyraldehyde, isobutanol, isobutyraldehyde, 2-ethylhexanol, 2-ethylhexaldehyde, octanol, octanal, dodecanol, dodecanal, hexadecanol, hexadecanal, cyclopentanol, cyclohexanol, cyclooctanol, cyclododecanol, benzyl alcohol, benzaldehyde, phenethyl alcohol, phenylacetaldehyde, 1,4-butanediol, 1,4-butanedial, 1,5-pentanediol, 1,5-glutaraldehyde, 1,6-hexanediol, 1,6-hexanedial, 1,8-octanediol, 1,8-octanedial, ethanolamine, propanolamine, isopropanolamine, 6-aminohexanol, diethanolamine, dimethylethanolamine, acetone, ethylene glycol, 1,3-propanediol, and 1,12-dodecanediol;
and/or the amination reagent is at least one selected from the group consisting of ammonia, C1-12 primary amines and C1-12 secondary amines, preferably at least one of ammonia, monomethylamine, dimethylamine, methyl ethyl amine, monoethylamine and diethylamine.

D13, the method according to Item D12, wherein, where the amination raw material is a monohydric alcohol, the amination conditions include: a mole ratio of hydrogen to the amination reagent and to the amination raw material of 1-4 : 2-8 : 1, a temperature of 130-200 °C, a pressure of 1-2.5 MPa, and a liquid phase volume space velocity of the amination raw material of 0.1-0.8 m$^3$/(m$^3$·h);

or, where the amination raw material is a ketone or an aldehyde, the amination conditions include: a mole ratio of hydrogen to the amination reagent and to the amination raw material of 1-4 : 2-5 : 1, a temperature of 105-180 °C, a pressure of 0.7-2.5 MPa, and a liquid phase volume space velocity of the amination raw material of 0.1-0.8 m$^3$/(m$^3$·h);
or, where the amination raw material is an alcohol amine, the amination conditions include: a mole ratio of hydrogen to the amination reagent and to the amination raw material of 1-4 : 3-25 : 1, a temperature of 130-200 °C, a pressure of 5-18 MPa, and a liquid phase volume space velocity of the amination raw material of 0.06-0.8 m$^3$/(m$^3$·h);
or, where the amination raw material is a mixture of 1,6-hexanediol, hexamethyleneimine and 6-amino-1-hexanol or a dihydric alcohol, the amination conditions include: a mole ratio of hydrogen to the amination reagent and to the amination raw material of 1-4 : 3-35 : 1, a temperature of 130-220 °C, a pressure of 2-25 MPa, and a liquid phase volume space velocity of the amination raw material of 0.1-0.8 m$^3$/(m$^3$·h).

E1, a titanium-containing catalyst having an amination function, comprising a carrier, and an active metal component and optionally a metal promoter supported on the carrier, and the catalyst comprises alumina and titania at a weight ratio of 1.5-5 : 1, and the active metal component is cobalt and/or nickel.
E2, the titanium-containing catalyst according to Item E1, wherein the weight ratio of alumina to titania is 2-4.5 : 1;

and/or the active metal component is present in an amount of 13 to 40 g, preferably 20 to 36 g, per 100 g of the carrier;
and/or the content of the metal promoter is 0-10 g, preferably 2.5-8 g, relative to 100 g of the carrier;
and/or the metal promoter is at least one selected from the group consisting of Group VIB, Group VIIB, Group IB, Group IIB and lanthanide series elements, preferably at least one of Cr, Mo, W, Mn, Re, Cu, Ag, Au, Zn, La and Ce.

E4, the titanium-containing catalyst according to any one of Items E1 to E3, wherein the titanium-containing catalyst has an ammonia adsorption capacity of 0.3 to 0.4 mmol/g, a carbon dioxide adsorption capacity of 0.07 to 0.2 mmol/g, a specific surface area of 130-180 m$^2$/g, a pore volume of 0.55 to 0.75 ml/g, a proportion of the pore volume of pores having a pore radius of less than 4 nm to the total pore volume of less than 20%, and a proportion of the pore volume of pores having a pore radius of greater than 10 nm to the total pore volume of less than 15%;
and/or the carrier is doped with sulfur in an amount of 0.1 to 0.5 g, preferably 0.15 to 0.4g, per 100g of alumina and titania.
E5, a method for producing a titanium-containing catalyst having an amination function, comprising:

(1) subjecting pseudo-boehmite and titanium white powder to kneading, shaping and calcining to obtain a carrier, wherein the pseudo-boehmite and titanium white powder are used in such an amount that the weight ratio of alumina to titania in the carrier obtained is 2-5 : 1;
(2) loading an active metal component and optionally a metal promoter on the carrier obtained, wherein the active metal component comprises cobalt and/or nickel.

E6, the method according to Item E4, wherein the pseudo-boehmite and the titanium white powder are used in such an amount that the weight ratio of the alumina to the titania in the carrier obtained is from 2 to 4.5 : 1;

and/or, the pseudo-boehmite is prepared by at least one of carbonization method, organic aluminum hydrolysis method, aluminum sulfate method and nitric acid method, preferably by carbonization method or aluminum sulfate method;
and/or the titanium white powder is prepared by precipitation method and/or sol-gel method, and preferably the titanium white powder prepared by using titanyl sulfate as a raw material.

E7, the method according to Item E5, wherein the content of sulfate in the titanium white powder is 0.2 to 3 wt%.
E8, the method according to any one of Items E4 to E6, wherein the active metal component is used in such an amount that the active metal component is present in an amount of 13 to 40 g, preferably 20 to 36g, per 100 g of the carrier;

and/or the amount of the metal promoter is such that the metal promoter is present in an amount of 0 to 10 g, preferably 2.5 to 8g, per 100 g of the carrier;
and/or the metal promoter is at least one selected from the group consisting of Group VIB, Group VIIB, Group IB, Group IIB and lanthanide series elements, preferably at least one of Cr, Mo, W, Mn, Re, Cu, Ag, Au, Zn, La and Ce.

E8, a titanium-containing catalyst obtained by the method according to any one of Items E4 to E7.
E9, Use of a titanium-containing catalyst according to any one of Items E1 to E3 or E8, or the method according to any one of Items E4 to E7, in the amination of alcohol.
E10, a process for producing an organic amine, comprising: contacting ammonia and/or an organic amine with an alcohol in the presence of the titanium-containing catalyst according to any one of Items E1 to E3 or E8 for amination reaction;
or alternatively, the titanium-containing catalyst is prepared by the method according to any one of Items E4 to E7, and then ammonia and/or an organic amine is contacted with an alcohol in the presence of the resulting titanium-containing catalyst for amination reaction.

Examples

The present application will be further illustrated with reference to the following examples, but the present application is not limited thereto.
In the following examples and comparative examples, unless otherwise specified, reagents and starting materials used are commercially available products, which are chemically pure.
The test instrument and methods used in the following examples are as follows:

1) $NH_3$-TPD test

Test instrument: Autochem 2920 Full-automatic Chemical Adsorption Instrument (Automated Catalyst Characterization System), manufactured by MICROMERITICS, USA;
Test conditions: about 0.1g of sample was accurately weighed, put into a sample tube, heated to a temperature of 600 °C at a rate of 10 °C/min while purging with helium gas, stayed for 1h, and cooled to a temperature of 120 °C; the gas was changed to 10% $NH_3$-He mixed gas, adsorbed for 60min, then the gas was changed again to helium gas purging for 1h, counting was started after the baseline became stable; and the resultant was heated to a temperature of 600 °C at a rate of 10 °C/min, kept for 30min, the recording was stopped, and the experiment was completed. An integral computation was conducted on the peak area, obtaining an $NH_3$ desorption amount, and the desorption amount was used for characterizing the ammonia adsorption capacity of the sample.

2) $CO_2$-TPD test

Test instrument: Autochem 2920 Full-automatic Chemical Adsorption Instrument (Automated Catalyst Characterization System), manufactured by MICROMERITICS, USA;
Test conditions: about 0.1g of sample was accurately weighed, put into a sample tube, heated to a temperature of 600 °C at a rate of 10 °C/min while purging with helium gas, stayed for 1h, and cooled to a temperature of 120 °C,

the gas was changed to 10% $CO_2$-He mixed gas, adsorbed for 60min, then the gas was changed again to helium gas purging for 1h, counting was started after the baseline became stable; and the resultant was heated to a temperature of 600 °C at a rate of 10 °C/min, kept for 30min, the recording was stopped, and the experiment was completed. An integral computation was conducted on the peak area, obtaining a $CO_2$ desorption amount, and the desorption amount was used for characterizing the $CO_2$ adsorption capacity of the sample.

3) BET test

Test instrument: ASAP2420 Full-automatic Physicochemical Adsorption Analyzer (Automatic Micropore & Chemisorption Analyzer), manufactured by MICROMERITICS, USA;

Test conditions: test gas: $N_2$ (99.999% pure); degassing conditions: heating to 350 °C at a rate of 10 °C/min, and vacuumizing for 4 h; analysis conditions: conducting a full analysis on the mesopore isotherm to obtain the specific surface area and the pore volume.

4) XRD analysis

Test instrument: Empyrean X-ray diffractometer manufactured by PANalytical B.V., with an anode target of Cu target, and a Pixcel 3D detector;

Test conditions: tube voltage 40KV, tube current 40mA, divergence slit 1/4°, anti-divergence slit 1/2°, receiving slit height 7.5mm, scanning speed 0.013°/step, scanning range 5°-90°.

The grain size of the active metal component and the metal promoter, if present, was obtained by calculation using the Scherrer equation.

Example series I

The first type of embodiments of the present application are further illustrated in detail hereinbelow with reference to the examples of Example series I. In the following examples of Example series I, the pseudo-boehmite powder used had a ($Al_2O_3$) content on a dry basis of 72 wt%.

Example I-1

Calcium nitrate tetrahydrate (analytically pure), nitric acid and a dilute acid aqueous solution of phosphoric acid were sequentially added into pseudo-boehmite powder (with a specific surface area of 298 $m^2$/g, and a pore volume of 1.21 ml/g) while kneading, and after kneading the resultant was extruded into strips with a diameter of 5 mm, cut to a length of 4 mm, dried at 100 °C for 12 h, and then calcined at 720 °C for 8h to obtain a desired carrier, wherein the amount of calcium nitrate tetrahydrate (analytically pure) was 2.95g, the amount of the nitric acid was 6.5g, and the amount of the phosphoric acid was 0.63g, per 100g of the pseudo-boehmite powder used, calculated as $Al_2O_3$.

176.4 g of cobalt nitrate hexahydrate (industrial grade, with a purity of 98%) was dissolved in water to obtain a 182 mL solution, and the solution was loaded onto 100 g of the carrier obtained by spray impregnation in two times, dried at 120 °C for 8 hours after each time of spray impregnation, then calcined at 400 °C for 4 hours, then reduced with hydrogen while gradually increasing the temperature at a rate of 20 °C/h, and finally reduced at 430 °C for 3 hours, to obtain a catalyst A-I-1.

Example I-2

A potassium nitrate (analytically pure) aqueous solution, nitric acid and a dilute acid aqueous solution of boric acid were sequentially added into pseudo-boehmite powder (with a specific surface area of 286 $m^2$/g, and a pore volume of 0.88 ml/g) while kneading, extruded into granules of clover shape with a thickness of 3 mm, dried at 120 °C for 8h, and then calcined at 690 °C for 10h to obtain a desired carrier, wherein the amount of the potassium nitrate (analytically pure) was 0.26 g, the amount of the nitric acid was 5.2 g and the amount of the boric acid was 4.57 g, per 100g of the pseudo-boehmite powder used, calculated as $Al_2O_3$.

151.7 g of nickel nitrate hexahydrate (industrial grade, with a purity of 98%) was dissolved in water to obtain a 168mL solution, the solution was loaded onto 100 g of the carrier obtained by spray impregnation in two times, dried at 120 °C for 4 hours after each time of spray impregnation, then calcined at 390 °C for 4 hours, then reduced with hydrogen while gradually increasing the temperature at a rate of 20 °C/h, and finally reduced at 440 °C for 3 hours, to obtain a catalyst A-I-2.

Example I-3

A magnesium nitrate hexahydrate (analytically pure) aqueous solution, nitric acid and a dilute acid aqueous solution of sulfuric acid were sequentially added into pseudo-boehmite powder (with a specific surface area of 310 $m^2$/g, and a pore volume of 0.92 ml/g) while kneading, extruded into dentate spheres with a diameter of 4 mm, dried at 100 °C for 15 h, and then calcined at 780 °C for 10h to obtain a desired carrier, wherein the amount of magnesium nitrate hexahydrate (analytically pure) was 0.84 g, the amount of nitric acid was 6.1 g and the amount of sulfuric acid was 1.22 g, per 100g of the pseudo-boehmite powder used, calculated as $Al_2O_3$.
50.4 g of cobalt nitrate hexahydrate (industrial grade, with a purity of 98%) and 32.6g of a 50wt% aqueous solution of manganese nitrate were dissolved in water to obtain a 156mL solution, the solution was loaded onto 100 g of the carrier obtained by spray impregnation in two times, dried at 120 °C for 4 hours after each time of spray impregnation, then calcined at 400 °C for 4 hours, then reduced with hydrogen while gradually increasing the temperature at a rate of 20 °C/h, and finally reduced at 430 °C for 3 hours to obtain a catalyst A-I-3.

Example I-4

A bismuth nitrate pentahydrate (analytically pure) aqueous solution, nitric acid and a dilute acid aqueous solution of phosphoric acid were sequentially added into pseudo-boehmite powder (with a specific surface area of 321 $m^2$/g and a pore volume of 0.93 ml/g) while kneading, extruded into strips with a diameter of 5 mm, cut to a length of 4 mm, dried at 80 °C for 20h, and then calcined at 660 °C for 15h to obtain a desired carrier, wherein the amount of bismuth nitrate pentahydrate (analytically pure) was 1.86 g, the amount of nitric acid was 6.5 g and the amount of phosphoric acid was 1.9 g, per 100g of the pseudo-boehmite powder used, calculated as $Al_2O_3$.
75.6 g of cobalt nitrate hexahydrate (industrial grade, with a purity of 98%) and 50.6g of nickel nitrate hexahydrate (industrial grade, with a purity of 98%) were dissolved in water to obtain a 166 mL solution, and the solution was loaded onto 100 g of the carrier obtained by spray impregnation in two times, dried at 120 °C for 4 hours after each time of spray impregnation, then calcined at 400 °C for 4 hours, then reduced with hydrogen while gradually increasing the temperature at a rate of 20 °C/h, and finally reduced at 430 °C for 3 hours, to obtain a catalyst A-I-4.

Example I-5

A barium nitrate (analytically pure) aqueous solution, nitric acid and a dilute acid aqueous solution of boric acid were sequentially added into pseudo-boehmite powder (with a specific surface area of 275 $m^2$/g and a pore volume of 0.85 ml/g) while kneading, extruded into granules of clover shape with a thickness of 3 mm, dried at 150 °C for 6h, and calcined at 810 °C for 5h to obtain a desired carrier, wherein the amount of barium nitrate (analytically pure) was 0.19 g, the amount of nitric acid was 6.5 g and the amount of boric acid was 2.29 g, per 100g of the pseudo-boehmite powder used, calculated as $Al_2O_3$.
126.4 g of nickel nitrate hexahydrate (industrial grade, with a purity of 98%) and 2.9g of ammonium perrhenate (with a purity of 99%) were dissolved in water to obtain a 160 mL solution, and the solution was loaded onto 100 g of the carrier obtained by spray impregnation in two times, dried at 120 °C for 4 hours after each time of spray impregnation, then calcined at 390 °C for 4 hours, then reduced with hydrogen while gradually increasing the temperature at a rate of 20 °C/h, and finally reduced at 440 °C for 3 hours, to obtain a catalyst A-I-5.

Example I-6

A cesium nitrate (analytically pure) aqueous solution, nitric acid and a dilute acid aqueous solution of sulfuric acid were sequentially added into pseudo-boehmite powder (with a specific surface area of 269 $m^2$/g, and a pore volume of 0.86 ml/g) while kneading, extruded into dentate spheres with a diameter of 4 mm, dried at 120 °C for 8h, and then calcined at 830 °C for 4h to obtain a desired carrier, wherein the amount of cesium nitrate was 0.03 g, the amount of nitric acid was 6.2 g, and the amount of sulfuric acid was 0.09 g, per 100g of the pseudo-boehmite powder used, calculated as $Al_2O_3$.
201.6 g of cobalt nitrate hexahydrate (industrial grade, with a purity of 98%) was dissolved in water to obtain a 156mL solution; 7.4g of ammonium molybdate tetrahydrate (analytically pure) was dissolved in water to obtain a 78ml solution; the cobalt nitrate solution was loaded onto 100 g of the carrier obtained by spray impregnation in two times; and then the ammonium molybdate solution was loaded onto the carrier by spray impregnation in one time, dried for 4 hours at 120 °C after the spray impregnation, then calcined for 4 hours at 400 °C, then reduced with hydrogen while gradually increasing the temperature at a rate of 20 °C/h, and finally reduced for 3 hours at 430 °C to obtain a catalyst A-I-6.

Example I-7

A lanthanum nitrate hexahydrate (analytically pure) aqueous solution, nitric acid and a dilute acid aqueous solution of sulfuric acid were sequentially added into pseudo-boehmite powder (with a specific surface area of 259 m$^2$/g, and a pore volume of 0.88 ml/g) while kneading, after kneading the resultant was extruded into dentate spheres with a diameter of 4 mm, dried at 100 °C for 10 h, and then calcined at 980 °C for 5h to obtain a desired carrier, wherein the amount of lanthanum nitrate hexahydrate (analytically pure) was 0.47 g, the amount of nitric acid was 5g, and the amount of sulfuric acid was 0.61 g, per 100g of the pseudo-boehmite powder used, calculated as Al$_2$O$_3$.

100.8 g of cobalt nitrate hexahydrate (industrial grade, with a purity of 98) and 14.1g of copper nitrate trihydrate (analytically pure) were dissolved in water to obtain a 176 mL solution, the solution was loaded onto 100 g of the carrier obtained by spray impregnation in two times, dried at 120 °C for 4 hours after each time of spray impregnation, then calcined at 400 °C for 4 hours, then reduced with hydrogen while gradually increasing the temperature at a rate of 20 °C/h, and finally reduced at 430 °C for 3 hours to obtain a catalyst A-I-7.

Example I-8

A lanthanum nitrate hexahydrate (analytically pure) aqueous solution, nitric acid and a dilute acid aqueous solution of hydrofluoric acid were sequentially added into pseudo-boehmite powder (with a specific surface area of 291 m$^2$/g, and a pore volume of 0.93 ml/g) while kneading, extruded into dentate spheres with a diameter of 4 mm, dried at 90 °C for 12 h, and then calcined at 900 °C for 3h to obtain a desired carrier, wherein the amount of lanthanum nitrate hexahydrate (analytically pure) was 0.62 g, the amount of nitric acid was 5.5g and the amount of hydrofluoric acid was 0.05 g, per 100g of the pseudo-boehmite powder used, calculated as Al$_2$O$_3$.

126 g of cobalt nitrate hexahydrate (industrial grade, with a purity of 98%) and 25.3g of nickel nitrate hexahydrate (industrial grade, with a purity of 98%) were dissolved in water to obtain a 177mL solution, the solution was loaded onto 100 g of the carrier obtained by spray impregnation in 3 times, dried at 120 °C for 4 hours after each time of spray impregnation, then calcined at 400 °C for 4 hours, then reduced with hydrogen while gradually increasing the temperature at a rate of 20 °C/h, and finally reduced at 430 °C for 3 hours, to obtain a catalyst A-I-8.

Example I-9

A calcium nitrate tetrahydrate (analytically pure) aqueous solution, nitric acid and a dilute acid aqueous solution of hydrofluoric acid were sequentially added into pseudo-boehmite powder (with a specific surface area of 312 m$^2$/g, and a pore volume of 1.02 ml/g) while kneading, extruded into granules of clover shape with a thickness of 4 mm, dried at 100 °C for 8h, and then calcined at 930 °C for 3h to obtain a desired carrier, wherein the amount of calcium nitrate tetrahydrate (analytically pure) was 3.54g, the amount of nitric acid was 6.5 g, and the amount of hydrofluoric acid was 0.13 g, per 100g of the pseudo-boehmite powder used, calculated as Al$_2$O$_3$.

176.4 g of cobalt nitrate hexahydrate (industrial grade, with a purity of 98%) and 1.3g of silver nitrate (analytically pure) were dissolved in water to obtain a 188 mL solution, the solution was loaded onto 100 g of the carrier obtained by spray impregnation in 3 times, dried at 120 °C for 4 hours after each time of spray impregnation, then calcined at 390 °C for 4 hours, then reduced with hydrogen while gradually increasing the temperature at a rate of 20 °C/h, and finally reduced at 440 °C for 3 hours to obtain a catalyst A-I-9.

Example I-10

Silica gel powder (with a specific surface area of 385 m$^2$/g, and a pore volume of 0.95 ml/g) was used as a raw material, shaped by ball rolling with a dilute acid aqueous solution containing magnesium nitrate, nitric acid and sulfuric acid into dentate spheres with a diameter of 4 mm, dried at 80 °C for 15 hours, and then calcined at 750 °C for 8 hours to obtain a desired carrier, wherein the amount of magnesium nitrate hexahydrate (analytically pure) was 8.44g, the amount of nitric acid was 6.1 g and the amount of sulfuric acid was 4.28 g, per 100g of the silica gel powder, calculated as SiO$_2$.

The remaining of the procedure was the same as described in Example 3, obtaining a catalyst A-I-10.

Example I-11

A potassium nitrate (analytically pure) aqueous solution, nitric acid and a dilute acid aqueous solution of phosphoric acid were sequentially added into pseudo-boehmite powder (with a specific surface area of 261 m$^2$/g, and a pore volume of 0.83 ml/g) while kneading, extruded into dentate spheres with a diameter of 4mm, dried at 100 °C for 10h, and then calcined at 1030 °C for 5h to obtain a desired carrier, wherein the amount of potassium nitrate (analytically pure) was 1.29 g, the amount of nitric acid was 6.3 g, and the amount of phosphoric acid was 4.9 g, per 100g of the pseudo-boehmite powder used, calculated as $Al_2O_3$.

The remaining of the procedure was the same as described in Example 3, obtaining a catalyst A-I-11.

Example I-12

ZSM-5 powder (with a specific surface area of 354 m$^2$/g, a pore volume of 0.57 ml/g, a crystallinity of 97.5%, $SiO_2/Al_2O_3$ = 61) was kneaded, a calcium nitrate tetrahydrate (analytically pure) aqueous solution, nitric acid and a dilute acid aqueous solution of phosphoric acid were sequentially added thereto while kneading, the resultant was extruded into dentate spheres with a diameter of 3.0 mm, dried at 120 °C for 10 hours, and then calcined at 920 °C for 5 hours to obtain a desired carrier, wherein the amount of calcium nitrate tetrahydrate (analytically pure) was 1.56 g, the amount of nitric acid was 8.2g, and the amount of phosphoric acid was 2.9 g, per 100g of the ZSM-5 powder.

The remaining of the procedure was the same as described in Example 7, obtaining a catalyst A-I-12.

Example I-13

Preparation of the carrier was the same as described in Example I-7

2.56 g of palladium chloride (with a purity of 99.5%) and 38.2g of copper nitrate trihydrate (analytically pure) were dissolved in water to obtain a 171 mL solution, and the solution was loaded onto 100 g of the carrier obtained by spray impregnation in two times, dried at 120 °C for 4 hours after each time of spray impregnation, then calcined at 400 °C for 4 hours, then reduced with hydrogen by gradually increasing the temperature at a rate of 20 °C/h, and finally reduced at 230 °C for 3 hours, to obtain a catalyst A-I-13.

Comparative Example I-1

A catalyst was prepared as described in Example 5, except that the amount of boric acid used was 3.43 g, the amount of barium nitrate (analytically pure) used was 4.76g, and the amount of nitric acid used was 6.5 g, per 100g of the pseudo-boehmite powder used, calculated as $Al_2O_3$. The catalyst obtained was designated as D-I-1.

Comparative Example I-2

A catalyst was prepared as described in Example 3, except that only a dilute acid aqueous solution of nitric acid was added during the kneading process, wherein the amount of nitric acid used was 6.1g, per 100g of the pseudo-boehmite powder used, calculated as $Al_2O_3$. The catalyst obtained was designated as D-I-2.

Comparative Example I-3

A catalyst was prepared as described in Example 3, except that nitric acid and a dilute acid aqueous solution of phosphoric acid were added during the kneading process, wherein the amount of phosphoric acid used was 15.82g and the amount of nitric acid used was 6.2g, per 100g of the pseudo-boehmite powder used, calculated as $Al_2O_3$. The catalyst obtained was designated as D-I-3.

Test example I-1

The elemental composition of the carriers and the catalysts were analyzed by plasma emission spectroscopy, the content of the element (ion) other than the carrier was expressed in the weight of the element relative to 100g of the matrix, i.e. the carrier calculated on the basis of non-doping element (e.g. calculated on the basis of $Al_2O_3$, when pseudo-boehmite was used as the source of the carrier); the carriers obtained above were characterized by the methods of $NH_3$-TPD, $CO_2$-TPD, BET nitrogen adsorption desorption, and the results are shown in Table I-1.

Table I-1 Properties of the carriers of the examples and comparative examples

| Example. No. | Doping element | | | | Active metal component and metal promoter | | | | Ammonia adsorption capacity, mmol/g | $CO_2$ adsorption capacity, mmol/g | Specific surface area, $m^2/g$ | Pore volume, ml/g |
|---|---|---|---|---|---|---|---|---|---|---|---|---|
| | Species | Relative content, g | Species | Relative content, g | Active metal component | Content, g | Metal promoter | Content, g | | | | |
| Ex. I-1 | Ca | 0.5 | P | 0.2 | Co | 35 | - | - | 0.35 | 0.15 | 215 | 0.91 |
| Ex. I-2 | K | 0.1 | B | 0.8 | Ni | 30 | - | - | 0.35 | 0.13 | 208 | 0.84 |
| Ex. I-3 | Mg | 0.08 | S | 0.4 | Co | 10 | Mn | 5 | 0.4 | 0.13 | 196 | 0.78 |
| Ex. I-4 | Bi | 0.8 | P | 0.6 | Co+Ni | 15+10 | - | - | 0.35 | 0.12 | 220 | 0.83 |
| Ex. I-5 | Ba | 0.1 | B | 0.4 | Ni | 25 | Re | 2 | 0.35 | 0.13 | 200 | 0.8 |
| Ex. I-6 | Cs | 0.02 | S | 0.03 | Co | 40 | Mo | 4 | 0.31 | 0.13 | 185 | 0.78 |
| Ex. I-7 | La | 0.15 | S | 0.2 | Co | 20 | Cu | 5 | 0.43 | 0.11 | 138 | 0.64 |
| Ex. I-8 | La | 0.2 | F | 0.05 | Co+Ni | 25+5 | - | - | 0.33 | 0.17 | 173 | 0.59 |
| Ex. I-9 | Ca | 0.6 | F | 0.12 | Co | 35 | Ag | 0.8 | 0.27 | 0.23 | 188 | 0.62 |
| Ex. I-10 | Mg | 0.8 | S | 1.4 | Co | 10 | Mn | 5 | 0.46 | 0.26 | 192 | 0.72 |
| Ex. I-11 | K | 0.5 | P | 1.55 | Co | 10 | Mn | 5 | 0.52 | 0.23 | 135 | 0.74 |
| Ex. I-12 | Ca | 0.2 | P | 0.7 | Co | 20 | Cu | 5 | 0.59 | 0.16 | 249 | 0.51 |
| Ex. I-13 | La | 0.15 | S | 0.2 | Pd | 1.37 | Cu | 9 | 0.41 | 0.15 | 142 | 0.62 |
| Comp. Ex. I-1 | Ba | 2.5 | B | 0.6 | Ni | 25 | Re | 2 | 0.22 | 0.38 | 203 | 0.81 |
| Comp. Ex. I-2 | - | 0 | - | 0 | Co | 10 | Mn | 5 | 0.16 | 0.12 | 198 | 0.78 |
| Comp. Ex. I-3 | - | 0 | P | 5 | Co | 10 | Mn | 5 | 0.72 | 0.07 | 195 | 0.77 |

Test Example I-2

This test example illustrates a process for producing 1,6-hexanediamine by hydroamination of 1,6-hexanediol using the catalysts of the first type of embodiments of the present application.

100 mL of the catalysts obtained in the above examples were respectively measured out, loaded into a fixed bed reactor, activated with hydrogen at 220 °C for 2 hours, then cooled to 165 °C, the pressure of the system was raised to 8.8 MPa using hydrogen, then ammonia was metered into the reaction system via a metering pump, preheated to 100 °C and then sent to the upper end of the reactor, heated and melted 1,6-hexanediol was fed into the upper end of the reactor via a metering pump, hydrogen was stably introduced via a gas mass flowmeter, wherein the molar ratio of hydrogen to ammonia and to 1,6-hexanediol was 3 : 14 : 1, and the liquid phase volume space velocity of 1,6-hexanediol was 0.42 h$^{-1}$, a catalytic amination reaction was conducted in the reactor, at a reaction temperature of 185 °C and a reaction pressure of 8.8 MPa, and after the reaction became stable, the reaction solution was sampled and analyzed (namely after 360 h of reaction). The analysis results are shown in Table I-2.

The analysis of the sample was conducted by gas chromatography and was calibrated using a correction factor of a standard sample formulated;

The conversion and selectivity were calculated based on the molar content of each component in the reaction solution.

$$\begin{array}{l}\text{Conversion of}\\ \text{hexanediol}\end{array} = 100\% - \frac{\text{Molar content of hexanediol}}{\text{Molar content of (hexanediol + hexanediamine + hexamethyleneimine + aminohexanol) + Molar content of dimers of amine} \times 2} \times 100\%$$

$$\begin{array}{l}\text{Selectivity of}\\ \text{hexanediamine}\end{array} = \frac{\text{Molar content of hexanediamine}}{\text{Molar content of (hexanediamine + hexamethyleneimine + aminohexanol) + Molar content of dimers of amine} \times 2} \times 100\%$$

The selectivity of hexamethyleneimine was calculated by changing the numerator in the equation above for calculating the selectivity of hexanediamine to the molar content of hexamethyleneimine, the selectivity of aminohexanol was calculated by changing the numerator in the equation above for calculating the selectivity of hexanediamine to the molar content of aminohexanol, and so on, and the selectivity to "others" was calculated by changing the numerator in the equation above for calculating the selectivity of hexanediamine to the molar content of dimers of amine $\times 2$, the dimer of amine refers to the dimer of 1,6-hexanediamine (i.e. bis(hexamethylene) triamine, also known as N-(6-aminohexyl)-1,6-hexanediamine) and the dimer of 1,6-hexanediamine with hexamethyleneimine (i.e. N-(6-aminohexyl) hexamethyleneimine).

Table I-2 Test results for the catalysts of the examples and comparative examples

| Catalyst | Composition of catalyst | Conversion , % | Selectively, % | | | |
|---|---|---|---|---|---|---|
| | | | Hexanediamine | Hexamethyleneimine | Aminohexanol | Others |
| A-I-1 | $Co/Al_2O_3$ | 88 | 48.8 | 20.6 | 26.7 | 3.9 |
| A-I-2 | $Ni/Al_2O_3$ | 90 | 48.3 | 19.5 | 28.4 | 3.8 |
| A-I-3 | $Co-Mn/Al_2O_3$ | 92 | 51 | 19.2 | 26.5 | 3.3 |
| A-I-4 | $Co-Ni/Al_2O_3$ | 87 | 47.5 | 21.0 | 27.4 | 4.1 |
| A-I-5 | $Ni-Re/ Al_2O_3$ | 91 | 50.1 | 20.9 | 25.9 | 3.1 |
| A-I-6 | $Co-Mo/Al_2O_3$ | 86 | 48.7 | 17.6 | 30.2 | 3.5 |
| A-I-7 | $Co-Cu/Al_2O_3$ | 90 | 49.3 | 20.3 | 27.2 | 3.2 |
| A-I-8 | $Co-Ni/Al_2O_3$ | 91 | 48.8 | 20.2 | 27.3 | 3.7 |
| A-I-9 | $Co-Ag/Al_2O_3$ | 82 | 46.8 | 19.6 | 30.3 | 3.3 |
| A-I-10 | $Co-Mn/SiO_2$ | 78 | 45.5 | 20.1 | 29.6 | 4.8 |
| A-I-11 | $Co-Mn/Al_2O_3$ | 79 | 44.9 | 22.3 | 28.3 | 4.5 |
| A-I-12 | Co-Cu/ZSM-5 | 93 | 50.7 | 23.1 | 22.4 | 3.8 |
| A-I-13 | $Pd-Cu/ Al_2O_3$ | 90 | 50.1 | 21.9 | 25.0 | 3.0 |
| D-I-1 | $Ni-Re/Al_2O_3$ | 64 | 32.9 | 22.6 | 34.3 | 10.2 |
| D-I-2 | $Co-Mn/Al_2O_3$ | 56 | 33.1 | 23.7 | 35.8 | 7.4 |
| D-I-3 | $Co-Mn/Al_2O_3$ | 62 | 36.7 | 27.9 | 25.6 | 9.8 |

As can be seen from the data in Table I-2, the catalyst of the present application has higher conversion and higher activity than the comparative catalysts, indicating that the catalyst of the present application provides a faster reaction rate.

All the catalysts were tested for 360 hours and then discharged for characterization, and the catalysts D-I-1, D-I-2 and D-I-3 obtained in the comparative examples are found to have obviously reduced specific surface areas and pore volumes, and their carbon deposition amounts are respectively 11 wt%, 9.1 wt% and 8.9 wt%, while the catalysts obtained in the examples of the present application have no obvious change in the specific surface areas and the pore volumes, and their carbon deposition amounts are lower than 2 wt%.

In addition, when the catalysts A-I-1 to A-I-11 were subjected to catalytic reaction for 1000 hours, a sample of the reaction solution was taken and analyzed, and the conversion and the selectivity are not obviously changed compared with 360 hours, namely the reduction value of the conversion is not higher than 2%, the reduction value of the selectivity is not higher than 1%, while the conversion and the selectivity of the catalysts D-I-1 to D-I-3 are obviously reduced compared with 360 hours when the catalysts D-I-1 to D-I-3 are subjected to catalytic reaction for 1000 hours, and the conversion isrespectively reduced to 31%, 28% and 35%. The selectivity of hexanediamine was reduced to 25%, 23% and 26%, respectively.

Test Example I-3

This test example illustrates a process for producing ethylamine by hydroamination of ethanol using a catalyst according to the first type of embodiments of the present application

100 mL of the catalyst A-I-3 obtained in Example I-3 was measured out, loaded into a fixed bed reactor, activated with hydrogen at 220 °C for 2 hours, then cooled to 160 °C, the pressure of the system was raised to 1.75 MPa using hydrogen, then ammonia was metered into the reaction system via a metering pump, preheated to 110 °C, then sent to the upper end of the reactor, ethanol was fed into the upper end of the reactor via a metering pump, hydrogen was stably introduced via a gas mass flowmeter, the molar ratio of hydrogen to ammonia and to ethanol was 2: 5 : 1, the liquid phase volume space velocity of ethanol was 0.5 h$^{-1}$, a catalytic amination reaction was conducted in the reactor at a reaction temperature of 175 °C and a reaction pressure of 1.75 MPa, and after the reaction became stable, the reaction solution was sampled and analyzed (the analysis conditions, and the methods for calculating the conversion and the selectivity are similar to those of Test Example I-2), the analysis results are shown in Table I-3.

Table I-3 Results of Test Example I-3

|  | Ethanol conversion (%) | Selectivity (%) | | |
|---|---|---|---|---|
|  |  | Monoethylamine | Diethylamine | Triethylamine |
| 360h | 98.95 | 27.4 | 47.3 | 24.8 |
| 1000h | 98.91 | 27.3 | 47.4 | 24.8 |

Test Example I-4

This test example illustrates a process for producing ethylenediamine by hydroamination of ethanolamine using a catalyst according to the first type of embodiments of the present application

100 mL of the catalyst A-I-3 obtained in Example I-3 was measured out, loaded into a fixed bed reactor, activated with hydrogen at 220 °C for 2 hours, then cooled to 180 °C, the pressure of the system was raised to 9.6 MPa using hydrogen, then ammonia was metered into the reaction system via a metering pump, preheated to 100 °C and sent to the upper end of the reactor, then ethanolamine was fed into the upper end of the reactor via a metering pump, hydrogen was stably introduced via a gas mass flowmeter, the molar ratio of hydrogen to ammonia and to ethanolamine was 3 : 12 : 1, the liquid phase volume space velocity of ethanolamine was 0.5 h$^{-1}$, a catalytic amination reaction was conducted in the reactor at a reaction temperature of 180 °C and a reaction pressure of 9.6 MPa, and after the reaction became stable, the reaction solution was sampled and analyzed (the analysis conditions, and the methods for calculating the conversion and the selectivity are similar to those of Test Example I-2), the analysis results are shown in Table I-4.

Table I-4 Results of Test Example I-4

| | Conversion of ethanolamine (%) | Selectivity (%) | | | | | |
|---|---|---|---|---|---|---|---|
| | | Ethylenediamine | Piperazine | Diethylenetriamine | Hydroxyethyl ethylenediamine | N-aminoethyl-piperazine | N-hydroxyethyl-piperazine |
| 360h | 90.0 | 57.6 | 25.3 | 7.68 | 8.31 | 0.50 | 0.40 |
| 1000h | 90.2 | 57.7 | 25.2 | 7.55 | 8.32 | 0.56 | 0.47 |

Test Example I-5

This test example illustrates a process for producing hexanediamine from a mixture of 1,6-hexanediol, hexamethyleneimine and aminohexanol using the catalyst according to the first type of embodiments of the present application.

100 mL of the catalyst A-I-3 obtained in Example I-3 was measured out, loaded into a fixed bed reactor, activated with hydrogen at 220 °C for 2 hours, then cooled to 170 °C, the pressure of the system was raised to 8.2 MPa using hydrogen, then ammonia was metered into the reaction system via a metering pump, preheated to 110 °C and then sent to the upper end of the reactor, a mixed solution of 53 wt% of 1,6-hexanediol, 30 wt% of hexamethyleneimine and 17 wt% of 6-amino-1-hexanol was fed into the upper end of the reactor via a metering pump, hydrogen was stably introduced via a gas mass flowmeter, the molar ratio of hydrogen to ammonia and to the total amount of the three substances in the mixed solution was 4 : 15 : 1, the liquid phase volume space velocity of the mixed solution was 0.5 h$^{-1}$, a catalytic amination reaction was conducted in the reactor at a reaction temperature of 190 °C and a reaction pressure of 8.2 MPa, and after the reaction became stable, the reaction solution was sampled and analyzed (the analysis conditions, and the methods for calculating the conversion and the selectivity are similar to those of Test Example I-2). The analysis results are shown in Table I-5.

Table I-5 Results of Test Example I-5

|  | Conversion of hexanediol (%) | Selectivity (%) | | |
|---|---|---|---|---|
|  |  | Hexanediamine | Hexamethylene -imine | Aminohexanol |
| 360h | 98.0 | 98.4 | 0.8 | 0.2 |
| 1000h | 98.1 | 98.5 | 0.7 | 0.2 |

Example series II

The second type of embodiments of the present application are further illustrated hereinbelow in detail with reference to the examples of Example series II. In the following examples of Example series II, pseudo-boehmite powders was produced by aluminum sulfate method and had a ($Al_2O_3$) content on a dry basis of 72 wt%; the silica sol was purchased from Qingdao Ocean Chemical Co., Ltd, under a trade name of JN-40.

Example II-1

Pseudo-boehmite powder (with a specific surface area of 346 m$^2$/g, a pore volume of 1.13 ml/g, a phosphorus content of 0.22 g, per 100g of the powder calculated as $Al_2O_3$) was kneaded with a dilute acid aqueous solution containing 15 vol% of nitric acid, extruded into strips with a diameter of 5 mm, dried at 120 °C for 12 hours, and then calcined at 800 °C for 5 hours to obtain a carrier. The parameters of the carrier are shown in Table II-1.

186.5 g of cobalt nitrate hexahydrate (industrial grade, with a purity of 98%), 19.5 g of a 50wt% aqueous manganese nitrate solution and 14.13 g of copper nitrate trihydrate were dissolved in water to obtain a 132mL solution, and the solution was loaded onto 100 g of the carrier obtained by spray impregnation in two times, dried at 120 °C for 4 hours after each time of spray impregnation, then calcined at 400 °C for 2 hours, and then reduced with hydrogen while gradually increasing the temperature at a rate of 20 °C/h, and finally reduced at 480 °C for 2 h to obtain a catalyst A-II-1.

Example II-2

Pseudo-boehmite powder (with a specific surface area of 333 m$^2$/g, a pore volume of 1.04 ml/g, a boron content of 0.53 g, per 100g of the powder calculated as $Al_2O_3$) was kneaded with a dilute acid aqueous solution containing 10 vol% of nitric acid, extruded into granules of clover shape with a thickness of 3 mm, dried at 150 °C for 6 hours, and then calcined at 820 °C for 4 hours to obtain a carrier. The parameters of the carrier are shown in Table II-1.

151.7 g of nickel nitrate hexahydrate (industrial grade, with a purity of 98%), 13.0 g of a 50wt% aqueous manganese nitrate solution and 8.48 g of copper nitrate trihydrate were dissolved in water to obtain a 148 mL solution, and the solution was loaded onto 100 g of the carrier obtained by spray impregnation in two times, dried at 120 °C for 4 hours after each time of spray impregnation, then calcined at 400 °C for 4 hours. 1.12g of ammonium perrhenate was dissolved in water to obtain a 78 mL solution, the solution was loaded onto the intermediate product obtained above by spray impregnation, dried at 120 °C for 4 hours, and then calcined at 370 °C for 4 hours, and then reduced with hydrogen while gradually increasing the temperature at a rate of 20 °C/h, and finally reduced at 450 °C for 3 h to obtain a catalyst A-II-2.

Example II-3

Pseudo-boehmite powder (with a specific surface area of 369 $m^2$/g, a pore volume of 0.99 ml/g, sulfur content of 2.88 g, per 100g of the powder calculated as $Al_2O_3$) and silica sol were mixed uniformly, kneaded with a dilute acid aqueous solution containing 15 vol% of nitric acid, extruded into dentate spheres with a diameter of 4 mm, dried at 100 °C for 20 h, and then calcined at 840 °C for 5h to obtain a carrier. The parameters of the carrier are shown in Table II-1.
45.4 g of cobalt nitrate hexahydrate (industrial grade, with a purity of 98%) and 1.57g of silver nitrate were dissolved in water to obtain a 102mL solution, and the solution was loaded onto 100 g of the carrier obtained by spray impregnation in two times, dried at 120 °C for 4 hours after each time of spray impregnation, and then calcined at 400 °C for 4 hours. 1.50 g of ammonium perrhenate was dissolved in water to obtain a 70mL solution, the solution was loaded onto the intermediate product obtained above by spray impregnation, dried at 120 °C for 4 hours and then calcined at 390 °C for 4 hours, and then reduced with hydrogen while gradually increasing the temperature at a rate of 20 °C/h, and finally reduced at 420 °C for 4 h to obtain a catalyst A-II-3.

Example II-4

Pseudo-boehmite powder (with a specific surface area of 375 m2/g, a pore volume of 1.15 ml/g, a fluorine content of 0.08g, per 100g of the powder calculated as $Al_2O_3$) and ZSM-5 (commercially available, produced by Nankai University, $SiO_2$/$Al_2O_3$=45 (molar ratio)) were mixed uniformly, kneaded with a dilute acid aqueous solution containing 12 vol% of nitric acid, extruded into cylindrical bars with a diameter of 4 mm, dried at 120 °C for 8h, and then calcined at 810 °C for 4h to obtain a carrier. The parameters of the carrier are shown in Table II-1.
126.0 g of cobalt nitrate hexahydrate (industrial grade, with a purity of 98%) and 0.79 g of silver nitrate were dissolved in water to obtain a 192 mL solution, and the solution was loaded onto 100 g of the carrier obtained by spray impregnation in two times, dried at 120 °C for 4 hours after each time of spray impregnation, then calcined at 380 °C for 4 hours. 8.70 g of ammonium perrhenate was dissolved in water to obtain a 81mL solution, the solution was loaded onto the intermediate product obtained above by spray impregnation, dried at 120 °C for 4 hours and then calcined at 400 °C for 3 hours, and then reduced with hydrogen while gradually increasing the temperature at a rate of 20 °C/h, and finally reduced at 420 °C for 4 h to obtain a catalyst A-II-4.

Example II-5

Pseudo-boehmite powder (with a specific surface area of 298 $m^2$/g, a pore volume of 0.85 ml/g, a phosphorus content of 0.18 g, per 100g of the powder calculated as $Al_2O_3$) was kneaded with a dilute acid aqueous solution containing 15 vol% of nitric acid, extruded into cylindrical bars with a diameter of 2.5mm, dried at 110 °C for 14 h, and then calcined at 890 °C for 4h to obtain a carrier. The parameters of the carrier are shown in Table II-1.
75.6 g of cobalt nitrate hexahydrate (industrial grade, with a purity of 98%), 13.0 g of a 50 wt% aqueous manganese nitrate solution and 3.15 g of silver nitrate were dissolved in water to obtain a 112 mL solution, and the solution was loaded onto 100 g of the carrier obtained by spray impregnation in two times, dried at 120 °C for 4 hours after each time of spray impregnation, and then calcined at 380 °C for 4 hours. 2.90 g of ammonium perrhenate was dissolved in water to obtain a 70mL solution, the solution was loaded onto the intermediate product obtained above by spray impregnation, dried at 120 °C for 4 hours and then calcined at 400 °C for 2 hours, and then reduced with hydrogen while gradually increasing the temperature at a rate of 20 °C/h, and finally reduced at 450 °C for 3 h to obtain a catalyst A-II-5.

Example II-6

Pseudo-boehmite powder (with a specific surface area of 348 m2/g, a pore volume of 1.09 ml/g, and a boron content of 3.66 g, per 100g of the powder calculated as $Al_2O_3$) and silica sol were mixed uniformly, kneaded with a dilute acid aqueous solution containing 15 vol% of nitric acid, extruded into cylindrical bars with a diameter of 4 mm, dried at 120 °C for 7h, and then calcined at 810 °C for 6h to obtain a carrier. The parameters of the carrier are shown in Table II-1.
126.4 g of nickel nitrate hexahydrate (industrial grade, with a purity of 98%), 26.1 g of a 50 wt% aqueous manganese nitrate solution and 1.57 g of silver nitrate were dissolved in water to obtain a 146mL solution, and the solution was loaded onto 100 g of the carrier obtained by spray impregnation in two times, dried at 120 °C for 4 hours after each time of spray impregnation, and then calcined at 380 °C for 4 hours. 4.40 g of ammonium perrhenate was dissolved in water to obtain a 74mL solution, the solution was loaded onto the intermediate product obtained above by spray impregnation, dried at 120 °C for 4 hours and then calcined at 350 °C for 6 hours, and then reduced with hydrogen while gradually increasing the temperature at a rate of 20 °C/h, and finally reduced at 440 °C for 3 h to obtain a catalyst A-II-6.

Example II-7

Pseudo-boehmite powder (with a specific surface area of 374 $m^2$/g, a pore volume of 1.18 ml/g, a sulfur content of 0.81 g, per 100g of the powder calculated as $Al_2O_3$) and silica sol were mixed uniformly, kneaded with a dilute acid aqueous solution containing 10 vol% of nitric acid, extruded into cylindrical bars with a diameter of 4 mm, dried at 120 °C for 7h, and then calcined at 750 °C for 6h to obtain a carrier. The parameters of the carrier are shown in Table II-1.

201.6 g of cobalt nitrate hexahydrate (industrial grade, with a purity of 98%), 11.30 g of copper nitrate trihydrate and 9.80 g of a 50 wt% aqueous manganese nitrate solution were dissolved in water to obtain a 132 mL solution, and the solution was loaded onto 100 g of the carrier obtained by spray impregnation in two times, dried at 120 °C for 4 hours after each time of spray impregnation, then calcined at 380 °C for 4 hours. 4.70 g of ammonium perrhenate was dissolved in water to obtain a 74mL solution, the solution was loaded onto the intermediate product obtained above by spray impregnation, dried at 120 °C for 4 hours and then calcined at 400 °C for 2 hours, and then reduced with hydrogen while gradually increasing the temperature at a rate of 20 °C/h, and finally reduced at 460 °C for 2 h to obtain a catalyst A-II-7.

Example II-8

Pseudo-boehmite powder (with a specific surface area of 355 $m^2$/g, a pore volume of 1.05 ml/g, and a sulfur content of 0.88 g, per 100g of the powder calculated as $Al_2O_3$) and silica sol were mixed uniformly, kneaded with a dilute acid aqueous solution containing 10 vol% of nitric acid, extruded into cylindrical bars with a diameter of 4 mm, dried at 120 °C for 7h, and then calcined at 810 °C for 6h to obtain a carrier. The parameters of the carrier are shown in Table II-1.

100.8 g of cobalt nitrate hexahydrate (industrial grade, with a purity of 98%), 14.30 g of copper nitrate trihydrate and 1.57 g of silver nitrate were dissolved in water to obtain a 116mL solution, and the solution was loaded onto 100 g of the carrier obtained by spray impregnation in two times, dried at 120 °C for 4 hours after each time of spray impregnation, then calcined at 360 °C for 4 hours, and then reduced with hydrogen while gradually increasing the temperature at a rate of 20 °C/h, and finally reduced for 5 h at 420 °C to obtain a catalyst A-II-8.

Example II-9

Pseudo-boehmite powder (with a specific surface area of 380 $m^2$/g, a pore volume of 1.01 ml/g, a fluorine content of 0.82 g, per 100g of the powder calculated as $Al_2O_3$) and silica sol were mixed uniformly, kneaded with a dilute acid aqueous solution containing 15 vol% of nitric acid, extruded into cylindrical bars with a diameter of 4 mm, dried at 120 °C for 7h, and then calcined at 810 °C for 6h to obtain a carrier. The parameters of the carrier are shown in Table II-1.

176.4 g of cobalt nitrate hexahydrate (industrial grade, with a purity of 98%), 13.0 g of a 50 wt% manganese nitrate aqueous solution and 1.57 g of silver nitrate were dissolved in water to obtain a 154mL solution, and the solution was loaded onto 100 g of the carrier obtained by spray impregnation in two times, dried at 120 °C for 4 hours after each time of spray impregnation, then calcined at 380 °C for 4 hours. 1.50 g of ammonium perrhenate was dissolved in water to obtain a 74mL solution, the solution was loaded onto the intermediate product obtained above by spray impregnation, dried at 120 °C for 4 hours and then calcined at 380 °C for 3 hours, and then reduced with hydrogen while gradually increasing the temperature at a rate of 20 °C/h, and finally reduced at 450 °C for 3 h to obtain a catalyst A-II-9.

Example II-10

Pseudo-boehmite powder (with a specific surface area of 328 $m^2$/g, a pore volume of 0.97 ml/g, a sulfur content of 0.95 g, per 100g of the powder calculated as $Al_2O_3$) was kneaded with a dilute acid aqueous solution containing 5 vol% of nitric acid, extruded into cylindrical bars with a diameter of 4 mm, dried at 120 °C for 7h, and then calcined at 810 °C for 6h to obtain a carrier. The parameters of the carrier are shown in Table II-1.

226.8 g of cobalt nitrate hexahydrate (industrial grade, with a purity of 98%), 26.1 g of a 50wt% aqueous manganese nitrate solution and 0.79 g of silver nitrate were dissolved in water to obtain a 162mL solution, and the solution was loaded onto 100 g of the carrier obtained by spray impregnation in two times, dried at 120 °C for 4 hours after each time of spray impregnation, then calcined at 380 °C for 4 hours. 0.7 g of ammonium perrhenate was dissolved in water to obtain a 74mL solution, the solution was loaded onto the intermediate product obtained above by spray impregnation, dried at 120 °C for 4 hours, and then calcined at 420 °C for 2 hours, and then reduced with hydrogen while gradually increasing the temperature at a rate of 20 °C/h, and finally reduced at 430 °C for 4 h to obtain a catalyst A-II-10.

Example II-11

Pseudo-boehmite powder (with a specific surface area of 304 m$^2$/g, and a pore volume of 1.06 ml/g, and a phosphorus content of 4.6 g, per 100g of the powder calculated as Al$_2$O$_3$) and silica sol were mixed uniformly, kneaded with a dilute acid aqueous solution containing 5 vol% of nitric acid, extruded into cylindrical bars with a diameter of 4 mm, dried at 120 °C for 7h, and then calcined at 810 °C for 6h to obtain a carrier. The parameters of the carrier are shown in Table II-1.

141.1 g of cobalt nitrate hexahydrate (industrial grade, with a purity of 98%), 13.0 g of a 50 wt% manganese nitrate aqueous solution and 0.79 g of silver nitrate were dissolved in water to obtain a 152mL solution, and the solution was loaded onto 100 g of the carrier obtained by spray impregnation in two times, dried at 120 °C for 4 hours after each time of spray impregnation, then calcined at 380 °C for 4 hours. 2.90 g of ammonium perrhenate was dissolved in water to obtain a 74mL solution, the solution was loaded onto the intermediate product obtained above by spray impregnation, dried at 120 °C for 4 hours and then calcined at 400 °C for 4 hours, and then reduced with hydrogen while gradually increasing the temperature at a rate of 20 °C/h, and finally reduced at 460 °C for 2 h to obtain a catalyst A-II-11.

Example II-12

A catalyst was prepared as described in Example II-3, except that the pseudo-boehmite powder used was free of doping elements, and had a specific surface area of 298 m$^2$/g and a pore volume of 1.08 ml/g, and the catalyst obtained was designated as A-II-12.

Comparative Example II-1

Pseudo-boehmite powder (with a specific surface area of 376 m$^2$/g, a pore volume of 1.01 ml/g and no sulfur) and silica sol were mixed uniformly, kneaded with a dilute acid aqueous solution containing 10 vol% of nitric acid and extruded into dentate spheres with a diameter of 4 mm, dried at 120 °C for 5 h, and then calcined at 860 °C for 6h to obtain a carrier. The parameters of the carrier are shown in Table II-1.

The remaining of the procedure was the same as described in Example 8, obtaining a catalyst D-II-1.

Comparative Example II-2

Pseudo-boehmite powder (with a specific surface area of 358 m$^2$/g, a pore volume of 1.15 ml/g and no sulfur) and silica sol were mixed uniformly, kneaded with a dilute acid aqueous solution containing 10 vol% of nitric acid, and extruded into dentate spheres with a diameter of 4 mm, dried at 100 °C for 12 h, and then calcined at 890 °C for 6h to obtain a carrier. The parameters of the carrier are shown in Table II-1.

226.8 g of cobalt nitrate hexahydrate (industrial grade, with a purity of 98%) and 0.79 g of silver nitrate were dissolved in water to obtain a 162mL solution, and the solution was loaded onto 100 g of the carrier obtained by spray impregnation in two times, dried at 120 °C for 4 hours after each time of spray impregnation, then calcined at 380 °C for 4 hours, and then reduced with hydrogen while gradually increasing the temperature at a rate of 20 °C/h, and finally reduced at 450 °C for 4 h to obtain a catalyst D-II-2.

Test Example II-1

The elemental composition of the carriers and the catalysts was analyzed by plasma emission spectroscopy, wherein the contents of the doping element, the active metal component and the metal promoter are expressed by weight relative to 100g of the matrix; the carriers obtained above were characterized by the NH$_3$-TPD, BET nitrogen adsorption-desorption methods, and the results are shown in Table II-1.

Table II-1 Properties of the carriers of the examples and comparative examples

| Example No. | Active metal component and content thereof, g | Metal promoter and content thereof, g | Matrix and composition thereof, (wt%) | Specific surface area, m²/g | Pore volume, ml/g | Ammonia adsorption capacity, mmol/g | Distribution of pores having a pore diameter of 7-27nm | Distribution of pores having a pore diameter < 7nm | Doping element and content thereof, g |
|---|---|---|---|---|---|---|---|---|---|
| Ex. II-1 | Co 37 | Mn 2/Cu 5 | $Al_2O_3$ | 150 | 0.71 | 0.33 | 0.75 | 0.03 | P 0.22 |
| Ex. II-2 | Ni 30 | Mn 2/Cu 3 | $Al_2O_3$ | 158 | 0.79 | 0.35 | 0.72 | 0.07 | B 0.53 |
| Ex. II-3 | Co 9 | Re 1/Ag 1 | $Al_2O_3$ 78/$SiO_2$ 22 | 144 | 0.56 | 0.56 | 0.71 | 0.06 | S 2.25 |
| Ex. II-4 | Co 25 | Re 6/Ag 0.5 | $Al_2O_3$ 85/ZSM-5 15 | 180 | 1.01 | 0.38 | 0.76 | 0.04 | F 0.07 |
| Ex. II-5 | Co 15 | Mn 2/Re 2/Ag 2 | $Al_2O_3$ | 132 | 0.61 | 0.32 | 0.73 | 0.05 | P 0.18 |
| Ex. II-6 | Ni 25 | Mn 4/Re 3/Ag 1 | $Al_2O_3$ 80/$SiO_2$ 20 | 188 | 0.78 | 0.46 | 0.7 | 0.02 | B 2.93 |
| Ex. II-7 | Co 40 | Mn 1.5/Re 3.2/Cu 4 | $Al_2O_3$ 82/$SiO_2$ 18 | 171 | 0.71 | 0.45 | 0.72 | 0.08 | S 0.66 |
| Ex. II-8 | Co 20 | Mn 3/Cu 5/Ag 1 | $Al_2O_3$ 86/$SiO_2$ 14 | 165 | 0.63 | 0.48 | 0.76 | 0.06 | S 0.76 |
| Ex. II-9 | Co 35 | Mn 2/Re 1/Ag 1 | $Al_2O_3$ 89/$SiO_2$ 11 | 181 | 0.82 | 0.55 | 0.74 | 0.05 | F 0.73 |
| Ex. II-10 | Co 45 | Mn 4/Re 0.5/Ag 0.5 | $Al_2O_3$ | 178 | 0.86 | 0.52 | 0.71 | 0.04 | S 0.95 |
| Ex. II-11 | Co 28 | Mn 2/Re 2/Ag 0.5 | $Al_2O_3$ 95/$SiO_2$ 5 | 176 | 0.81 | 0.46 | 0.73 | 0.07 | P 4.37 |
| Ex. II-12 | Co 9 | Re 1/Ag 1 | $Al_2O_3$ 78/$SiO_2$ 22 | 135 | 0.59 | 0.28 | 0.82 | 0.06 | - |
| Comp. Ex. II-1 | Co 20 | Mn 3/Cu 5/Ag 1 | $Al_2O_3$ 92/$SiO_2$ 8 | 178 | 0.75 | 0.23 | 0.65 | 0.12 | - |
| Comp. Ex. II-2 | Co 45 | Ag 0.5 | $Al_2O_3$ 96/$SiO_2$ 4 | 162 | 0.62 | 0.20 | 0.69 | 0.04 | - |

Test Example II-2

This test example illustrates a process for producing 1,6-hexanediamine by hydroamination of 1,6-hexanediol using the catalyst of the second type of embodiments of the present application.

100 mL of the catalysts obtained in the examples and comparative examples were respectively measured out, loaded into a fixed bed reactor, activated with hydrogen at 220 °C for 2 hours, then cooled to 168 °C, the pressure of the system was raised to 9.5 MPa using hydrogen, then ammonia was metered into the reaction system via a metering pump, preheated to 155 °C and then sent to the upper end of the reactor, then heated and melted 1,6-hexanediol was fed into the upper end of the reactor via a metering pump, hydrogen was stably introduced via a gas mass flowmeter, wherein the molar ratio of hydrogen to ammonia and to 1,6-hexanediol was 3 : 8 : 1, the liquid phase volume space velocity of 1,6-hexanediol was 0.5 $h^{-1}$, a catalytic amination reaction was conducted in the reactor at a reaction temperature of 200 °C and a reaction pressure of 11 MPa, and after the reaction became stable (namely after 500 h of reaction), the reaction solution was sampled and analyzed. The analysis results are shown in Table II-2.

The analysis of the sample was conducted by gas chromatography, and was calibrated using a correction factor of a standard sample formulated.

The conversion and selectivity were calculated based on the molar content of each component in the reaction solution, and the calculation methods were the same as described in Test Example I-2.

Table II-2 Test results for the catalysts of the examples and comparative examples

| Catalyst | Composition of catalyst | Conversion, % | Selectively, % | | | |
|---|---|---|---|---|---|---|
| | | | Hexanediamine | Hexamethyleneimine | Aminohexanol | Others |
| A-II-1 | Co-Mn-Cu/Al$_2$O$_3$ | 89.6 | 49.1 | 23.2 | 24.5 | 3.2 |
| A-II-2 | Ni-Mn-Cu/Al$_2$O$_3$ | 91.3 | 50.5 | 24.8 | 21.6 | 3.1 |
| A-II-3 | Co-Re-Ag/Al$_2$O$_3$-SiO$_2$ | 89.5 | 51.2 | 19.2 | 26.8 | 2.8 |
| A-II-4 | Co-Re-Ag/Al$_2$O$_3$-ZSM-5 | 90.4 | 47.9 | 21.0 | 27.4 | 3.7 |
| A-II-5 | Co-Mn-Re-Ag/ Al$_2$O$_3$ | 92.8 | 45.5 | 20.1 | 31.3 | 3.1 |
| A-II-6 | Ni-Mn-Re-Ag/Al$_2$O$_3$-SiO$_2$ | 89.6 | 49.8 | 19.8 | 26.9 | 3.5 |
| A-II-7 | Co-Mn-Re-Cu/Al$_2$O$_3$-SiO$_2$ | 92.3 | 52 | 23.1 | 21.3 | 3.6 |
| A-II-8 | Co-Mn-Cu-Ag/Al$_2$O$_3$-SiO$_2$ | 85.1 | 48.8 | 20.2 | 27.9 | 3.1 |
| A-II-9 | Co-Mn-Re-Ag/Al$_2$O$_3$-SiO$_2$ | 84.2 | 49 | 22 | 25.1 | 3.9 |
| A-II-10 | Co-Zn/Al$_2$O$_3$ | 82.1 | 46.1 | 19.9 | 29.9 | 4.1 |
| A-II-11 | Co-Mn-Re-Ag/Al$_2$O$_3$-SiO$_2$ | 83.2 | 45.8 | 21.8 | 29.8 | 2.6 |
| A-II-12 | Co-Re-Ag/Al$_2$O$_3$-SiO$_2$ | 72.3 | 37.9 | 25.1 | 30.5 | 6.5 |
| D-II-1 | Co-Mn-Cu-Ag/Al$_2$O$_3$-SiO$_2$ | 73.4 | 36.4 | 23.1 | 32.4 | 8.1 |
| D-II-2 | Co-Ag/Al$_2$O$_3$-SiO$_2$ | 68.5 | 34.5 | 24.5 | 30.6 | 10.4 |

As can be seen from the data in Table II-2, the catalyst of the present application shows a higher conversion and a higher selectivity of hexanediamine, which indicates that the catalytic activity was improved compared with the catalyst falling out of the present application, and a lower selectivity to "others", i.e. amines with 12 or more carbon atoms, which indicates that the catalyst of the present application has a lower carbon deposition and a higher stability.

After a long-period stability test of 500 h conducted respectively on the catalysts A-II-3 and D-II-2, BET and XRD tests were respectively carried out on the catalysts before and after use, of which the results are shown in Table II-3. The results show that the specific surface area, the pore volume and the grain size of the active metal of the catalyst A-II-3 are substantially unchanged, while the specific surface area and the pore volume of the catalyst D-II-2 are reduced to a certain extent, and the grain size of the active metal is obviously increased compared with the catalysts before reaction, which indicates that the catalyst of the present application has better stability and less carbon deposition.

Table II-3 Change in properties of the catalysts before and after reaction

| Catalyst | Specific surface area, $m^2/g$ | Pore volume, ml/g | Grain size of active metal component, nm |
|---|---|---|---|
| A-II-3, before reaction | 128 | 0.63 | 7.4 |
| A-II-3, after reaction | 125 | 0.62 | 7.8 |
| D-II-2, before reaction | 129 | 0.65 | 8.9 |
| D-II-2, after reaction | 110 | 0.53 | 16.7 |

Test Example II-3

100 mL of the catalyst A-II-3 obtained in Example II-3 was measured out, loaded into a fixed bed reactor, activated with hydrogen at 240 °C for 2 hours, then cooled to 168 °C, the pressure of the system was raised to 1.8 MPa using hydrogen, then ammonia was metered into the reaction system via a metering pump, preheated to 160 °C and then sent to the upper end of the reactor, ethanol was fed into the upper end of the reactor via a metering pump, hydrogen was stably introduced via a gas mass flowmeter, the molar ratio of hydrogen to ammonia and to ethanol was 3 : 5 : 1, the liquid phase volume space velocity of ethanol was 0.6 $h^{-1}$, a catalytic amination reaction was conducted in the reactor at a reaction temperature of 185 °C and a reaction pressure of 2.8 MPa, and after the reaction became stable, the reaction solution was sampled and analyzed. The analysis results are shown in Table II-4.

Table II-4 Results of Test Example II-3

| Continuous reaction time | Conversion of ethanol/% | Selectivity/%) | | | |
|---|---|---|---|---|---|
| | | Monoethylamine | Diethylamine | Triethylamine | Others |
| 500h | 98.4 | 25.9 | 50.9 | 22.9 | 0.3 |
| 1000h | 98.5 | 26.1 | 50.8 | 22.8 | 0.3 |

Test Example II-4

100 mL of the catalyst A-II-5 obtained in Example II-5 was measured out, loaded into a fixed bed reactor, activated with hydrogen at 240 °C for 2 hours, then cooled to 168 °C, the pressure of the system was raised to 8 MPa using hydrogen, then ammonia was metered into the reaction system via a metering pump, preheated to 150 °C and sent to the upper end of the reactor, ethanolamine was fed into the upper end of the reactor via a metering pump, hydrogen was stably introduced via a gas mass flowmeter, wherein the molar ratio of hydrogen to ammonia and to ethanolamine was 3 : 8 : 1, the liquid phase volume space velocity of ethanolamine was 0.6 $h^{-1}$, a catalytic amination reaction was conducted in the reactor at a reaction temperature of 195 °C and a reaction pressure of 9.5 MPa, and after the reaction became stable, the reaction solution was sampled and analyzed (the analysis conditions, and the methods for calculating the conversion and the selectivity are similar to those of Test Example II-2). The analysis results are shown in Table II-5.

Table II-5 Results of Test Example II-4

| Continuous reaction time | Conversion of ethanolamine | Selectivity/% | | | | | | |
|---|---|---|---|---|---|---|---|---|
| | | Ethylenediamine | Piperazine | Diethylenetriamine | Hydroxyethyl ethylenediamine | N-aminoethyl piperazine | N-hydroxyethyl piperazine | Others |
| 500h | 91.5 | 58.4 | 25.2 | 7.99 | 7.5 | 0.5 | 0.3 | 0.1 |
| 1000h | 91.4 | 58.3 | 25.3 | 8.02 | 7.56 | 0.42 | 0.3 | 0.1 |

Example series III

The Third type of embodiments of the present application are further illustrated in detail with reference to the examples of Examples series III. In the following examples of Example series III, pseudo-boehmite powder was produced by aluminum sulfate method, and had a ($Al_2O_3$) content on a dry basis of 72 wt%; the silica sol was purchased from Qingdao Ocean Chemical Co., Ltd, under a trade name of JN-40.

Example III-1

Pseudo-boehmite powder (with a specific surface area of 375 $m^2$/g, a pore volume of 0.99 ml/g, a boron content of 3.89 g, per 100g of $Al_2O_3$) was kneaded with a dilute acid aqueous solution (containing calcium nitrate in a metered amount, with a calcium content of 0.11 g, per 100g of $Al_2O_3$), and extruded into strips with a diameter of 5 mm, dried at 120 °C for 12 hours, and then calcined at 800 °C for 10 hours to obtain a carrier. The parameters of the carrier are shown in Table III-1.

176.4 g of cobalt nitrate hexahydrate (industrial grade, with a purity of 98%), 26.1g of a 50wt% aqueous manganese nitrate solution and 6.83g of zinc nitrate were dissolved in water to obtain a 138 mL solution, and the solution was loaded onto 100 g of the carrier obtained by spray impregnation in two times, dried at 120 °C for 4 hours after each time of spray impregnation, and then calcined at 400 °C for 4 hours, and then reduced with hydrogen while gradually increasing the temperature at a rate of 20 °C/h, and finally reduced for 3 h at 430 °C to obtain a catalyst A-III-1.

Example III-2

Pseudo-boehmite powder (with a specific surface area of 405 $m^2$/g, a pore volume of 1.06 ml/g, a sulfur content of 0.79 g, per 100g of $Al_2O_3$) was kneaded with a dilute acid aqueous solution containing 5 vol% of nitric acid (containing magnesium nitrate in a metered amount, with a magnesium content of 0.78 g, per 100g of $Al_2O_3$), extruded into granules of clover shape with a thickness of 3 mm, dried at 120 °C for 15 hours, and then calcined at 840 °C for 4 hours to obtain a carrier. The parameters of the carrier are shown in Table III-1.

161.8 g of nickel nitrate hexahydrate (industrial grade, with a purity of 98%), 39.1g of a 50wt% aqueous manganese nitrate solution and 6.83g of zinc nitrate were dissolved in water to obtain a 146 mL solution, and the solution was loaded onto 100 g of the carrier obtained by spray impregnation in two times, dried at 120 °C for 5 hours after each time of spray impregnation, and then calcined at 390 °C for 4 hours, and then reduced with hydrogen while gradually increasing the temperature at a rate of 20 °C/h, and finally reduced at 440 °C for 3 h to obtain a catalyst A-III-2.

Example III-3

Pseudo-boehmite powder (with a specific surface area of 386 $m^2$/g, a pore volume of 1.09 ml/g, a sulfur content of 0.84 g, per 100g of $Al_2O_3$) and silica sol were mixed uniformly, kneaded with a dilute acid aqueous solution containing 5 vol% of nitric acid (containing potassium nitrate in a metered amount, with a potassium content of 2.1 g, per 100g of $Al_2O_3$), extruded into dentate spheres with a diameter of 4 mm, dried at 100 °C for 20 hours, and then calcined at 850 °C for 6 hours to obtain a carrier. The parameters of the carrier are shown in Table III-1.

50.4 g of cobalt nitrate hexahydrate (industrial grade, with a purity of 98%), 13.0 g of a 50 wt% aqueous manganese nitrate solution and 6.83g of a zinc nitrate solution were dissolved in water to obtain a 104mL solution, and the solution was loaded onto 100 g of the carrier obtained by spray impregnation in two times, dried at 120 °C for 4 hours after each time of spray impregnation, then calcined at 400 °C for 4 hours, and then reduced with hydrogen while gradually increasing the temperature at a rate of 20 °C/h, and finally reduced for 3 h at 430 °C to obtain a catalyst A-III-3.

Example III-4

Pseudo-boehmite powder (with a specific surface area of 398 $m^2$/g, a pore volume of 0.99 ml/g, a fluorine content of 0.85 g, per 100g of $Al_2O_3$) and ZSM-5 (commercially available, produced by Nankai University, $SiO_2/Al_2O_3$=45 (molar ratio)) were mixed uniformly, kneaded with a dilute acid aqueous solution containing 5 vol% of nitric acid (containing bismuth nitrate in a metered amount, with a bismuth content of 1.8 g per 100g of the pseudo-boehmite powder used calculated as $Al_2O_3$), extruded into dentate spheres with a diameter of 4 mm, dried at 140 °C for 10 h, and then calcined at 750 °C for 10 h to obtain a carrier. The parameters of the carrier are shown in Table III-1.

100.8 g of cobalt nitrate hexahydrate (industrial grade, with a purity of 98%), 3.3 g of a 50 wt% aqueous manganese nitrate solution and 4.55g of a zinc nitrate solution were dissolved in water to obtain a 188mL solution, and the solution was loaded onto 100 g of the carrier obtained by spray impregnation in two times, dried at 120 °C for 4 hours after each time of spray impregnation, then calcined at 400 °C for 4 hours, and then reduced with hydrogen while gradually increasing the temperature at a rate of 20 °C/h, and finally reduced for 3 h at 430 °C to obtain a catalyst A-III-4.

Example III-5

Pseudo-boehmite powder (with a specific surface area of 375 m$^2$/g, a pore volume of 1.15 ml/g, a phosphorus content of 0.33 g, per 100g of Al$_2$O$_3$) was kneaded with a dilute acid aqueous solution containing 5 vol% of nitric acid (containing barium nitrate in a measured amount, with a barium content of 1.7 g, per 100g of Al$_2$O$_3$), extruded into dentate spheres with a diameter of 4 mm, dried at 150 °C for 6 hours, and then calcined at 950 °C for 3 hours to obtain a carrier. The parameters of the carrier are shown in Table III-1.

75.6 g of cobalt nitrate hexahydrate (industrial grade, with a purity of 98%) and 4.55g of a zinc nitrate solution were dissolved in water to obtain a 104mL solution, and the solution was loaded onto 100 g of the carrier obtained by spray impregnation in two times, dried at 120 °C for 4 hours after each time of spray impregnation, then calcined at 380 °C for 4 hours. 2.9g of ammonium perrhenate were dissolved in water to obtain a 45mL solution, the solution was loaded onto the intermediate product obtained above by spray impregnation, dried at 120 °C for 4 hours, and then calcined at 380 °C for 4 hours, and then reduced with hydrogen while gradually increasing the temperature at a rate of 30 °C/h, and finally reduced at 450 °C for 4 h to obtain a catalyst A-III-5.

Example III-6

Pseudo-boehmite powder (with a specific surface area of 320 m$^2$/g, a pore volume of 1.03 ml/g, a boron content of 0.24 g, per 100g of Al$_2$O$_3$) and silica sol were mixed uniformly, kneaded with a dilute acid aqueous solution containing 5 vol% of nitric acid (containing calcium nitrate in a metered amount, with a calcium content of 1.20 g, per 100g of Al$_2$O$_3$), extruded into strips with a diameter of 5 mm, cut to a length of 4 mm, dried at 110 °C for 16 h, and then calcined at 880 °C for 5h to obtain a carrier. The parameters of the carrier are shown in Table III-1.

126.4 g of nickel nitrate hexahydrate (industrial grade, with a purity of 98%) and 22.75g of zinc nitrate were dissolved in water to obtain a 150mL solution, and the solution was loaded onto 100 g of the carrier obtained by spray impregnation in two times, dried at 120 °C for 8 hours and then calcined at 360 °C for 6 hours after each time of spray impregnation. 4.30g of ammonium perrhenate was dissolved in water to obtain a 67mL solution, the solution was loaded onto the intermediate product obtained above by spray impregnation, dried at 120 °C for 4 hours and then calcined at 400 °C for 4 hours, and then reduced with hydrogen while gradually increasing the temperature at a rate of 20 °C/h, and finally reduced for 3 h at 430 °C to obtain a catalyst A-III-6.

Example III-7

Pseudo-boehmite powder (with a specific surface area of 355 m$^2$/g, a pore volume of 0.99 ml/g, a sulfur content of 2.65 g, per 100g of Al$_2$O$_3$) and silica sol were mixed uniformly, kneaded with a dilute acid aqueous solution containing 5 vol% of nitric acid (containing magnesium nitrate in a measured amount, with a magnesium content of 1.4 g, per 100g of Al$_2$O$_3$), extruded into strips with a diameter of 5 mm, dried at 120 °C for 15 hours, and then calcined at 550 °C for 20 hours to obtain a carrier. The parameters of the carrier are shown in Table III-1.

201.6 g of cobalt nitrate hexahydrate (industrial grade, with a purity of 98%) and 2.28g of zinc nitrate were dissolved in water to obtain a 136 mL solution, and the solution was loaded onto 100 g of the carrier obtained by spray impregnation in two times, dried at 120 °C for 4 hours and then calcined at 400 °C for 4 hours after each time of spray impregnation. 1.40g of ammonium perrhenate was dissolved in water to obtain a 62mL solution, the solution was loaded onto the intermediate product obtained above by spray impregnation, dried at 120 °C for 4 hours, and then calcined at 400 °C for 4 hours, and then reduced with hydrogen while gradually increasing the temperature at a rate of 20 °C/h, and finally reduced at 480 °C for 2 h to obtain a catalyst A-III-7.

Example III-8

Pseudo-boehmite powder (with a specific surface area of 275 m$^2$/g, a pore volume of 0.99 ml/g, a fluorine content of 0.08 g, per 100g of Al$_2$O$_3$) was kneaded with a dilute acid aqueous solution containing 5 vol% of nitric acid (containing calcium nitrate in a metered amount, with a calcium content f 0.80 g, per 100g of Al$_2$O$_3$), extruded into strips with a diameter of 5 mm, dried at 150 °C for 8 hours, and then calcined at 800 °C for 8 hours to obtain a carrier. The parameters of the carrier are shown in Table III-1.

100.8 g of cobalt nitrate hexahydrate (industrial grade, with a purity of 98%) and 13.65g of zinc nitrate were dissolved in water to obtain a 122mL solution, and the solution was loaded onto 100 g of the carrier obtained by spray impregnation in two times, dried at 120 °C for 4 hours and then calcined at 400 °C for 4 hours after each time of spray impregnation. 1.40g of ammonium perrhenate was dissolved in water to obtain a 55 mL solution, the solution was loaded onto the intermediate product obtained above by spray impregnation, dried at 120 °C for 4 hours and then calcined at 400 °C for 4 hours, and then reduced with hydrogen while gradually increasing the temperature at a rate of 20 °C/h, and finally reduced at 450 °C for 3 h to obtain a catalyst A-III-8.

Example III-9

Pseudo-boehmite powder (with a specific surface area of 379 $m^2$/g, a pore volume of 0.99 ml/g, a phosphorus content of 0.2 g, per 100g of $Al_2O_3$) was kneaded with a dilute acid aqueous solution containing 5 vol% of nitric acid (containing calcium nitrate in a metered amount, with a calcium content of 1.30 g, per 100g of $Al_2O_3$), extruded into cylindrical bars with a diameter of 5 mm, dried at 100 °C for 18 hours, and then calcined at 880 °C for 4 hours to obtain a carrier. The parameters of the carrier are shown in Table III-1.

176.4 g of cobalt nitrate hexahydrate (industrial grade, with a purity of 98%), 19.5g of a 50wt% aqueous manganese nitrate solution and 19.36g of zinc nitrate were dissolved in water to obtain a 152mL solution, and the solution was loaded onto 100 g of the carrier obtained by spray impregnation in two times, dried at 120 °C for 4 hours after each time of spray impregnation, and then calcined at 380 °C for 4 hours. 1.44g of ammonium perrhenate was dissolved in water to obtain a 70mL solution, the solution was loaded onto the intermediate product obtained above by spray impregnation, dried at 120 °C for 4 hours and then calcined at 420 °C for 3 hours, and then reduced with hydrogen while gradually increasing the temperature at a rate of 20 °C/h, and finally reduced for 6 h at 420 °C to obtain a catalyst A-III-9.

Example III-10

Pseudo-boehmite powder (with a specific surface area of 392 $m^2$/g, a pore volume of 0.99 ml/g, a sulfur content of 0.97 g, per 100g of $Al_2O_3$) was kneaded with a dilute acid aqueous solution containing 5 vol% of nitric acid (containing calcium nitrate in a metered amount, with a calcium content of 0.05 g, per 100g of $Al_2O_3$), extruded into strips with a diameter of 5 mm, dried at 80 °C for 20 hours, and then calcined at 700 °C for 10 hours to obtain a carrier. The parameters of the carrier are shown in Table III-1.

226.8 g of cobalt nitrate hexahydrate (industrial grade, with a purity of 98%), 26.1g of 50wt% of manganese nitrate and 2.28g of zinc nitrate were dissolved in water to obtain a 162mL solution, and the solution was loaded onto 100 g of the carrier obtained by spray impregnation in two times, dried at 120 °C for 6 hours after each time of spray impregnation, and then calcined at 400 °C for 4 hours. 2.88g of ammonium perrhenate were dissolved in water to obtain a 75mL solution, the solution was loaded onto the intermediate product obtained above by spray impregnation, dried at 120 °C for 4 hours and then calcined at 400 °C for 4 hours, and then reduced with hydrogen while gradually increasing the temperature at a rate of 20 °C/h, and finally reduced for 4 h at 440 °C to obtain a catalyst A-III-10.

Example III-11

Pseudo-boehmite powder (with a specific surface area of 315 $m^2$/g, a pore volume of 0.99 ml/g, a phosphorus content of 4.64 g, per 100g of $Al_2O_3$) was kneaded with a dilute acid aqueous solution containing 5 vol% of nitric acid (containing calcium nitrate in a metered amount, with a calcium content of 1.25 g, per 100g of $Al_2O_3$), extruded into clovers with a diameter of 3mm, dried at 120 °C for 8 hours, and then calcined at 750 °C for 8 hours to obtain a carrier. The parameters of the carrier are shown in Table III-1.

141.1 g of cobalt nitrate hexahydrate (industrial grade, with a purity of 98%), 32.6g of a 50wt% aqueous manganese nitrate solution and 2.28g of zinc nitrate were dissolved in water to obtain a 154 mL solution, and the solution was loaded onto 100 g of the carrier obtained by spray impregnation in two times, dried at 120 °C for 4 hours after each time of spray impregnation, then calcined at 400 °C for 4 hours. 5.76g of ammonium perrhenate were dissolved in water to obtain a 71mL solution, the solution was loaded onto the intermediate product obtained above by spray impregnation, dried at 120 °C for 4 hours and then calcined at 390 °C for 6 hours, and then reduced with hydrogen while gradually increasing the temperature at a rate of 20 °C/h, and finally reduced at 450 °C for 3 h to obtain a catalyst A-III-11.

Example III-12

A catalyst A-III-12 was prepared as described in Example III-6, except that the pseudo-boehmite powder used was free of doping elements, and had a specific surface area of 366 $m^2$/g and a pore volume of 1.05 ml/g.

Comparative Example III-1

A catalyst was prepared as described in Example III-6, except that the pseudo-boehmite powder used was free of doping elements, and had a specific surface area of 325 $m^2$/g and a pore volume of 0.95 ml/g, and zinc nitrate was replaced with 15.2g of nickel nitrate hexahydrate (industrial grade, with a purity of 98%), obtaining a catalyst D-III-1.

Comparative Example III-2

A catalyst was prepared as described in Example III-9, except that the pseudo-boehmite powder used was free of doping elements, and had a specific surface area of 349 $m^2/g$ and a pore volume of 1.13 ml/g, and that manganese nitrate and ammonium perrhenate were replaced with 20.1g of cobalt nitrate hexahydrate (industrial grade, with a purity of 98%), obtaining a catalyst D-III-3.

Comparative Example III-3

A catalyst was prepared as described in Example III-2, except that a pseudo-boehmite powder (with a specific surface area of of 396 $m^2/g$, a pore volume of 1.21 ml/g, a sulfur content of 4.25 g, per 100g of $Al_2O_3$) was used, and zinc nitrate was replaced with 5.79g of iron nitrate nonahydrate, obtaining a catalyst D-III-3.

Test Example III-1

The elemental composition of the carriers and the catalysts was analyzed by plasma emission spectroscopy, wherein the contents of the doping element, the active metal component and the metal promoter are expressed by weight relative to 100g of the matrix; the carriers obtained above were characterized by $NH_3$-TPD, $CO_2$-TPD, BET nitrogen adsorption-desorption methods, and the results are shown in Table III-1.

Table III-1 Properties of the carriers of the examples and comparative examples

| Example No. | Metal active component and composition thereof, g | Metal promoter and composition thereof, g | Matrix and composition thereof (wt%) | Specific surface area of carrier, $m^2/g$ | Pore volume of carrier, ml/g | Ammonia adsorption capacity, mmol/g | $CO_2$ adsorption capacity, mmol/g | Proportion of pore volume of pores having a pore diameter of 7-27nm | Proportion of pore volume of pores having a pore diameter < 7nm | Doping element and content thereof |
|---|---|---|---|---|---|---|---|---|---|---|
| Ex. III-1 | Co 35 | Mn 4/Zn 1.5 | $Al_2O_3$ | 165 | 0.74 | 0.33 | 0.08 | 0.75 | 0.08 | B 3.89/Ca 0.11 |
| Ex. III-2 | Ni 32 | Mn 6/Zn 1.5 | $Al_2O_3$ | 163 | 0.78 | 0.34 | 0.11 | 0.72 | 0.05 | S 0.79/Mg 0.78 |
| Ex. III-3 | Co 10 | Mn 2/Zn 1.5 | $Al_2O_3$ 74/$SiO_2$ 26 | 149 | 0.57 | 0.52 | 0.28 | 0.71 | 0.07 | S 0.62/K 1.6 |
| Ex. III-4 | Co 20 | Mn 0.5/Zn 1 | $Al_2O_3$ 85/ZSM-5 15 | 181 | 0.99 | 0.37 | 0.22 | 0.76 | 0.05 | F 0.72/Bi 1.5 |
| Ex. III-5 | Co 15 | Re 2/Zn 1 | $Al_2O_3$ | 139 | 0.62 | 0.34 | 0.18 | 0.73 | 0.03 | P 0.33/Ba 1.7 |
| Ex. III-6 | Ni 25 | Re 3/Zn 5 | $Al_2O_3$ 80/$SiO_2$ 20 | 190 | 0.8 | 0.44 | 0.13 | 0.7 | 0.02 | B 0.19/Ca 0.96 |
| Ex. III-7 | Co 40 | Re 1/Zn 0.5 | $Al_2O_3$ 82/$SiO_2$ 18 | 175 | 0.73 | 0.47 | 0.16 | 0.72 | 0.08 | S 2.17/Mg 1.1 |
| Ex. III-8 | Co 20 | Re 1/Zn 3 | $Al_2O_3$ | 168 | 0.66 | 0.45 | 0.1 | 0.76 | 0.09 | F 0.08/Ca 0.8 |
| Ex. III-9 | Co 35 | Mn 3/Re 1/Zn 0.5 | $Al_2O_3$ | 191 | 0.81 | 0.52 | 0.14 | 0.74 | 0.01 | P 0.2/Ca 1.3 |
| Ex. III-10 | Co 45 | Mn 4/Re 2/Ag 0.5 | $Al_2O_3$ | 182 | 0.86 | 0.49 | 0.07 | 0.71 | 0.04 | S 0.97/Ca 0.05 |
| Ex. III-11 | Co 28 | Mn 5/Re 4/Ag 0.5 | $Al_2O_3$ | 185 | 0.82 | 0.44 | 0.23 | 0.73 | 0.03 | P 4.64/Ca 1.25 |
| Ex. III-12 | Ni 25 | Re 3/Zn 5 | $Al_2O_3$ 80/$SiO_2$ 20 | 182 | 0.79 | 0.28 | 0.12 | 0.77 | 0.04 | Ca 0.96 |
| Comp. Ex. III-1 | Ni 28 | Re 3 | $Al_2O_3$ 80/$SiO_2$ 20 | 170 | 0.61 | 0.23 | 0.20 | 0.72 | 0.05 | Ca 0.96 |

(continued)

| Example No. | Metal active component and composition thereof, g | Metal promoter and composition thereof, g | Matrix and composition thereof (wt%) | Specific surface area of carrier, $m^2/g$ | Pore volume of carrier, ml/g | Ammonia adsorption capacity, mmol/g | $CO_2$ adsorption capacity, mmol/g | Proportion of pore volume of pores having a pore diameter of 7-27nm | Proportion of pore volume of pores having a pore diameter < 7nm | Doping element and content thereof |
|---|---|---|---|---|---|---|---|---|---|---|
| Comp. Ex. III-2 | Co 39 | Zn 0.5 | $Al_2O_3$ | 183 | 0.75 | 0.19 | 0.26 | 0.78 | 0.03 | Ca 1.3 |
| Comp. Ex. III-3 | Ni 30 | Mn 5/Fe 0.8 | $Al_2O_3$ | 159 | 0.69 | 0.71 | 0.06 | 0.74 | 0.05 | S 4.25/Mg 0.78 |

Test Example III-2

This test example illustrates a process for producing 1,6-hexanediamine by hydroamination of 1,6-hexanediol using the catalyst of the third type of embodiments of the present application.

100 mL of the catalysts obtained in the examples and comparative examples were respectively measured out, loaded into a fixed bed reactor, activated with hydrogen at 220 °C for 2 hours, then cooled to 168 °C, the pressure of the system was raised to 12 MPa using hydrogen, then ammonia was metered into the reaction system via a metering pump, preheated to 160 °C and sent to the upper end of the reactor, then heated and melted 1,6-hexanediol was fed into the upper end of the reactor via a metering pump, hydrogen was stably introduced via a gas mass flowmeter, wherein the molar ratio of hydrogen to ammonia and to 1,6-hexanediol was 3 : 8 : 1, and the liquid phase volume space velocity of 1,6-hexanediol was 0.6 h$^{-1}$, a catalytic amination reaction was conducted in the reactor at a reaction temperature of 205 °C and a reaction pressure of 13 MPa, and after the reaction became stable (namely after 10 h of reaction), the reaction solution was sampled and analyzed. The analysis results are shown in Table III-2.

The analysis of the sample was conducted by gas chromatography, and was calibrated using a correction factor of a standard sample formulated.

The conversion and selectivity were calculated based on the molar content of each component in the reaction solution, and the calculation methods were the same as described in Test Example I-2.

Table III-2 Test results for the catalysts of the examples and comparative examples

| Catalyst | Composition of catalyst | Conversion , % | Selectively, % | | | |
|---|---|---|---|---|---|---|
| | | | Hexanediamine | Hexamethyleneimine | Aminohexanol | Others |
| A-III-1 | Co-Mn-Zn/$Al_2O_3$ | 88.4 | 48.9 | 21.6 | 26.7 | 2.8 |
| A-III-2 | Ni-Mn-Zn/$Al_2O_3$ | 91.2 | 50.1 | 20.9 | 26.1 | 2.9 |
| A-III-3 | Co-Mn-Zn/$Al_2O_3$-$SiO_2$ | 88.9 | 42.3 | 19.8 | 35.5 | 2.4 |
| A-III-4 | Co-Mn-Zn/$Al_2O_3$-ZSM-5 | 91.4 | 48.5 | 20.7 | 27.7 | 3.1 |
| A-III-5 | Co-Re-Zn/ $Al_2O_3$ | 87.9 | 46.5 | 21.2 | 28.9 | 3.4 |
| A-III-6 | Ni-Re-Zn/$Al_2O_3$-$SiO_2$ | 88.1 | 47.8 | 19.7 | 29.6 | 2.9 |
| A-III-7 | Co-Re-Zn/$Al_2O_3$-$SiO_2$ | 91.8 | 51.8 | 22.7 | 22.4 | 3.1 |
| A-III-8 | Co-Re-Zn/$Al_2O_3$ | 86.5 | 48.5 | 21.3 | 27.3 | 2.9 |
| A-III-9 | Co-Mn-Re-Zn/$Al_2O_3$ | 87.8 | 48.6 | 21.9 | 26.9 | 2.6 |
| A-III-10 | Co-Mn-Re-Zn/$Al_2O_3$ | 84.9 | 45.8 | 20.1 | 31.4 | 2.7 |
| A-III-11 | Co-Mn-Re-Zn/$Al_2O_3$ | 85.9 | 46.2 | 22.3 | 28.9 | 2.6 |
| A-III-12 | Co-Mn-Zn/$Al_2O_3$-$SiO_2$ | 74.2 | 36.3 | 20.9 | 34.6 | 8.2 |
| D-III-1 | Ni-Re/$Al_2O_3$-$SiO_2$ | 65.2 | 32.3 | 26.8 | 31.2 | 9.7 |
| D-III-2 | Co-Zn/$Al_2O_3$ | 64.1 | 36.1 | 28.2 | 25.4 | 10.3 |
| D-III-3 | Ni-Mn-Fe/$Al_2O_3$ | 71.6 | 35.7 | 30.6 | 24.3 | 9.4 |

The catalysts were subjected to long-period stability test for 500 h under the same conditions, and BET and XRD tests were carried out on the catalysts before and after use. The results show that the specific surface areas and the pore volumes of the catalysts A-III-1 to A-III-11 are substantially unchanged (i.e. the reduction was not more than 2%) before and after use, while the specific surface areas and the pore volumes of the catalysts D-III-1 to D-III-3 are reduced significantly (both are more than 8%) after use, which indicates that the catalyst of the present application has better stability and less carbon deposition.

Further detection revealed that the catalyst A-III-2 still shows a reduction of not more than 5% in conversion after 1000 h of use, and the reduction value of the specific surface area and pore volume is not more than 5%. It can be seen that the catalyst of the present application has a longer life.

Test Example III-3

This test example illustrates a process for producing n-propylamine by hydroamination of n-propanol using the catalyst of the third type of embodiments of the present application.

100 mL of the catalyst A-III-2 obtained in Example III-3 was measured out, loaded into a fixed bed reactor, activated with hydrogen at 220 °C for 2 hours, then cooled to 172 °C, the pressure of the system was raised to 1.5 MPa using hydrogen, then ammonia was metered into the reaction system via a metering pump, preheated to 150 °C and then sent to the upper end of the reactor, n-propanol was fed into the upper end of the reactor via a metering pump, hydrogen was stably introduced via a gas mass flowmeter, the molar ratio of hydrogen to ammonia and to n-propanol was 3 : 5 : 1, the liquid phase volume space velocity of propanol was 0.75 h$^{-1}$, a catalytic amination reaction was conducted in the reactor at a reaction temperature of 180 °C and a reaction pressure of 2 MPa, and after the reaction became stable, the reaction solution was sampled and analyzed (the analysis conditions, and the methods for calculating the conversion and the selectivity are similar to those of Test Example III-2). The analysis results are shown in Table III-3.

Table III-3 Results of Test Example III-3

| Continuous reaction time | Conversion of n-propanol, % | Selectively, % | | | |
| --- | --- | --- | --- | --- | --- |
| | | N-propylamine | Di-n-propylamine | Tri-n-propylamine | Others |
| 200h | 99.0 | 27.5 | 48.6 | 23.5 | 0.4 |
| 500h | 99.1 | 26.9 | 48.3 | 24.4 | 0.4 |

Example series IV

The Fourth type of embodiments of the present application is further illustrated hereinbelow in detail with reference to the examples of Example series IV. In the following examples of Example series IV, the pseudo-boehmite powder used had a (Al$_2$O$_3$) content on a dry basis of 72 wt%; the silica sol was purchased from Qingdao Ocean Chemical Co., Ltd, under a trade name of JN-40.

Example IV-1

Pseudo-boehmite powder prepared by aluminum sulfate method (with a specific surface area of 380 m$^2$/g, and a pore volume of 1.02 ml/g, the pseudo-boehmite powder containing a doping element of sulfur, with a S content of 2.15g, per 100g of the pseudo-boehmite powder calculated as Al$_2$O$_3$; in the preparation of the pseudo-boehmite powder, water glass (i.e. aqueous sodium silicate solution) used as SiO$_2$ precursor was added at the beginning, so that the mass of SiO$_2$ derived from the SiO$_2$ precursor in the calcined carrier accounted for 4% of the total mass of the carrier) was kneaded with a dilute acid aqueous solution containing 5 vol% of nitric acid, extruded into strips with a diameter of 5 mm, cut to a length of 4 mm, dried at 120 °C for 8h, and calcined at 650 °C for 5 h to obtain a desired carrier.

186.5 g of cobalt nitrate hexahydrate (industrial grade, with a purity of 98%), 6.83g of zinc nitrate hexahydrate (analytically pure) and 5.65g of copper nitrate trihydrate (analytically pure) were dissolved in water to obtain a 148 mL solution, and the solution was loaded onto 100 g of the carrier obtained by spray impregnation in two times, dried at 120 °C for 4 hours after each time of spray impregnation and then calcined at 400 °C for 4 hours. 1.8g of ammonium molybdate tetrahydrate (analytically pure) was then dissolved in water to obtain a 74mL solution, the solution was loaded onto the intermediate product obtained above by spray impregnation, dried at 120 °C for 4 hours, then calcined at 400 °C for 4 hours, and then reduced with hydrogen while gradually increasing the temperature at a rate of 20 °C/h, and finally reduced for 3 h at 430 °C to obtain a catalyst A-IV-1.

Example IV-2

Pseudo-boehmite powder prepared by aluminum sulfate method (with a specific surface area of 375 m$^2$/g, and a pore volume of 0.98 ml/g, the pseudo-boehmite powder containing a doping element of boron, with a B content of 0.53g, per 100g of the pseudo-boehmite powder calculated as $Al_2O_3$; in the preparation of the pseudo-boehmite powder, water glass (i.e. aqueous sodium silicate solution) used as $SiO_2$ precursor was added at the beginning, so that the mass of $SiO_2$ derived from the $SiO_2$ precursor in the calcined carrier accounted for 11% of the total mass of the carrier) was kneaded with a dilute acid aqueous solution containing 5 vol% of nitric acid, extruded into granules of clover shape with a thickness of 3 mm, dried at 100 °C for 12 h, and calcined at 590 °C for 8h to obtain a desired carrier.

151.7 g of nickel nitrate hexahydrate (industrial grade, with a purity of 98%), 6.83g of zinc nitrate hexahydrate (analytically pure) and 5.65g of copper nitrate trihydrate (analytically pure) were dissolved in water to obtain a 156 mL solution, and the solution was loaded onto 100 g of the carrier obtained by spray impregnation in two times, dried at 120 °C for 4 hours after each time of spray impregnation, and then calcined at 400 °C for 4 hours. Then, 3.7g of ammonium molybdate tetrahydrate was dissolved in water to obtain a 78mL solution, the solution was loaded onto the intermediate product obtained above by spray impregnation, dried at 120 °C for 4 hours, then calcined at 400 °C for 4 hours, and then reduced with hydrogen while gradually increasing the temperature at a rate of 20 °C/h, and finally reduced for 3 h at 430 °C to obtain a catalyst A-IV-2.

Example IV-3

Pseudo-boehmite powder prepared by aluminum sulfate method (with a specific surface area of 380 m$^2$/g, and a pore volume of 1.02 ml/g, the pseudo-boehmite powder containing a doping element of sulfur, with a S content of 2.15g, per 100g of the pseudo-boehmite powder calculated as $Al_2O_3$; in the preparation of the pseudo-boehmite powder, water glass (i.e. aqueous sodium silicate solution) used as $SiO_2$ precursor was added at the beginning, so that the mass of $SiO_2$ derived from the $SiO_2$ precursor in the calcined carrier accounted for 4% of the total mass of the carrier) was kneaded with a dilute acid aqueous solution containing 5 vol% of nitric acid, extruded into strips with a diameter of 5 mm, cut to a length of 4 mm, dried at 120 °C for 8h, and calcined at 650 °C for 5 h to obtain a desired carrier.

55.4 g of cobalt nitrate hexahydrate (industrial grade, with a purity of 98%), 6.83g of zinc nitrate hexahydrate (analytically pure) and 5.65g of copper nitrate trihydrate (analytically pure) were dissolved in water to obtain a 144 mL solution, and the solution was loaded onto 100 g of the carrier obtained by spray impregnation in two times, dried at 120 °C for 4 hours after each time of spray impregnation, and then calcined at 400 °C for 4 hours. 1.8g of ammonium molybdate tetrahydrate was dissolved in water to obtain a 72mL solution, the solution was loaded onto the intermediate product obtained above by spray impregnation, dried at 120 °C for 4 hours, then calcined at 400 °C for 4 hours, and then reduced with hydrogen while gradually increasing the temperature at a rate of 20 °C/h, and finally reduced for 3 h at 430 °C to obtain a catalyst A-IV-3.

Example IV-4

Pseudo-boehmite powder prepared by aluminum sulfate method (with a specific surface area of 340 m$^2$/g, and a pore volume of 1.13 ml/g, the pseudo-boehmite powder containing a doping element of phosphorus, with a P content of 0.18g, per 100g of the pseudo-boehmite powder calculated as $Al_2O_3$; in the preparation of the pseudo-boehmite powder, ZSM-5 molecular sieve precursor (ZSM-5 powder, available form Catalyst Plant of Nankai University, $SiO_2/Al_2O_3$=45 (molar ratio), the same below) was added at the beginning, so that the mass of $Al_2O_3$ derived from the pseudo-boehmite powder in the calcined carrier accounted for 85% of the total mass of the carrier) was kneaded with a dilute acid aqueous solution containing 5 vol% of nitric acid, extruded into dentate spheres with a diameter of 4mm, dried at 80 °C for 20 h, and then calcined at 530 °C for 6 h, to obtain a desired carrier.

126 g of cobalt nitrate hexahydrate (industrial grade, with a purity of 98%), 4.55g of zinc nitrate and 0.79g of silver nitrate (analytically pure) were dissolved in water to obtain a 210 mL solution, and the solution was loaded onto 100 g of the carrier obtained by spray impregnation in two times, dried at 120 °C for 4 hours after each time of spray impregnation, and then calcined at 400 °C for 4 hours. 3.7g of ammonium molybdate tetrahydrate (analytically pure) were then dissolved in water to obtain a 105 mL solution, the solution was loaded onto the intermediate product obtained above by spray impregnation, dried at 120 °C for 4 hours, then calcined at 400 °C for 4 hours, and then reduced with hydrogen while gradually increasing the temperature at a rate of 20 °C/h, and finally reduced for 3 h at 430 °C to obtain a catalyst A-IV-4.

Example IV-5

The carrier was prepared as described in Example IV-4, except that ZSM-5 molecular sieve precursor was added at the beginning in the production of the pseudo-boehmite powder, so that the $Al_2O_3$ derived from the pseudo-boehmite powder in the calcined carrier accounted for 85% of the total mass of the carrier.

126 g of cobalt nitrate hexahydrate (industrial grade, with a purity of 98%), 9.1g of zinc nitrate hexahydrate (analytically pure) and 1.57g of silver nitrate (analytically pure) were dissolved in water to obtain a 208 mL solution, and the solution was loaded onto 100 g of the carrier obtained by spray impregnation in two times, dried at 120 °C for 4 hours after each time of spray impregnation and then calcined at 400 °C for 4 hours. 7.4g of ammonium molybdate tetrahydrate (analytically pure) were then dissolved in water to obtain a 104mL solution, the solution was loaded onto the intermediate product obtained above by spray impregnation, dried at 120 °C for 4 hours, then calcined at 400 °C for 4 hours, and then reduced with hydrogen while gradually increasing the temperature at a rate of 20 °C/h, and finally reduced for 3 h at 430 °C to obtain a catalyst A-IV-5.

Example IV-6

Pseudo-boehmite powder prepared by aluminum sulfate method (with a specific surface area of 341 m$^2$/g, and a pore volume of 1.11 ml/g, the pseudo-boehmite powder containing a doping element of phosphorus, with a P content of 4.2g, per 100g of the pseudo-boehmite powder calculated as $Al_2O_3$; in the preparation of the pseudo-boehmite powder, ZSM-5 molecular sieve precursor was added at the beginning, so that the mass of $Al_2O_3$ derived from the pseudo-boehmite powder in the calcined carrier accounted for 85% of the total mass of the carrier) was kneaded with a dilute acid aqueous solution containing 5 vol% of nitric acid, extruded into dentate spheres with a diameter of 4mm, dried at 80 °C for 20 h, and then calcined at 530 °C for 6 h to obtain a desired carrier.

The remaining of the procedure was the same as described in Example IV-4, obtaining a catalyst A-IV-6.

Example IV-7

Pseudo-boehmite powder prepared by aluminum sulfate method (with a specific surface area of 288 m$^2$/g, and a pore volume of 0.93 ml/g, the pseudo-boehmite powder containing a doping element of sulfur, with a S content of 0.88g, per 100g of the pseudo-boehmite powder calculated as $Al_2O_3$; in the preparation of the pseudo-boehmite powder, water glass (i.e. aqueous sodium silicate solution) used as $SiO_2$ precursor was added at the beginning, so that the mass of $SiO_2$ derived from the $SiO_2$ precursor in the calcined carrier accounted for 8% of the total mass of the carrier) was kneaded with a dilute acid aqueous solution containing 5 vol% of nitric acid, extruded into clovers with a diameter of 4mm, dried at 100 °C for 8 h, and then calcined for 4 h at 850 °C to obtain a desired carrier.

201.6 g of cobalt nitrate hexahydrate (industrial grade, with a purity of 98%), 2.28g of zinc nitrate hexahydrate (analytically pure) and 8.48g of copper nitrate trihydrate (analytically pure) were dissolved in water to obtain a 146 mL solution, and the solution was loaded onto 100 g of the carrier obtained by spray impregnation in two times, dried at 120 °C for 4 hours after each time of spray impregnation and then calcined at 400 °C for 4 hours. Then, 2.7g of ammonium metatungstate (analytically pure) was dissolved in water to obtain a 73mL solution, the solution was loaded onto the intermediate product obtained above by spray impregnation, dried at 120 °C for 4 hours, then calcined at 400 °C for 4 hours, and then reduced with hydrogen while gradually increasing the temperature at a rate of 20 °C/h, and finally reduced for 3 h at 430 °C to obtain a catalyst A-IV-7.

Example IV-8

The carrier obtained in Example IV-7 was used.

100.8 g of cobalt nitrate hexahydrate (industrial grade, with a purity of 98%), 2.28g of zinc nitrate hexahydrate (analytically pure) and 8.48g of copper nitrate trihydrate (analytically pure) were dissolved in water to obtain a 150 mL solution, and the solution was loaded onto 100 g of the carrier obtained by spray impregnation in two times, dried at 120 °C for 4 hours after each time of spray impregnation, and then calcined at 400 °C for 4 hours. 2.7g of ammonium metatungstate (analytically pure) were then dissolved in water to obtain a 75mL solution, the solution was loaded onto the intermediate product obtained above by spray impregnation, dried at 120 °C for 4 hours, then calcined at 400 °C for 4 hours, and then reduced with hydrogen while gradually increasing the temperature at a rate of 20 °C/h, and finally reduced for 3 h at 430 °C to obtain a catalyst A-IV-8.

Example IV-9

Pseudo-boehmite powder prepared by aluminum sulfate method (with a specific surface area of 281 m$^2$/g, and a pore volume of 0.87 ml/g, the pseudo-boehmite powder containing a doping element of fluorine, with a F content of 0.82g, per 100g of the pseudo-boehmite powder calculated as $Al_2O_3$; in the preparation of the pseudo-boehmite powder, water glass (i.e. aqueous sodium silicate solution) used as $SiO_2$ precursor was added at the beginning, so that the mass

of $SiO_2$ derived from the $SiO_2$ precursor in the calcined carrier accounted for 21% of the total mass of the carrier) was kneaded with a dilute acid aqueous solution containing 5 vol% of nitric acid, extruded into granules of clover shape with a thickness of 4 mm, dried at 90 °C for 18 h, and calcined at 770 °C for 9 h to obtain a desired carrier.

176.4 g of cobalt nitrate hexahydrate (industrial grade, with a purity of 98%), 2.28g of zinc nitrate hexahydrate (analytically pure) and 11.3g of copper nitrate trihydrate (analytically pure) were dissolved in water to obtain a 130mL solution, and the solution was loaded onto 100 g of the carrier obtained by spray impregnation in two times, dried at 120 °C for 4 hours after each time of spray impregnation and then calcined at 400 °C for 4 hours. 2.0g of ammonium metatungstate (analytically pure) was then dissolved in water to a solution of 65mL, the solution was loaded onto the intermediate product obtained above by spray impregnation, dried at 120 °C for 4 hours, then calcined at 400 °C for 4 hours, and then reduced with hydrogen while gradually increasing the temperature at a rate of 20 °C/h, and finally reduced for 3 h at 430 °C to obtain a catalyst A-IV-9.

Example IV-10

Pseudo-boehmite powder prepared by aluminum sulfate method (with a specific surface area of 274 $m^2$/g, and a pore volume of 0.85 ml/g, the pseudo-boehmite powder containing a doping element of sulfur, with a S content of 0.95g, per 100g of the pseudo-boehmite powder calculated as $Al_2O_3$; in the preparation of the pseudo-boehmite powder, water glass (i.e. aqueous sodium silicate solution) used as $SiO_2$ precursor was added at the beginning, so that the mass of $SiO_2$ derived from the $SiO_2$ precursor in the calcined carrier accounted for 25% of the total mass of the carrier) was kneaded with a dilute acid aqueous solution containing 5 vol% of nitric acid, extruded into granules of clover shape with a diameter of 3.5mm, dried at 150 °C for 6h, and then calcined for 6h at 930 °C to obtain a desired carrier.

227.5 g of nickel nitrate hexahydrate (industrial grade, with a purity of 98%), 2.28g of zinc nitrate hexahydrate (analytically pure) and 1.10g of silver nitrate (analytically pure) were dissolved in water to obtain a 156mL solution, and the solution was loaded onto 100 g of the carrier obtained by spray impregnation in 3 times, dried at 120 °C for 4 hours after each time of spray impregnation, then calcined at 400 °C for 4 hours. 5.4g of ammonium metatungstate (analytically pure) were then dissolved in 52mL of water, the solution was loaded onto the intermediate product obtained above by spray impregnation, dried at 120 °C for 4 hours, then calcined at 400 °C for 4 hours, and then reduced with hydrogen while gradually increasing the temperature at a rate of 20 °C/h, and finally reduced for 3 h at 430 °C to obtain a catalyst A-IV-10.

Example IV-11

Pseudo-boehmite powder prepared by aluminum sulfate method (with a specific surface area of 265 $m^2$/g, and a pore volume of 0.81 ml/g, the pseudo-boehmite powder containing a doping element of phosphorus, with a P content of 3.1g, per 100g of the pseudo-boehmite powder calculated as $Al_2O_3$; in the preparation of the pseudo-boehmite powder, water glass (i.e. aqueous sodium silicate solution) used as $SiO_2$ precursor was added at the beginning, so that the mass of $SiO_2$ derived from the $SiO_2$ precursor in the calcined carrier accounted for 28% of the total mass of the carrier) was kneaded with a dilute acid aqueous solution containing 5 vol% of nitric acid, extruded into granules of clover shape with a diameter of 3 mm, dried at 100 °C for 8 hours, and calcined at 1020 °C for 5 hours to obtain a desired carrier.

141.1 g of cobalt nitrate hexahydrate (industrial grade, with a purity of 98%), 2.28g of zinc nitrate hexahydrate (analytically pure) and 2.20g of silver nitrate (analytically pure) were dissolved in water to obtain a 144mL solution, and the solution was loaded onto 100 g of the carrier obtained by spray impregnation in 3 times, dried at 120 °C for 4 hours after each time of spray impregnation, and then calcined at 400 °C for 4 hours. 5.4g of ammonium metatungstate (analytically pure) were then dissolved in 48mL of water, the solution was loaded onto the intermediate product obtained above by spray impregnation, dried at 120 °C for 4 hours, then calcined at 400 °C for 4 hours, and then reduced with hydrogen while gradually increasing the temperature at a rate of 20 °C/h, and finally reduced for 3 h at 430 °C to obtain a catalyst A-IV-11.

Example IV-12

A catalyst was prepared as described in Example IV-1, except that the pseudo-boehmite powder used had a content of doping element P of 1.33 g, per 100g of the pseudo-boehmite powder calculated as $Al_2O_3$, a specific surface area of 375 $m^2$/g and a pore volume of 0.98 ml/g, to obtain a catalyst A-IV-12.

Comparative Example IV-1

A catalyst was prepared as described in Example IV-2, except that the pseudo-boehmite powder used was free of the doping element, and had a specific surface area of 391 $m^2$/g and a pore volume of 1.06 ml/g, and zinc nitrate hexahydrate was absent in the solution used in the spray impregnation, to obtain a catalyst D-IV-1.

Comparative Example IV-2

A catalyst was prepared as described in Example IV-8, except that the pseudo-boehmite powder used was free of the doping element, and had a specific surface area of 349 m$^2$/g and a pore volume of 1.17 ml/g, and copper nitrate trihydrate is absent in the solution used in the spray impregnation, to obtain a catalyst D-IV-2.

Comparative Example IV-3

A catalyst was prepared as described in Example IV-2, except that the pseudo-boehmite powder used was free of the doping element and had a specific surface area of 391 m$^2$/g and a pore volume of 1.06 ml/g, and that the zinc nitrate hexahydrate was replaced with 16.03 g of magnesium nitrate hexahydrate (analytically pure), to obtain a catalyst D-IV-3.

Comparative Example IV-4

A catalyst was prepared as described in Example IV-2, except that the pseudo-boehmite powder used was free of the doping element and had a specific surface area of 391 m$^2$/g and a pore volume of 1.06 ml/g, ammonium molybdate was not added, and zinc nitrate hexahydrate was replaced with 8.84 g of calcium nitrate tetrahydrate, to obtain a catalyst D-IV-4.

Test Example IV-1

The elemental composition of the carriers and the catalysts was analyzed by plasma emission spectroscopy, wherein the content of the doping element was expressed by weight relative to 100g of the matrix, and the contents of the active metal component and the metal promoter were both expressed by weight relative to 100g of the matrix; the carriers obtained above were characterized by NH$_3$-TPD, BET nitrogen adsorption-desorption methods, and the results are shown in Table IV-1.

Table IV-1 Properties of the carriers of the examples and comparative examples

| Example No. | Doping element | | Relative content, g | | Ammonia adsorption capacity, mmol/g | Proportion of pore volume of pores having a pore diameter < 7 nm, % | Proportion of pore volume of pores having a pore diameter of 7-27 nm, % | Specific surface area, $m^2/g$ | Pore volume, ml/g |
|---|---|---|---|---|---|---|---|---|---|
| | Species | Relative content, g | Active metal component | Metal promoter | | | | | |
| Ex. IV-1 | S | 2.02 | 37 | Mo 1/Cu 2/Zn 1.5 | 0.44 | 4 | 75 | 198 | 0.74 |
| Ex. IV-2 | B | 0.47 | 30 | Mo 2/Cu 2/Zn 1.5 | 0.34 | 4 | 72 | 191 | 0.78 |
| Ex. IV-3 | S | 2.02 | 11 | Mo 1/Cu 2/Zn 1.5 | 0.44 | 5 | 74 | 196 | 0.72 |
| Ex. IV-4 | P | 0.15 | 25 | Mo 2/Ag 0.5/Zn 1 | 0.32 | 3 | 76 | 181 | 1.05 |
| Ex. IV-5 | P | 0.15 | 25 | Mo 4/Ag 1/Zn 2 | 0.33 | 2 | 75 | 179 | 1.03 |
| Ex. IV-6 | P | 3.45 | 25 | Mo 2/Ag 0.5/Zn 1 | 0.52 | 3 | 75 | 177 | 1.02 |
| Ex. IV-7 | S | 0.80 | 40 | W 2/Cu 3/Zn 0.5 | 0.36 | 5 | 72 | 183 | 0.73 |
| Ex. IV-8 | S | 0.80 | 20 | W 2/Cu 3/Zn 0.5 | 0.37 | 4 | 72 | 185 | 0.75 |
| Ex. IV-9 | F | 0.64 | 35 | W 1.5/Cu 4/Zn 0.5 | 0.36 | 3 | 74 | 152 | 0.65 |
| Ex. IV-10 | S | 0.71 | 45 | W 4/Ag 0.7/Zn 0.5 | 0.41 | 2 | 71 | 135 | 0.52 |
| Ex. IV-11 | P | 2.18 | 28 | W 4/Ag 1.4/Zn 0.5 | 0.48 | 4 | 73 | 128 | 0.48 |
| Ex. IV-12 | P | 1.33 | 37 | Mo 1/Cu 2/Zn 1.5 | 0.29 | 6 | 71 | 165 | 0.69 |
| Comp. Ex. IV-1 | None | 0 | 30 | Mo 2/Cu 2 | 0.24 | 4 | 70 | 186 | 0.77 |

(continued)

| Example No. | Doping element | | Relative content, g | | Ammonia adsorption capacity, mmol/g | Proportion of pore volume of pores having a pore diameter < 7 nm, % | Proportion of pore volume of pores having a pore diameter of 7-27 nm, % | Specific surface area, m$^2$/g | Pore volume, ml/g |
|---|---|---|---|---|---|---|---|---|---|
| | Species | Relative content, g | Active metal component | Metal promoter | | | | | |
| Comp. Ex. IV-2 | 0 | 0 | 20 | W 2/Zn 0.5 | 0.16 | 4 | 71 | 189 | 0.76 |
| Comp. Ex. IV-3 | None | 0 | 30 | Mo 2/Cu 2/Mg 1.5 | 0.20 | 4 | 74 | 184 | 0.74 |
| Comp. Ex. IV-4 | None | 0 | 30 | Cu 2/Ca 1.5 | 0.22 | 5 | 70 | 185 | 0.79 |

Test Example IV-2

This test example illustrates a process for producing 1,6-hexanediamine by hydroamination of 1,6-hexanediol using the catalyst of the fourth type of embodiments of the present application.

100 mL of the catalysts obtained in the examples and comparative examples were respectively measured out, loaded into a fixed bed reactor, activated with hydrogen at 220 °C for 2 hours, then cooled to 165 °C, the pressure of the system was raised to 11 MPa using hydrogen, then ammonia was metered into the reaction system via a metering pump, preheated to 125 °C and sent to the upper end of the reactor, then heated and melted 1,6-hexanediol was fed into the upper end of the reactor via a metering pump, hydrogen was stably introduced via a gas mass flowmeter, wherein the molar ratio of hydrogen to ammonia and to 1,6-hexanediol was 2 : 12 : 1, the liquid phase volume space velocity of 1,6-hexanediol was 0.35 h$^{-1}$, a catalytic amination reaction was conducted in the reactor at a reaction temperature of 170 °C and a reaction pressure of 11 MPa, and after a reaction of 20 h, the reaction solution was sampled and analyzed. The analysis results are shown in Table IV-2.

The analysis of the sample was conducted by gas chromatography, and was calibrated using a correction factor of a standard sample formulated.

The conversion and selectivity were calculated based on the molar content of each component in the reaction solution, and the calculation methods were the same as described in Test Example I-2.

Table IV-2 Test results for the catalysts of the examples and comparative examples

| Catalyst | Composition of catalyst | Conversion, % | Selectively, % | | | |
|---|---|---|---|---|---|---|
| | | | Hexanediamine | Hexamethyleneimine | Aminohexanol | Others |
| A-IV-1 | Co-Mo-Cu-Zn/Al$_2$O$_3$-SiO$_2$ | 95 | 67.3 | 15.4 | 14.9 | 2.4 |
| A-IV-2 | Ni-Mo-Cu-Zn/Al$_2$O$_3$-SiO$_2$ | 95 | 67.9 | 14.3 | 15.3 | 2.5 |
| A-IV-3 | Co-Mo-Cu-Zn/Al$_2$O$_3$-SiO$_2$ | 90 | 64.7 | 13.9 | 18.2 | 3.2 |
| A-IV-4 | Co-Mo-Ag-Zn/Al$_2$O$_3$-ZSM-5 | 94 | 66.3 | 16.2 | 15.1 | 2.4 |
| A-IV-5 | Co-Mo-Ag-Zn/Al$_2$O$_3$-ZSM-5 | 91 | 63.1 | 15.8 | 18.1 | 3 |
| A-IV-6 | Co-Mo-Ag-Zn/Al$_2$O$_3$-ZSM-5 | 90 | 60.7 | 20.3 | 15.7 | 3.3 |
| A-IV-7 | Co-W-Cu-Zn/Al$_2$O$_3$-SiO$_2$ | 91 | 62.3 | 20.1 | 14.2 | 3.4 |
| A-IV-8 | Co-W-Cu-Zn/Al$_2$O$_3$-SiO$_2$ | 94 | 65.8 | 14.8 | 16.5 | 2.9 |
| A-IV-9 | Co-W-Cu-Zn/Al$_2$O$_3$-SiO$_2$ | 92 | 64.3 | 15.7 | 17.2 | 2.8 |
| A-IV-10 | Ni-W-Ag-Zn/Al$_2$O$_3$-SiO$_2$ | 89 | 62.1 | 23.8 | 10.6 | 3.5 |
| A-IV-11 | Co-W-Ag-Zn/Al$_2$O$_3$-SiO$_2$ | 91 | 62.7 | 17.3 | 17 | 3 |
| A-IV-12 | Co-Mo-Cu-Zn/Al$_2$O$_3$-SiO$_2$ | 85 | 56.3 | 20.2 | 19.2 | 4.3 |
| D-IV-1 | Ni-Mo-Cu/Al$_2$O$_3$-SiO$_2$ | 80 | 48.2 | 21.3 | 24.9 | 5.6 |
| D-IV-2 | Co-W-Zn/Al$_2$O$_3$-SiO$_2$ | 81 | 46.3 | 21.8 | 26.6 | 5.3 |
| D-IV-3 | Ni-Mo-Cu-Mg/Al$_2$O$_3$-SiO$_2$ | 75 | 42.3 | 21.6 | 29.9 | 6.2 |
| D-IV-4 | Ni-Cu-Ca/Al$_2$O$_3$-SiO$_2$ | 76 | 39.2 | 20.5 | 32.2 | 8.1 |

Test Example IV-3

This test example illustrates a process for producing n-propylamine by hydroamination of n-propanol using the catalyst of the fourth type of embodiments of the present application.

100 mL of the catalyst A-IV-3 obtained in Example IV-3 was measured out, loaded into a fixed bed reactor, and activated with hydrogen at 220 °C for 2 hours, then cooled to 140 °C, the pressure of the system was raised to 2 MPa using hydrogen, ammonia was metered into the reaction system via a metering pump, preheated to 110 °C and then sent to the upper end of the reactor, n-propanol was fed into the upper end of the reactor via a metering pump, hydrogen was stably introduced via a gas mass flowmeter, wherein the molar ratio of hydrogen to ammonia and to n-propanol was 3 : 6 : 1, the liquid phase volume space velocity of propanol was 0.5 h$^{-1}$, a catalytic amination reaction was conducted in the reactor, and after the reaction became stable, the reaction solution was sampled and analyzed (the analysis conditions, and the methods for calculating the conversion and the selectivity are similar to those of Test Example IV-2). The analysis results are shown in Table IV-3.

Table 3 Results of Test Example IV-3

| Continuous reaction time | Conversion of n-propanol, % | Selectively, % | | | |
|---|---|---|---|---|---|
| | | N-propylamine | Di-n-propylamine | Tri-n-propylamine | Others |
| 20h | 99.12 | 25.3 | 53.1 | 20.8 | 0.8 |
| 400h | 99.13 | 25.2 | 53.2 | 20.7 | 0.9 |

Test Example IV-4

The catalysts A-IV-1, A-IV-4, D-IV-1, D-IV-2, and D-IV-3 were loaded into fixed bed reactors, respectively, under the same conditions as in Test Example IV-2, with an only change that the reaction time was prolonged, and a test was conducted for 400 hours. An analysis and comparison were conducted on the reaction solution after initial stabilization of the reaction, that is at a reaction time of 20 hours (the analysis conditions, and the methods for calculating the conversion and the selectivity are the same as in Test Example IV-2), and the reaction solution after 400 hours of reaction (the analysis conditions, and the methods for calculating the conversion and the selectivity are the same as in Test Example IV-2). The analysis results are shown in Table IV-4.

Table IV-4 Results of Test Example IV-4

| Catalyst | | | Composition of catalyst | Conversion, % | Selectively, % | | | |
|---|---|---|---|---|---|---|---|---|
| | | | | | Hexanediamine | Hexamethyleneimine | Aminohexanol | Others |
| 20 h of reaction | | A-IV-1 | Co-Mo-Cu-Zn/Al$_2$O$_3$-SiO$_2$ | 95 | 67.3 | 15.4 | 14.9 | 2.4 |
| | | A-IV-4 | Co-Mo-Ag-Zn/Al$_2$O$_3$-ZSM-5 | 94 | 66.3 | 16.2 | 15.1 | 2.4 |
| | | D-IV-1 | Ni-Mo-Cu/Al$_2$O$_3$-SiO$_2$ | 80 | 48.2 | 21.3 | 24.9 | 5.6 |
| | | D-IV-2 | Co-W-Zn/Al$_2$O$_3$-SiO$_2$ | 81 | 46.3 | 21.8 | 26.6 | 5.3 |
| | | D-IV-3 | Ni-Mo-Cu-Mg/Al$_2$O$_3$-SiO$_2$ | 75 | 42.3 | 21.6 | 29.9 | 6.2 |
| | | D-IV-4 | Ni-Cu-Ca/Al$_2$O$_3$-SiO$_2$ | 76 | 39.2 | 20.5 | 32.2 | 8.1 |
| 400h of reaction | | A-IV-1 | Co-Mo-Cu-Zn/Al$_2$O$_3$-SiO$_2$ | 94 | 66.9 | 15.3 | 15.3 | 2.5 |
| | | A-IV-4 | Co-Mo-Ag-Zn/Al$_2$O$_3$-ZSM-5 | 94 | 66.1 | 16.3 | 15.1 | 2.5 |
| | | D-IV-1 | Ni-Mo-Cu/Al$_2$O$_3$-SiO$_2$ | 75 | 42.3 | 25.3 | 26.2 | 6.2 |
| | | D-IV-2 | Co-W-Zn/Al$_2$O$_3$-SiO$_2$ | 71 | 40.5 | 24.3 | 28.7 | 6.5 |
| | | D-IV-3 | Ni-Mo-Cu-Mg/Al$_2$O$_3$-SiO$_2$ | 61 | 30.5 | 24.9 | 35.7 | 8.9 |
| | | D-IV-4 | Ni-Cu-Ca/Al$_2$O$_3$-SiO$_2$ | 45 | 30.2 | 21.3 | 36.8 | 11.7 |

As can be seen from the above table, after the catalysts A-IV-1 and A-IV-4 were tested for 400 hours, the activity and selectivity of the catalysts are not substantially reduced (namely, the reduction value is less than 2%), while the activity and selectivity of the catalysts D-IV-1 to D-IV-4 are significantly reduced, and more byproducts are produced, indicating that the catalyst of the present application has a higher stability.

Example V series

The fifth type of embodiments of the present application are further illustrated in detail hereinbelow with reference to the examples of Example series V. In the following examples of Example series V, the pseudo-boehmite powder used had a ($Al_2O_3$) content on a dry basis of 70 wt%; titanium white powder was produced by titanyl sulfate-ammonia hydrolysis, with a sulfate content of 2.5wt% and a $TiO_2$ content of 95 wt%, and was commercially available from Shandong Dongjia Chemical Company, under a trade name of SA-100.

Example V-1

820 g of pseudo-boehmite powder (produced by nitric acid method, with a specific surface area of 380 m$^2$/g, and a pore volume of 0.96 mL/g) and 300 g of titanium white powder were uniformly mixed in a mixer, kneaded with 790 mL of a dilute acid aqueous solution containing 5 vol% of nitric acid, extruded into strips with a diameter of 5 mm, cut into a length of 4 mm, dried at 120 °C overnight, and calcined at 800 °C for 4 hours to obtain a desired carrier.

126 g of cobalt nitrate hexahydrate (industrial grade, with a purity of 98%) was dissolved in water to obtain a 150 mL solution, the solution was loaded onto 100 g of the carrier obtained by spray impregnation in two times, dried at 120 °C for 4 hours after each time of spray impregnation, then calcined at 400 °C for 4 hours, then reduced with hydrogen while gradually increasing the temperature at a rate of 20 °C/h, and finally reduced at 430 °C for 3 hours to obtain a catalyst A-V-1.

Example V-2

780 g of pseudo-boehmite powder (produced by carbonization method, with a specific surface area of 405 m$^2$/g, and a pore volume of 1.01 mL/g) and 220g of titanium white powder were uniformly mixed in a mixer, kneaded with 775 mL of a dilute acid aqueous solution containing 7 vol% of acetic acid, extruded into granules of clover shape with a thickness of 3 mm, dried at 120 °C overnight, and calcined at 850 °C for 3 hours to obtain a desired carrier.

140.4 g of nickel nitrate hexahydrate (industrial grade, with a purity of 98%) was dissolved in water to obtain a 144 mL solution, the solution was loaded onto 100 g of the carrier obtained by spray impregnation in two times, dried at 120 °C for 4 hours after each time of spray impregnation, then calcined at 390 °C for 4 hours, and then reduced with hydrogen while gradually increasing the temperature at a rate of 20 °C/h, and finally reduced at 440 °C for 3 hours to obtain a catalyst A-V-2.

Example V-3

820g of pseudo-boehmite powder (produced by aluminum sulfate method, with a specific surface area of 380 m$^2$/g, and a pore volume of 1.26 mL/g) and 180g of titanium white powder were uniformly mixed in a mixer, kneaded with 785 mL of a dilute acid aqueous solution containing 5 vol% of nitric acid, extruded into dentate spheres with a diameter of 4 mm, dried at 120 °C overnight and then calcined at 820 °C for 3 hours to obtain a desired carrier.

134.4 g of cobalt nitrate hexahydrate (industrial grade, with a purity of 98%) and 43.4 g of a 50wt% manganese nitrate solution were dissolved in water to obtain a 155 mL solution, the solution was loaded onto 100g of the carrier obtained by spray impregnation in two times, dried at 120 °C for 4 hours after each time of spray impregnation, then calcined at 400 °C for 4 hours, then reduced with hydrogen while gradually increasing the temperature at a rate of 20 °C/h, and finally reduced at 430 °C for 3 hours, to obtain a catalyst A-V-3.

Example V-4

1000 g of pseudo-boehmite powder (produced by nitric acid method, with a specific surface area of 380 m$^2$/g, and a pore volume of 0.96 mL/g) and 200 g of titanium white powder were uniformly mixed in a mixer, kneaded with 785 mL of a dilute acid aqueous solution containing 5 vol% of nitric acid, extruded into strips with a diameter of 5 mm, cut into a length of 4 mm, dried at 120 °C overnight, and then calcined at 800 °C for 4 hours to obtain a desired carrier.

129.2g of cobalt nitrate hexahydrate (industrial grade, with a purity of 98%) and 4.0 g of silver nitrate (analytically pure) were dissolved in water to obtain a 162 mL solution, the solution was loaded onto 100 g of the carrier obtained by spray impregnation in two times, dried at 120 °C for 4 hours after each time of spray impregnation, then calcined at 400 °C for 4 hours, then reduced with hydrogen while gradually increasing the temperature at a rate of 20 °C/h, and finally reduced at 430 °C for 3 hours to obtain a catalyst A-V-4.

Example V-5

1500 g of pseudo-boehmite powder (produced by carbonization method, with a specific surface area of 405 m$^2$/g, and a pore volume of 1.01 mL/g) and 250 g of titanium white powder were mixed uniformly in a mixer, kneaded with 777 mL of a dilute acid aqueous solution containing 5 vol% of nitric acid, extruded into granules of clover shape with a thickness of 3 mm, dried at 120 °C overnight, and then calcined for 3.5 hours at 820 °C to obtain a desired carrier.
132.6 g of nickel nitrate hexahydrate (industrial grade, with a purity of 98%) and 7.7 g of ammonium perrhenate were dissolved in water to obtain a 140 mL solution, the solution was loaded onto 100 g of the carrier obtained by spray impregnation in two times, dried at 120 °C for 4 hours after each time of spray impregnation, then calcined at 390 °C for 4 hours, then reduced with hydrogen while gradually increasing the temperature at a rate of 20 °C/h, and finally reduced at 440 °C for 3 hours to obtain a catalyst A-V-5.

Example V-6

950 g of pseudo-boehmite powder (produced by aluminum sulfate method, with a specific surface area of 380 m$^2$/g, and a pore volume of 0.96 mL/g) and 320 g of titanium white powder were uniformly mixed in a mixer, kneaded with 767 mL of a dilute acid aqueous solution containing 5 vol% of nitric acid and 2 vol% of sulfuric acid, extruded into dentate spheres with a diameter of 4 mm, dried at 120 °C overnight, and then calcined at 820 °C for 5h to obtain a desired carrier.
182.6 g of cobalt nitrate hexahydrate (industrial grade, with a purity of 98%) was dissolved in water to obtain a 170 mL solution, 16 g of ammonium molybdate tetrahydrate (analytically pure) was dissolved in water to obtain a 138 mL solution, the cobalt nitrate solution was loaded onto 100 g of the carrier obtained by spray impregnation in two times, then the ammonium molybdate solution was loaded onto the carrier obtained by spray impregnation in one time, then dried for 4 hours at 120 °C and calcined for 4 hours at 400 °C, then reduced with hydrogen while gradually increasing the temperature at a rate of 20 °C/h, and finally reduced for 3 hours at 430 °C, to obtain a catalyst A-V-6.

Example V-7

1000 g of pseudo-boehmite powder (produced by aluminum sulfate method, with a specific surface area of 380 m$^2$/g, and a pore volume of 0.96 mL/g) and 150 g of titanium white powder were uniformly mixed in a mixer, kneaded with 770 mL of a dilute acid aqueous solution containing 5 vol% of nitric acid and 2 vol% of sulfuric acid, extruded into dentate spheres with a diameter of 4 mm, dried at 120 °C overnight, and calcined at 820 °C for 3.5 hours to obtain a desired carrier.
177.4 g of cobalt nitrate hexahydrate (industrial grade, with a purity of 98%) and 15.9 g of copper nitrate trihydrate (analytically pure) were dissolved in water to obtain a 140 mL solution, the solution was loaded onto 100 g of the carrier obtained by spray impregnation in two times, dried at 120 °C for 4 hours after each time of spray impregnation, then calcined at 400 °C for 4 hours, then reduced with hydrogen while gradually increasing the temperature at a rate of 20 °C/h, and finally reduced at 430 °C for 3 hours to obtain a catalyst A-V-7.

Example V-8

780 g of pseudo-boehmite powder (produced by aluminum sulfate method, with a specific surface area of 380 m$^2$/g, and a pore volume of 0.96 mL/g) and 220 g of titanium white powder were uniformly mixed in a mixer, kneaded with 782 mL of a dilute acid aqueous solution containing 5 vol% of nitric acid, 2 vol% of hydrofluoric acid and 0.5 wt% of lanthanum nitrate, extruded into dentate spheres with a diameter of 4 mm, dried at 120 °C overnight, and calcined for 4 hours at 820 °C to obtain a desired carrier.
134.4 g of cobalt nitrate hexahydrate (industrial grade, with a purity of 98%) and 30.3 g of zinc nitrate hexahydrate (analytically pure) were dissolved in water to obtain a 160 mL solution, and the solution was loaded onto 100 g of the carrier obtained by spray impregnation in two times, dried at 120 °C for 4 hours after each time of spray impregnation, then calcined at 400 °C for 4 hours, then reduced with hydrogen while gradually increasing the temperature at a rate of 20 °C/h, and finally reduced at 430 °C for 3 hours, to obtain a catalyst A-V-8.

Comparative Example V-1

A catalyst was prepared as described in Example V-3, except that the amount of pseudo-boehmite powder used was 500g, and the amount of titanium white powder used was 500g, and 12.8 g of $KNO_3$ was added during the kneading process, to obtain a catalyst D-V-1.

Comparative Example V-2

The catalyst was prepared as described in Example V-2, except that the amount of pseudo-boehmite powder used was 900 g, and the amount of titanium white powder used was 100 g, to obtain a catalyst D-V-2.

Test Example V-1

The elemental composition of the catalyst was analyzed by plasma emission spectroscopy, and the results show that all sulfur in the titanium white powder is retained in the carrier, and the contents of other elements are substantially the same as the feeding amount; the catalysts obtained above were characterized by $NH_3$-TPD, $CO_2$-TPD, BET nitrogen adsorption-desorption methods, and the results are shown in Table V-1.

Table V-1 Properties of the catalysts of the examples and comparative examples

| Catalyst | Composition of catalyst | Ammonia adsorption capacity | $CO_2$ adsorption capacity | Specific surface area | Pore volume | Proportion of pore volume of pores having a pore diameter < 4nm | Proportion of pore volume of pores having a pore diameter > 10 nm | Doping elements and relative contents thereof, g |
|---|---|---|---|---|---|---|---|---|
| | | mmol/g | mmol/g | $m^2/g$ | ml/g | % | % | |
| A-V-1 | $Co/Al_2O_3$-$TiO_2$ | 0.33 | 0.16 | 161 | 0.65 | 18 | 8 | S 0.83 |
| A-V-2 | $Ni/Al_2O_3$-$TiO_2$ | 0.35 | 0.13 | 159 | 0.68 | 15 | 9.5 | S 0.69 |
| A-V-3 | Co-$Mn/Al_2O_3$-$TiO_2$ | 0.38 | 0.13 | 170 | 0.71 | 14 | 10 | S 1.54 |
| A-V-4 | Co-$Ag/Al_2O_3$-$TiO_2$ | 0.37 | 0.14 | 172 | 0.68 | 12 | 11 | S 0.53 |
| A-V-5 | Ni-Re/ $Al_2O_3$-$TiO_2$ | 0.36 | 0.16 | 168 | 0.66 | 11 | 12 | S 0.46 |
| A-V-6 | Co-$Mo/Al_2O_3$-$TiO_2$ | 0.32 | 0.09 | 143 | 0.59 | 8 | 14 | S 1.75 |
| A-V-7 | Co-$Cu/Al_2O_3$-$TiO_2$ | 0.32 | 0.1 | 139 | 0.61 | 17 | 7 | S 1.67 |
| A-V-8 | Co-$Zn/Al_2O_3$-$TiO_2$ | 0.34 | 0.12 | 157 | 0.68 | 15 | 12 | S 1.83/F 0.5/La 0.6 |
| D-V-1 | Co-Mn-$K/Al_2O_3$-$TiO_2$ | 0.16 | 0.11 | 131 | 0.57 | 20 | 17 | K 0.6 |
| D-V-2 | Co-$Mn/Al_2O_3$ | 0.24 | 0.07 | 155 | 0.75 | 21 | 16 | None |

Test Example V-2

100 mL of the catalysts A-V-1 to A-V-8 obtained and the catalysts D-V-1 and D-V-2 obtained were respectively measured out, loaded into a fixed bed reactor, activated with hydrogen at 220 °C for 2 hours, then cooled to 168 °C, the pressure of the system was raised to 12.5 MPa using hydrogen, ammonia was metered into the reaction system via a metering pump, preheated to 150 °C and sent to the reactor, heated and melted 1,6-hexanediol was fed into the reactor via a metering pump, hydrogen was stably introduced via a gas mass flowmeter, wherein the molar ratio of hydrogen to ammonia and to 1,6-hexanediol was 3 : 12 : 1, and the liquid phase volume space velocity of 1,6-hexanediol was 0.4 $h^{-1}$, a catalytic amination reaction was conducted in the reactor at a reaction temperature of 175 °C and a reaction pressure of 12.5 MPa, and the reaction solution was sampled and analyzed after the reaction conditions were stabilized for 2 hours. The analysis results are shown in Table V-2.

The analysis of the sample was conducted by gas chromatography, and was calibrated using a correction factor of a standard sample formulated.

The conversion and selectivity were calculated based on the molar content of each component in the reaction solution, and the calculation methods were the same as described in Test Example I-2.

Table V-2 Test results for the catalysts of the examples and comparative examples

| Catalyst | Conversion, % | Selectivity, % | | | |
|---|---|---|---|---|---|
| | | Hexanediamine | Hexamethyleneimine | Aminohexanol | Others |
| A-V-1 | 87 | 47.5 | 20.6 | 28.1 | 3.8 |
| A-V-2 | 89 | 47.2 | 19.1 | 29.3 | 4.4 |
| A-V-3 | 93 | 49.8 | 20.4 | 27.1 | 2.7 |
| A-V-4 | 92 | 50.9 | 18.4 | 27.9 | 2.8 |
| A-V-5 | 91 | 49.7 | 20.6 | 26.8 | 2.9 |
| A-V-6 | 91 | 48.8 | 16.9 | 30.7 | 3.6 |
| A-V-7 | 85 | 46.7 | 19.2 | 29.5 | 4.6 |
| A-V-8 | 90 | 49.6 | 19.6 | 28.2 | 2.6 |
| D-V-1 | 78 | 41.3 | 23.7 | 29.1 | 8.7 |
| D-V-2 | 69 | 36.5 | 25.2 | 36.4 | 6.5 |

As can be seen from the data in the table above, the content of "others", i.e. impurities, of the catalysts D-V-1 and D-V-2 is significantly higher, and the conversion of those catalysts is much lower, as compared to other catalysts.

After 196 hours of evaluation of each catalyst, the catalyst was discharged for characterization. It can be seen that the specific surface areas and pore volumes of the catalysts A-V-1 to A-V-8 are substantially unchanged, and no obvious carbon deposition was observed, while the specific surface areas of the catalysts D-V-1 and D-V-2 are respectively reduced by 7% and 9%, the pore volumes are respectively reduced by 7%, and their carbon deposition is respectively 5.6 wt% and 6.7 wt%, indicating that the catalyst of the present application has a more stable catalytic performance, and can accelerate the reaction rate, reduce the carbon deposition and retard the blockage of the pore channel.

Test Example V-3

100 mL of the catalysts obtained were measured out, loaded into a fixed bed reactor, activated with hydrogen at 220 °C for 2 hours, then cooled to 168 °C, the pressure of the system was raised to 8 MPa using hydrogen, then ammonia was metered into the reaction system via a metering pump, preheated to 150 °C and then sent to the upper end of the reactor, a mixed solution of hexanediol, hexamethyleneimine and 6-amino-1-hexanol (50 wt% of hexanediol, 32 wt% of hexamethyleneimine and 18 wt% of 6-amino-1-hexanol) was fed into the upper end of the reactor via a metering pump, hydrogen was stably introduced via a gas mass flowmeter, wherein the molar ratio of hydrogen to ammonia and to the mixed solution was 3 : 18 : 1, the liquid phase volume space velocity of the mixed solution was 0.5 $h^{-1}$, a catalytic amination reaction was conducted in the reactor at a reaction temperature of 185 °C and a reaction pressure of 8 MPa, and after the reaction conditions were stabilized for 2 hours, the reaction solution was sampled and analyzed (the analysis conditions, and the methods for calculating the conversion and the selectivity are the same as in Test Example V-2). The analysis results are shown in Table V-3.

Table V-3 Results of Test Example V-3

| Catalyst | Conversion of hexanediol, % | Selectivity, % | | | |
|---|---|---|---|---|---|
| | | Hexanediamine | Hexamethyleneimine | Aminohexanol | Others |
| A-V-1 | 94.9 | 92.8 | 0.8 | 3.8 | 2.6 |
| A-V-2 | 94.7 | 93.6 | 0.7 | 2.9 | 2.8 |
| A-V-3 | 97.7 | 96.4 | 0.8 | 1.8 | 1 |
| A-V-4 | 97.2 | 97 | 0.6 | 1.7 | 0.7 |
| A-V-5 | 96.6 | 96.1 | 0.6 | 2.1 | 1.2 |
| A-V-6 | 96.8 | 94.5 | 0.7 | 2.2 | 2.6 |
| A-V-7 | 94.6 | 93.9 | 0.5 | 2.5 | 3.1 |
| A-V-8 | 96.1 | 95.1 | 0.6 | 2.3 | 2 |
| D-V-1 | 79.5 | 85.2 | 1.6 | 5.6 | 7.6 |
| D-V-2 | 70.6 | 86.3 | 2.9 | 2.6 | 8.2 |

As can be seen from the data in the table above, the catalyst of the present application is capable of providing a higher hexanediol conversion and a higher selectivity of hexanediamine, as compared to the catalysts D-V-1 and D-V-2.

The present application is illustrated in detail hereinabove with reference to preferred embodiments, but is not intended to be limited to those embodiments. Various modifications may be made following the inventive concept of the present application, and these modifications shall be within the scope of the present application.

It should be noted that the various technical features described in the above embodiments may be combined in any suitable manner without contradiction, and in order to avoid unnecessary repetition, various possible combinations are not described in the present application, but such combinations shall also be within the scope of the present application.

In addition, the various embodiments of the present application can be arbitrarily combined as long as the combination does not depart from the spirit of the present application, and such combined embodiments should be considered as the disclosure of the present application.

## Claims

1. A catalyst useful for producing organic amines by catalytic amination, comprising an inorganic porous carrier containing aluminium and/or silicon and an active metal component supported on the carrier, wherein the active metal component comprises at least one metal selected from the group consisting of Group VIII and Group IB metals, preferably selected from cobalt, nickel, palladium, copper or a combination thereof, more preferably selected from cobalt, nickel or a combination thereof, wherein the carrier has an ammonia adsorption capacity of 0.25 to 0.65 mmol/g, preferably 0.3 to 0.6 mmol/g, as measured by $NH_3$-TPD test.

2. The catalyst according to claim 1, wherein the carrier comprises a matrix and a doping element, the matrix comprising a first carrier component and optionally a second carrier component, wherein the first carrier component is selected from alumina, silica, molecular sieves, aluminosilicates, or combinations thereof, the second carrier component is selected from diatomite and titania, and

the doping element is selected from a metallic element, a non-metallic element or a combination thereof and does not include sodium or chlorine, the metallic element is selected from Group IA metal elements, Group IIA metal elements, Group VA metal elements, lanthanide series metal elements or combinations thereof, and preferably selected from calcium, magnesium, potassium, bismuth, strontium, barium, lanthanum or a combination thereof; the non-metallic element is selected from Group IIIA non-metallic elements, Group VA non-metallic elements, Group VIA non-metallic elements, Group VIIA non-metallic elements or combinations thereof, preferably selected from boron, fluorine, phosphorus, sulfur, selenium or combinations thereof;
preferably, the doping element in the carrier is derived from metal cations and/or acid radical ions, but does not include sodium ion or chloride ion; the metal cation is selected from Group IA metal cations, Group IIA metal ions, Group VA metal ions, lanthanide series metal ions or combinations thereof, preferably selected from calcium ion, magnesium ion, potassium ion, bismuth ion, strontium ion, barium ion, lanthanum ion or combinations thereof; the acid radical ion is selected from non-metallic acid radical ions, preferably selected from borate ion, fluoride ion, phosphate ion, sulfate ion, selenate ion or combinations thereof.

3. The catalyst according to claim 1 or 2, wherein the carrier has at least one of the following characteristics:

the carrier has a carbon dioxide adsorption capacity of 0.05-0.4 mmol/g, preferably 0.05-0.3 mmol/g, more preferably 0.06-0.2 mmol/g;
the doping element is present in the carrier in an amount of 0.03 to 6 wt%, preferably 0.05 to 6 wt%, more preferably 0.08 to 4 wt%, relative to the total weight of the matrix;
the carrier has a specific surface area of 120-240 $m^2/g$, preferably 120-210 $m^2/g$, and more preferably 125-200 $m^2/g$;
the carrier has a pore volume of 0.45-1.2 ml/g, preferably 0.45-1.1 ml/g, and more preferably 0.5-1 ml/g;
the proportion of the pore volume of pores having a pore diameter in a range of 7-27 nm to the pore volume of the carrier is greater than 65%, preferably 70% or more, more preferably 70 to 90%, and preferably the proportion of the pore volume of pores having a pore diameter of less than 7 nm to the pore volume of the carrier is 0 to 10%, for example 0 to 8%;
the matrix in the carrier comprises a combination of alumina and titania at a weight ratio of 1.5-5 : 1, preferably 2-4.5 : 1; and
the alumina content in the carrier is 70 wt% or more, preferably 75 wt% or more, more preferably 80-100 wt%, based on the total weight of the matrix.

4. The catalyst according to any one of claims 1-3, wherein the active metal component is present in an amount of from 5 to 46 g, preferably from 10 to 42 g, for example from 13 to 40 g, per 100g of the matrix.

5. The catalyst according to any one of claims 1-4, further comprising a metal promoter supported on the carrier, and the metal promoter comprises at least one metal selected from the group consisting of Group VIB, Group VIIB, Group IB, Group IIB, and lanthanide series metals, preferably at least one metal selected from Cr, Mo, W, Mn, Re, Cu, Ag, Au, Zn, La, and Ce;
preferably, the metal promoter is present in an amount of 0 to 10g, preferably 0.1 to 10g, more preferably 0.5 to 8 g, per 100 g of the matrix.

6. The catalyst according to claim 5, wherein:

the metal promoter comprises a combination of at least one Group VIIB metal and at least one Group IB metal, wherein the weight ratio of the Group VIIB metal to the Group IB metal, calculated as metal element, is 0.05-15 : 1, preferably 0.1-12 : 1; or
the metal promoter comprises a combination of at least one Group VIIB metal and at least one Group IIB metal, wherein the weight ratio of the Group VIIB metal to the Group IIB metal, calculated as metal element, is 0.2-20 : 1, preferably 0.3-6 : 1; or
the metal promoter comprises a combination of at least one Group VIB metal, at least one Group IB metal and at least one Group IIB metal, wherein the weight ratio of the Group VIB metal to the Group IB metal and to the Group IIB metal, calculated as metal element, is 0.1-10 : 0.1-10 : 1, preferably 0.2-8 : 0.2-8 : 1,
preferably, the Group VIIB metal is selected from manganese, rhenium, or a combination thereof, the Group IB metal is selected from copper, silver, gold, or a combination thereof, the Group IIB metal is zinc, and the Group VIB metal is selected from molybdenum, tungsten, or a combination thereof.

7. A method for producing the catalyst according to any one of claims 1-6, comprising the steps of:

1) providing an inorganic porous carrier containing aluminium and/or silicon, which has an ammonia adsorption capacity of 0.25 to 0.65 mmol/g, preferably 0.3 to 0.6 mmol/g, as measured by $NH_3$-TPD test;
2) loading the active metal component and optionally the metal promoter on the carrier; and
3) carrying out a heat treatment and optionally a reduction treatment on the material obtained in step 2) to obtain the catalyst,

preferably, the heat treatment comprises calcining, or a combination of drying and calcining.

8. The method according to claim 7, wherein said "providing an inorganic porous carrier containing aluminum and/or silicon" of step 1) comprises subjecting a mixture comprising a doping element and a matrix or a precursor thereof to shaping, drying and calcining sequentially to obtain the carrier,

wherein the matrix comprises a first carrier component and optionally a second carrier component, wherein the first carrier component is selected from alumina, silica, molecular sieves, aluminosilicates or combinations thereof, the second carrier component is selected from diatomite, titanium white powder or a combination thereof, preferably, the first carrier component is alumina, and the precursor of the first carrier component is pseudo-boehmite having a specific surface area of 250-410 $m^2/g$, preferably 260-400 $m^2/g$, more preferably 260-380 $m^2/g$ and a pore volume of 0.7-1.3 ml/g, preferably 0.7-1.2 ml/g, more preferably 0.8-1.2 ml/g;

the doping element is selected from a metallic element, a non-metallic element or a combination thereof and does not include sodium or chlorine, the metallic element is selected from Group IA metal elements, Group IIA metal elements, Group VA metal elements, lanthanide series metal elements or combinations thereof, and preferably selected from calcium, magnesium, potassium, bismuth, strontium, barium, lanthanum or a combination thereof; the non-metallic element is selected from Group IIIA non-metallic elements, Group VA non-metallic elements, Group VIA non-metallic elements, Group VIIA non-metallic elements or combinations thereof, preferably selected from boron, fluorine, phosphorus, sulfur, selenium or combinations thereof,

preferably, the drying conditions of step 1) include: a temperature of 80-150 °C, and a drying time of 6-20 h; and preferably, the calcining conditions of step 1) include: a temperature of 500-1100 °C, and a calcining time of 2-20 h.

9. The method according to claim 8, wherein the doping element is provided using a carrier modifier comprising at least one compound capable of providing a cation and/or an anion, wherein the cation is selected from Group IA cations, Group IIA metal ions, Group VA metal ions, lanthanide series metal ions, or combinations thereof, preferably from calcium ion, magnesium ion, potassium ion, bismuth ion, strontium ion, barium ion, lanthanum ion, or combinations thereof;

the anion is selected from non-metallic acid radical ions, preferably selected from borate ion, fluoride ion, phosphate ion, sulfate ion, selenate ion or combinations thereof;

preferably, the carrier modifier is selected from boric acid, nickel borate, cobalt borate, potassium borate, hydrofluoric acid, potassium fluoride, cobalt fluoride, nickel fluoride, phosphoric acid, aluminum phosphate, tripotassium phosphate, potassium dihydrogen phosphate, potassium hydrogen phosphate, magnesium phosphate, calcium phosphate, sulfuric acid, cobalt sulfate, nickel sulfate, aluminum sulfate, calcium sulfate, bismuth nitrate, potassium nitrate, potassium sulfate, potassium carbonate, magnesium nitrate, magnesium sulfate, basic magnesium carbonate, calcium nitrate, basic calcium carbonate, strontium nitrate, strontium phosphate, strontium sulfate, barium nitrate, lanthanum nitrate, selenic acid, or combinations thereof.

10. The method according to any one of claims 7-9, wherein the loading of step 2) comprises impregnating the carrier with a solution comprising a precursor of the active metal component and optionally a precursor of the metal promoter.

11. A process for producing an organic amine, comprising: contacting an amination raw material and an amination reagent with the catalyst according to any one of claims 1-6 in the presence of hydrogen for amination reaction to obtain an organic amine,

wherein the amination raw material is selected from alcohols, ketones, alcohol amines, aldehydes or combinations thereof, preferably selected from C2-20 alcohols, C3-20 ketones, C2-20 alcohol amines, C2-20 aldehydes or combinations thereof, more preferably selected from ethanol, acetaldehyde, n-propanol, propionaldehyde, isopropanol, n-butanol, butyraldehyde, isobutanol, isobutyraldehyde, 2-ethylhexanol, 2-ethylhexaldehyde, octanol, octanal, dodecanol, dodecanal, hexadecanol, hexadecanal, cyclopentanol, cyclohexanol, cyclooctanol, cyclododecanol, benzyl alcohol, benzaldehyde, phenethyl alcohol, phenylacetaldehyde, 1,4-butanediol, 1,4-butanedial, 1,5-pentanediol, 1,5-glutaraldehyde, 1,6-hexanediol, 1,6-hexandial, 1,8-octanediol, 1,8-octanedial, 1,12-dodecanediol, 1,12-dodecanedialdehyde, ethanolamine, propanolamine, isopropanolamine, 6-aminohexanol, diethanolamine, diisopropanolamine, dimethylethanolamine, acetone, ethylene glycol, 1,3-propanediol, or combinations thereof;

the amination reagent is selected from ammonia, primary amines, secondary amines or combinations thereof, preferably selected from ammonia, C1-12 primary amines, C2-12 secondary amines, or combinations thereof, more preferably selected from ammonia, monomethylamine, dimethylamine, methyl ethyl amine, monoethylamine, diethylamine or a combination thereof.

**12.** The process according to claim 11, wherein the amination conditions include: a molar ratio of hydrogen to the amination reagent and to the amination raw material of 1-5 : 2-35 : 1, preferably 1-5 : 2-33 : 1, more preferably 1-5 : 2-30 : 1, a temperature of 105-220 °C, preferably 110-220 °C, more preferably 130-200 °C, a pressure of 0.7-25 MPa, preferably 0.8-25 MPa, more preferably 1-15 MPa, and a liquid phase volume space velocity of the amination raw material of 0.06-1 m$^3$/(m$^3$·h).

**13.** The process according to claim 12, wherein:

where the amination raw material is a monohydric alcohol, the amination conditions include: a molar ratio of hydrogen to the amination reagent and to the amination raw material of 1-4 : 2-10 : 1, preferably 1-4 : 2-9 : 1, more preferably 1-4 : 2-8 : 1, a temperature of 130-210 °C, preferably 130-200 °C, a pressure of 1-4 MPa, preferably 1-3.5 MPa, more preferably 1-2.5 MPa, and a liquid phase volume space velocity of the amination raw material of 0.1-0.8 m$^3$/(m$^3$·h);

where the amination raw material is a ketone or an aldehyde, the amination conditions include: a molar ratio of hydrogen to the amination reagent and to the amination raw material of 1-4 : 2-6 : 1, preferably 1-4 : 2-5 : 1, a temperature of 105-180 °C, preferably 110-180 °C, more preferably 110-170 °C, a pressure of 0.7-3.5 MPa, preferably 0.7-2.5 MPa, more preferably 0.8-2.5 MPa, and a liquid phase volume space velocity of the amination raw material of 0.1-1 m$^3$/(m$^3$·h), preferably 0.1-0.8 m$^3$/(m$^3$·h);

where the amination raw material is an alcohol amine, the amination conditions include: a molar ratio of hydrogen to the amination reagent and to the amination raw material of 1-4 : 3-25 : 1, preferably 1-4 : 3-20 : 1, a temperature of 130-200 °C, preferably 135-200 °C, a pressure of 1-18 MPa, preferably 1-15 MPa, more preferably 1-11 MPa, and a liquid phase volume space velocity of the amination raw material of 0.06-0.8 m$^3$/(m$^3$·h);

where the amination raw material is a dihydric alcohol, the amination conditions include: a molar ratio of hydrogen to the amination reagent and to the amination raw material of 0.3-4 : 3-45 : 1, preferably 1-4 : 3-35 : 1, more preferably 1-4 : 3-33 : 1, a temperature of 130-220 °C, preferably 130-210 °C, a pressure of 1-15 MPa, preferably 4-25 MPa, and a liquid phase volume space velocity of the amination raw material of 0.06-0.8 m$^3$/(m$^3$·h), preferably 0.1-0.8 m$^3$/(m$^3$·h), or

where the amination raw material is a mixture of 1,6-hexanediol, hexamethyleneimine and 6-amino-1-hexanol, the amination conditions include: a molar ratio of hydrogen to the amination reagent and to the amination raw material of 0.3-4 : 3-45 : 1, preferably 1-4 : 3-35 : 1, more preferably 1-4 : 3-33 : 1, more preferably 1-4 : 3-30 : 1, a temperature of 130-220 °C, preferably 130-210 °C, a pressure of 1-25 MPa, preferably 2-25 MPa, more preferably 4-25 MPa, and a liquid phase volume space velocity of the amination raw material of 0.06-0.8 m$^3$/(m$^3$·h), preferably 0.1-0.8 m$^3$/(m$^3$·h).

# EP 4 238 648 A1

## INTERNATIONAL SEARCH REPORT

| | International application No. |
|---|---|
| | **PCT/CN2021/126422** |

**A. CLASSIFICATION OF SUBJECT MATTER**

B01J 23/75(2006.01)i; B01J 23/755(2006.01)i; B01J 23/78(2006.01)i; B01J 23/80(2006.01)i; B01J 23/83(2006.01)i; C07C 211/03(2006.01)i; C07C 209/04(2006.01)i

According to International Patent Classification (IPC) or to both national classification and IPC

**B. FIELDS SEARCHED**

Minimum documentation searched (classification system followed by classification symbols)

B01J23/-; C07C211/-; C07C209/-

Documentation searched other than minimum documentation to the extent that such documents are included in the fields searched

Electronic data base consulted during the international search (name of data base and, where practicable, search terms used)

CNTXT, CNKI, VEN, WEB OF SCIENCE: 胺化, 氨化, 催化, 程序升温脱附法, 载体, 氧化铝, 氧化钛, 硅藻土, 磷酸, 硼酸, 硫酸, 氢氟酸, amination, ammonification, catal+, TPD, support, carrier, Al2O3, alumina, TiO2, titania, diatomite, phosphoric acid, boric acid, sulphuric acid, hydrofluoric acid

**C. DOCUMENTS CONSIDERED TO BE RELEVANT**

| Category* | Citation of document, with indication, where appropriate, of the relevant passages | Relevant to claim No. |
|---|---|---|
| Y | 吕养心 等 (LYU, Yangxin et al.). "CuO/NiO/γ—Al2O3催化剂合成环己胺 (Synthesis of Cyclohexylamine with CuO/NiO/γ-Al2O3 Catalyst)" 化学反应工程与工艺 (Chemical Reaction Engineering and Technology), Vol. 20, No. 2, 30 June 2004 (2004-06-30), sections 1.1.1, 1.2 and abstract | 1-5, 7-13 |
| Y | 马骏 等 (MA, Jun et al.). "β沸石在乙醇胺化反应中的催化行为 (Catalytic Behaviour of Amination of C2H5OH with NH3 over β Zeolite)" 石油化工 (Petrochemical Technology), Vol. 22, No. 9, 30 September 1993 (1993-09-30), section 3.1 and abstract | 1-5, 7-13 |
| A | CN 101569862 A (CHINA PETROLEUM & CHEMICAL CORPORATION et al.) 04 November 2009 (2009-11-04) entire document | 1-13 |
| A | KR 20120103323 A (KOREA RESEARCH INSTITUTE OF CHEMICAL TECHNOLOGY et al.) 19 September 2012 (2012-09-19) entire document | 1-13 |

☐ Further documents are listed in the continuation of Box C.     ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **19 January 2022** | **26 January 2022** |

| Name and mailing address of the ISA/CN | Authorized officer |
|---|---|
| **China National Intellectual Property Administration (ISA/ CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

INTERNATIONAL SEARCH REPORT
Information on patent family members

International application No.

**PCT/CN2021/126422**

| Patent document cited in search report | | | Publication date (day/month/year) | Patent family member(s) | | | Publication date (day/month/year) |
|---|---|---|---|---|---|---|---|
| CN | 101569862 | A | 04 November 2009 | CN | 101569862 | B | 30 November 2011 |
| KR | 20120103323 | A | 19 September 2012 | KR | 101336975 | B1 | 04 December 2013 |

Form PCT/ISA/210 (patent family annex) (January 2015)

**EP 4 238 648 A1**

**REFERENCES CITED IN THE DESCRIPTION**

**Patent documents cited in the description**

- US 4409399 A
- CN 102658162 A
- GB T6609352009 A